# EUROPEAN PATENT APPLICATION

(11) **EP 3 467 073 A1**
(43) Date of publication of application: **10.04.2019**
(21) Application number: 17806510.8
(22) Date of filing: 25.05.2017
(51) Int. Cl.: C09K 19/54, C09K 19/12, C09K 19/14, C09K 19/16, C09K 19/18, C09K 19/20, C09K 19/24, C09K 19/30, C09K 19/32, G02F 1/13, G02F 1/1337

(54) **SPONTANEOUS ORIENTATION AID FOR LIQUID CRYSTAL COMPOSITION, COMPOUND SUITABLE FOR SAID SPONTANEOUS ORIENTATION AID, LIQUID CRYSTAL COMPOSITION, AND LIQUID CRYSTAL DISPLAY ELEMENT**

(30) Priority: 03.06.2016 JP 2016111920
(71) Applicant: DIC Corporation, Tokyo 174-8520 (JP)
(72) Inventor: KIMURA, Masaomi, Kita-adachi-gun Saitama 362-8577 (JP); AKIYAMA, Hidenari, Kita-adachi-gun Saitama 362-8577 (JP); INOUE, Yuuichi, Kita-adachi-gun Saitama 362-8577 (JP); HAYASHI, Masanao, Kita-adachi-gun Saitama 362-8577 (JP); HAYASHI, Takuo, Kita-adachi-gun Saitama 362-8577 (JP)
(74) Representative: Adam, Holger
(86) International application number: PCT/JP2017/019475
(87) International publication number: WO 2017/208953

(57) **Abstract**

The present invention provides a spontaneous orientation aid for a liquid crystal composition, containing one kind or two or more kinds of a compound having a partial structure represented by the general formula (i), wherein Zⁱ¹ represents a single bond, etc.; Aⁱ¹ represents a divalent 6-membered aromatic group, etc.; mⁱ¹ represents an integer of 1 to 5; and Kⁱ¹ represents a structure represented by any of the following formulae (K-1) to (K-8), wherein W^{K1} represents a methine group, etc.; X^{K1} and Y^{K1} each represent -CH₂-, etc.; Z^{K1} represents an oxygen atom, etc.; and U^{K1}, V^{K1}, and S^{K1} each represent a methine group, etc.:

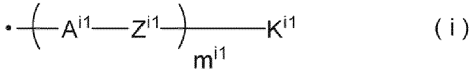

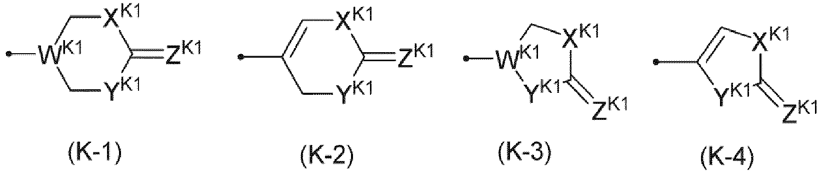

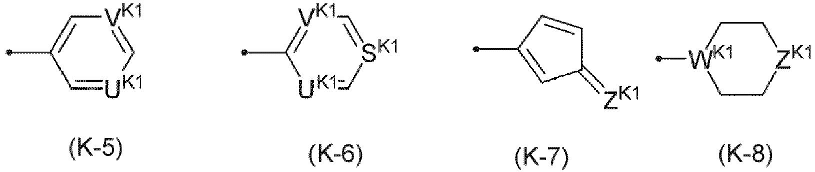

## Description

### Technical Field

The present invention relates to a spontaneous orientation aid for a liquid crystal composition, a compound suitable for the spontaneous orientation aid, a liquid crystal composition, and a liquid crystal display element.

### Background Art

In a VA mode liquid crystal display, a polyimide alignment film (PI) layer has been provided on the electrode for inducing vertical alignment of the liquid crystal molecules without application of voltage and achieving horizontal alignment of the liquid crystal molecules with application of voltage. However, the film formation of the PI layer requires large cost, and therefore a method for achieving the alignment of liquid crystal molecules without the PI layer provided has been investigated in recent years.

For example, PTL 1 describes a liquid crystal medium that is based on a mixture of a polar compound having a negative dielectric anisotropy and is characterized by at least one kind of a spontaneous orientation aid contained, and describes that the liquid crystal medium is highly suitable for the use in a display having no alignment layer. PTL 1 uses the particular compound having a hydroxyl group as the spontaneous alignment additive.

### Citation List

### Patent Literature

PTL 1: JP-T-2014-524951

### Summary of Invention

### Technical Problem

However, according to the investigation by the present inventors, it has been found that in the case where the spontaneous alignment additive described in PTL 1 is used, the alignment regulation force for aligning liquid crystal molecules vertically is insufficient, and there is room of improvement in storage stability of a liquid crystal composition containing the spontaneous alignment additive.

Under the circumstances, an object of the invention is to provide a spontaneous orientation aid for a liquid crystal composition, capable of ensuring the storage stability when added to a liquid crystal composition and capable of achieving vertical alignment of liquid crystal molecules without a PI layer provided, and a compound suitable for the spontaneous orientation aid. Another object of the invention is to provide a liquid crystal composition that is excellent in storage stability and contains the spontaneous orientation aid capable of achieving vertical alignment of liquid crystal molecules without a PI layer provided, and a liquid crystal display element using the liquid crystal composition.

### Solution to Problem

The invention provides a spontaneous orientation aid for a liquid crystal composition, containing one kind or two or more kinds of a compound having a partial structure represented by the general formula (i): 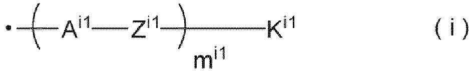 wherein Zⁱ¹ represents a single bond, -CH=CH-, -CF=CF-, -C=C-, -COO-, -OCO-, -CF₂O-, -OCF₂-, or an alkylene group having 2 to 20 carbon atoms, in which one or two or more of -CH₂- that are not adjacent to each other in the alkylene group each may be replaced by -O-, -COO-, or -OCO-; Aⁱ¹ represents a divalent 6-membered aromatic group, a divalent 6-membered heteroaromatic group, a divalent 6-membered alicyclic group, a divalent 6-membered heteroalicyclic group, or a single bond, in which a hydrogen atom in the cyclic structures may be replaced by a halogen atom, and in the case where a plurality of each of Zⁱ¹ and Aⁱ¹ are present, these may be the same as or different from each other; mⁱ¹ represents an integer of 1 to 5; and Kⁱ¹ represents a structure represented by any of the following formulae (K-1) to (K-8): 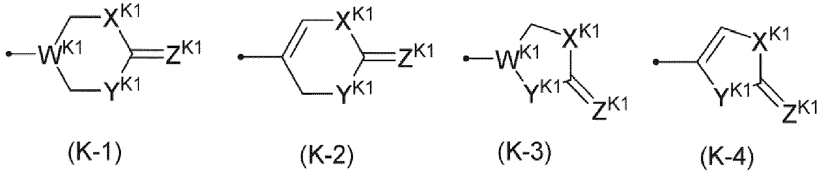 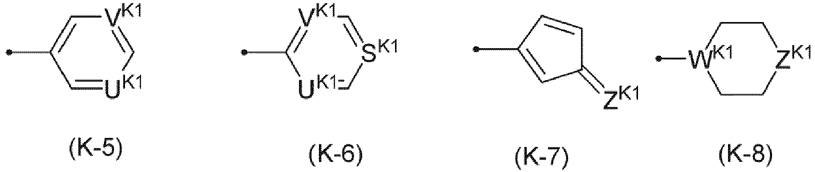 wherein W^{K1} represents a methine group or a nitrogen atom; X^{K1} and Y^{K1} each independently represent -CH₂-, an oxygen atom, or a sulfur atom; Z^{K1} represents an oxygen atom or a sulfur atom; and U^{K1}, V^{K1}, and S^{K1} each independently represent a methine group or a nitrogen atom, provided that a combination where U^{K1} represents a methine group, V^{K1} represents a methine group, and S^{K1} represents a nitrogen atom is excluded, and in the formula (i) and the formulae (K-1) to (K-8), the black dot on the left end represents a bonding site.

The invention also provides a liquid crystal composition having a negative dielectric anisotropy (**Δε**), containing one kind or two or more kinds of a compound having a partial structure represented by the general formula (i).

### Advantageous Effects of Invention

According to the spontaneous orientation aid for a liquid crystal composition and the compound suitable for the spontaneous orientation aid of the invention, the storage stability when added to a liquid crystal composition can be ensured, and vertical alignment of liquid crystal molecules can be achieved without a PI layer provided. According to the invention, furthermore, a liquid crystal composition that is excellent in storage stability and contains the spontaneous orientation aid capable of achieving vertical alignment of liquid crystal molecules without a PI layer provided, and a liquid crystal display element using the liquid crystal composition can be provided.

### Brief Description of Drawings

Fig. 1 is a diagram schematically showing one embodiment of a liquid crystal display element.
Fig. 2 is an enlarged plane view of the region surrounded by the line I in Fig. 1.

### Description of Embodiments

### Spontaneous Orientation aid for Liquid Crystal Composition

The spontaneous orientation aid for a liquid crystal composition of one embodiment contains one kind or two or more kinds of a compound having a partial structure represented by the general formula (i) (which may be hereinafter referred to as a "compound (i)").

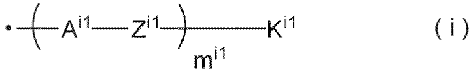

In the formula (i), Zⁱ¹ represents a single bond, -CH=CH-, -CF=CF-, -C≡C-, -COO-, -OCO-, -CF₂O-, -OCF₂-, or an alkylene group having 2 to 20 carbon atoms, in which the alkylene group may be linear or branched, and one or two or more of -CH₂- that are not adjacent to each other in the alkylene group each may be replaced by -O-, -COO-, or -OCO-; Aⁱ¹ represents a divalent 6-membered aromatic group, a divalent 6-membered heteroaromatic group, a divalent 6-membered alicyclic group, a divalent 6-membered heteroalicyclic group, or a single bond, in which a hydrogen atom in the cyclic structures may be replaced by a halogen atom, and in the case where a plurality of each of Zⁱ¹ and Aⁱ¹ are present, these may be the same as or different from each other; mⁱ¹ represents an integer of 1 to 5; Kⁱ¹ represents a structure represented by any of the following formulae (K-1) to (K-8); and the black dot on the left end represents a bonding site.

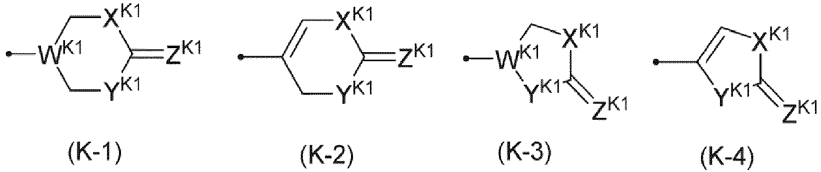

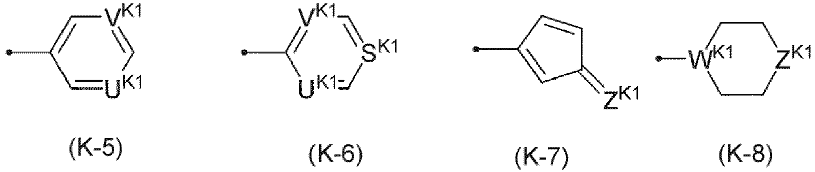

In the formulae (K-1) to (K-8), W^{K1} represents a methine group or a nitrogen atom; X^{K1} and Y^{K1} each independently represent -CH₂-, an oxygen atom, or a sulfur atom; Z^{K1} represents an oxygen atom or a sulfur atom; U^{K1}, V^{K1}, and S^{K1} each independently represent a methine group or a nitrogen atom, provided that a combination where U^{K1} represents a methine group, V^{K1} represents a methine group, and S^{K1} represents a nitrogen atom is excluded; and the black dot on the left end represents a bonding site.

The spontaneous orientation aid for a liquid crystal composition of the embodiment contains a compound having a partial structure represented by the formula (i), particularly a structure represented by any of the formulae (K-1) to (K-8) as Kⁱ¹ in the formula (i), and thereby it is expected that when added to a liquid crystal composition, the spontaneous orientation aid is adsorbed to substrates that hold the liquid crystal composition (liquid crystal layer), and retains the liquid crystal molecules in a state of alignment in the vertical direction. Accordingly, with the spontaneous orientation aid for a liquid crystal composition of the embodiment, the liquid crystal molecules can be aligned (i.e., vertical alignment of the liquid crystal molecules can be induced without application of voltage, and horizontal alignment of the liquid crystal molecules can be achieved with application of voltage) without a PI layer provided. The compound (i) thus can be favorably used for aiding the spontaneous orientation of liquid crystal molecules in a liquid crystal composition.

In addition to the above, the inventors also have found that the spontaneous orientation aid for a liquid crystal composition of the embodiment contains the compound having a partial structure represented by the formula (i), and thereby ensures not only the alignment of the liquid crystal molecules, but also the storage stability of the liquid crystal composition.

From the aforementioned points of view, it suffices that the compound contained in the spontaneous orientation aid for a liquid crystal composition of the embodiment has the partial structure represented by the formula (i) at the end of the molecule, preferably at the end of the main chain of the molecule, and the chemical structure of the site, to which the partial structure represented by the formula (i) is bonded, is not particularly limited unless the function of the liquid crystal composition is impaired. The compound contained in the spontaneous orientation aid for a liquid crystal composition of the embodiment may be, for example, a compound represented by the general formula (ii) (which may be hereinafter referred to as a "compound (ii)").

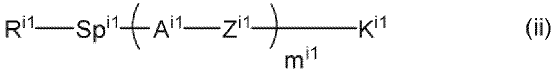

In the formula (ii), Zⁱ¹, Aⁱ¹, mⁱ¹, and Kⁱ¹ have the same meanings of Zⁱ¹, Aⁱ¹, mⁱ¹, and Kⁱ¹ in the formula (i) respectively; Spⁱ¹ represents a linear alkylene group having 1 to 18 carbon atoms, a branched alkylene group having 1 to 18 carbon atoms, or a single bond, in which one or two or more of -CH₂- that are not adjacent to each other in the alkylene group each may be replaced by -O-, -COO-, or -OCO-; and Rⁱ¹ represents a hydrogen atom or a substituent selected from the group consisting of the formulae (R-1) to (R-15): 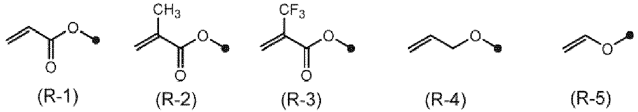 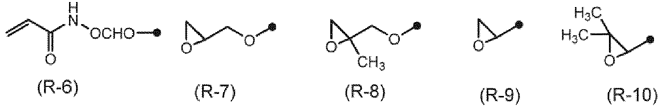 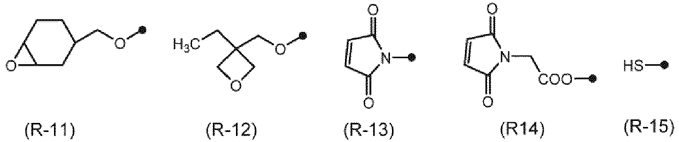 wherein in the formulae (R-1) to (R-15), the black dot on the right end represents a bonding site.

In the formulae (i) and (ii), Zⁱ¹ preferably represents a single bond, a linear or branched alkylene group having 2 to 20 carbon atoms, or a group obtained by replacing one or two or more of -CH₂- that are not adjacent to each other in the alkylene group by -O-, more preferably represents a single bond, a linear alkylene group having 2 to 15 carbon atoms, or a group obtained by replacing one or two or more of -CH₂- that are not adjacent to each other in the alkylene group by -O-, and further preferably represents a single bond, an alkylene group having two carbon atoms (i.e., an ethylene group (-CH₂CH₂-), a group obtained by replacing one of -CH₂- in an ethylene group by -O- (i.e., -CH₂O- or -OCH₂-), an alkylene group having 3 to 13 carbon atoms, or a group obtained by replacing one or two or more of -CH₂- that are not adjacent to each other in the alkylene group by -O-.

Aⁱ¹ preferably represents a divalent 6-membered aromatic group or a divalent 6-membered alicyclic group (in which a hydrogen atom in the cyclic structures may be replaced by a halogen atom), more preferably represents a divalent unsubstituted 6-membered aromatic group, a divalent unsubstituted 6-membered alicyclic group, or a group obtained by replacing a hydrogen atom in the cyclic structures by a fluorine atom, and further preferably represents a divalent unsubstituted 6-membered aromatic group, a group obtained by replacing a hydrogen atom in the cyclic structure by a fluorine atom, or a divalent unsubstituted 6-membered alicyclic group.

mⁱ¹ preferably represents an integer of 1 to 4, and more preferably represents an integer of 1 to 3.

Kⁱ¹ preferably represents a structure represented by any of the formulae (K-1) to (K-5) and (K-8), and more preferably represents a structure represented by any of the formulae (K-1), (K-5), and (K-8).

In the formulae (K-1) to (K-8), W^{K1} preferably represents a methine group. X^{K1} and Y^{K1} each preferably independently represent -CH₂- or an oxygen atom. Z^{K1} preferably represents an oxygen atom. It is preferred that one or both of U^{K1} and V^{K1} represent a nitrogen atom, and it is more preferred that one of U^{K1} and V^{K1} represents a methine group, and the other thereof represents a nitrogen atom. S^{K1} preferably represents a nitrogen atom.

In the formula (ii), Spⁱ¹ preferably represents a linear alkylene group having 1 to 18 carbon atoms or a single bond, more preferably represents a linear alkylene group having 2 to 15 carbon atoms or a single bond, and further preferably represents a linear alkylene group having 3 to 12 carbon atoms or a single bond.

Rⁱ¹ preferably represents a hydrogen atom or a substituent of the formula (R-1) or (R-2).

More specific examples of the compound (i) include compounds represented by any of the following formulae (R-1-1) to (R-1-48), (R-2-1) to (R-2-12), and (R-3-1) to (R-3-11). In the formulae, Rⁱ¹ has the same meaning as Rⁱ¹ in the formula (ii) .

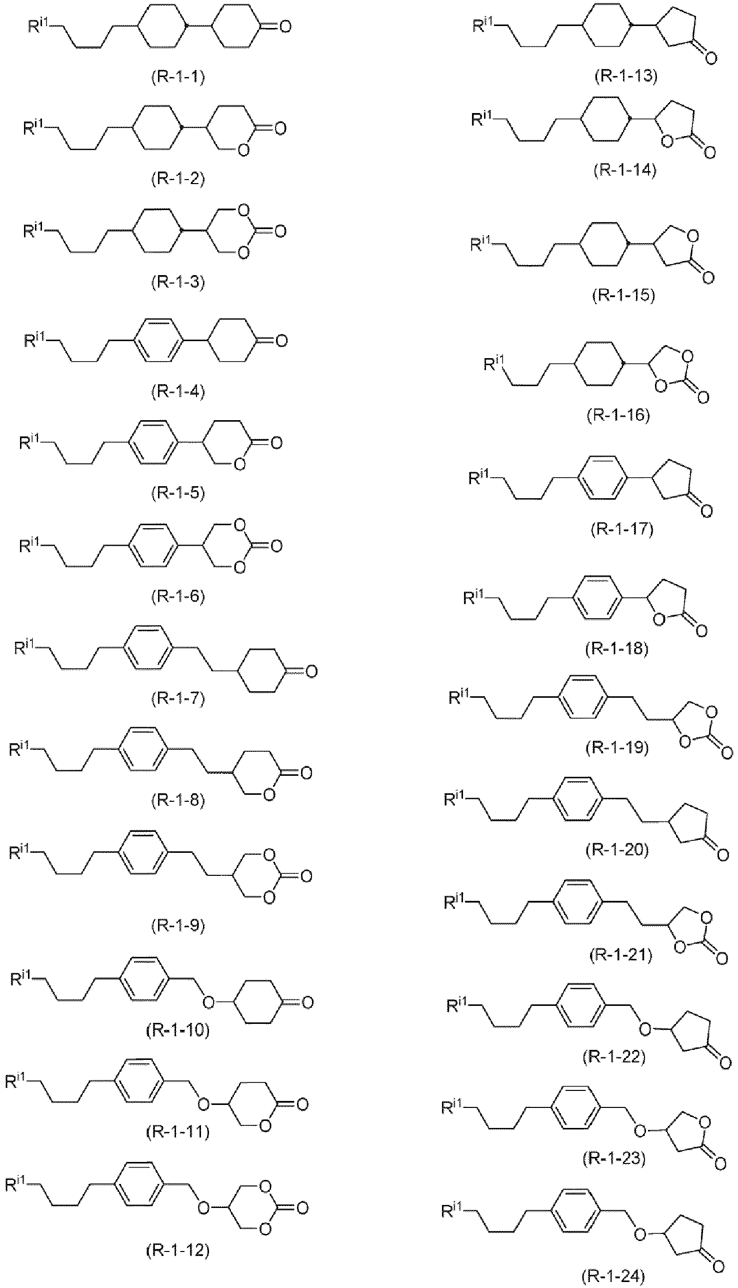

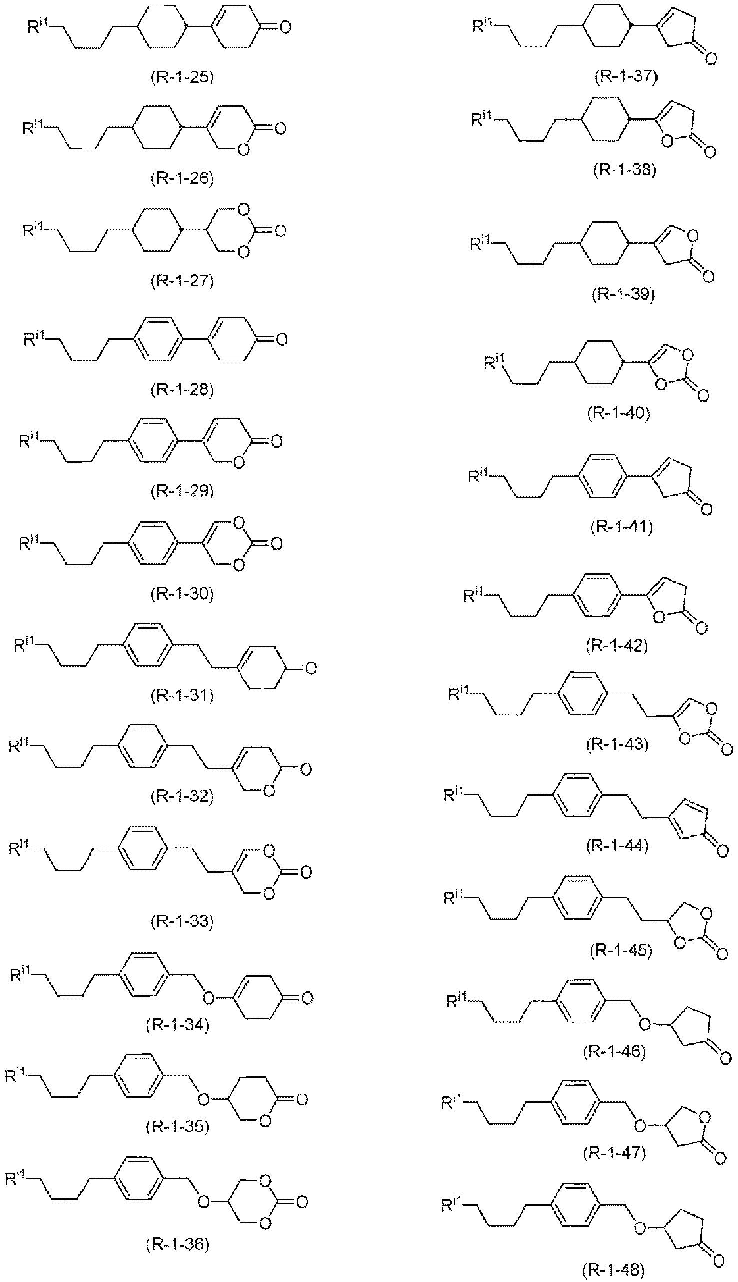

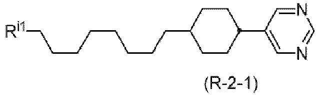

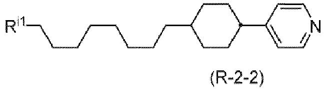

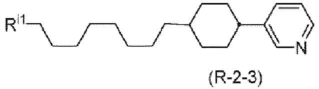

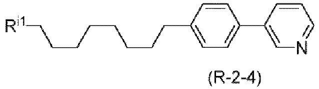

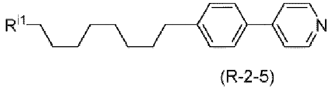

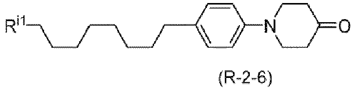

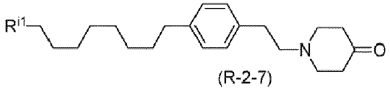

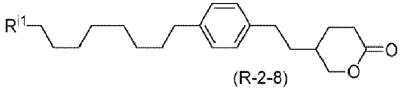

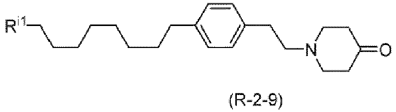

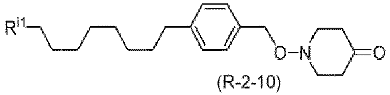

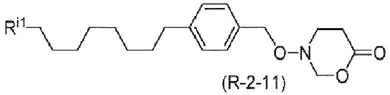

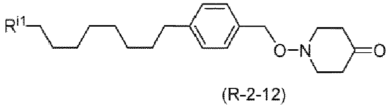

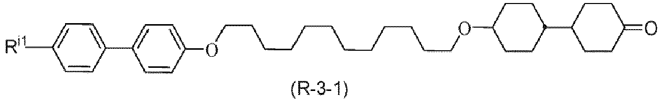

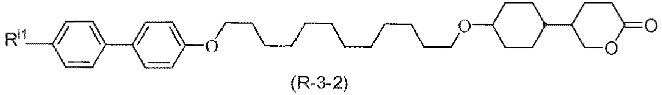

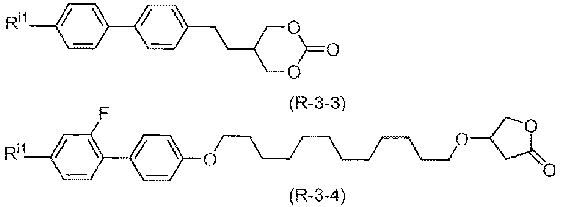

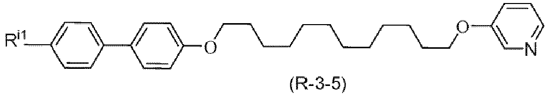

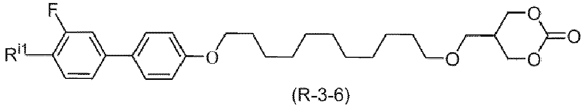

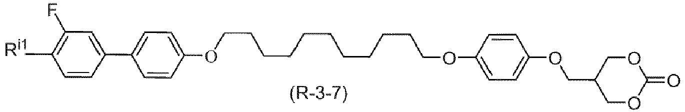

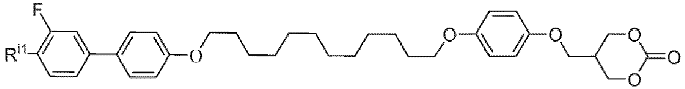

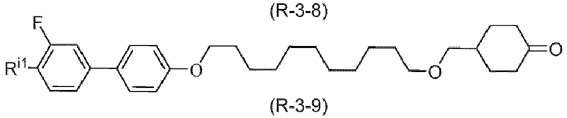

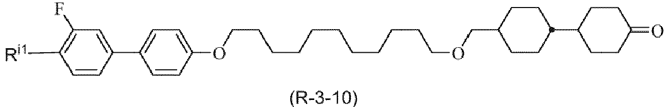

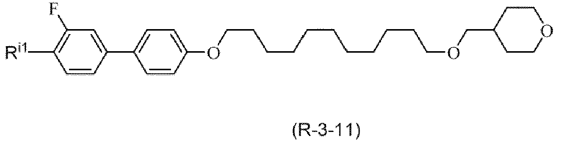

The spontaneous orientation aid for a liquid crystal composition may be formed of one kind or two or more kinds of the compound (i), and may further contain known compounds used in a liquid crystal composition, in addition to the one kind or two or more kinds of the compound (i).

### Liquid Crystal Composition

The liquid crystal composition of one embodiment contains one kind or two or more kinds of a compound having a partial structure represented by the general formula (i). The liquid crystal composition has a negative dielectric anisotropy (Δε). The compound having a partial structure represented by the general formula (i) contained in the liquid crystal composition, which includes the compound (ii) and the compounds represented by any of the formulae (R-1-1) to (R-1-48), (R-2-1) to (R-2-12), and (R-3-1) to (R-3-5) as specific examples thereof, is the same as the compound (i) in the spontaneous orientation aid for a liquid crystal composition, and therefore the explanation thereof is omitted herein.

The content of the compound (i) is preferably from 0.01 to 50% by mass, and the lower limit thereof is preferably 0.01% by mass or more, 0.1% by mass or more, 0.5% by mass or more, 0.7% by mass or more, or 1% by mass or more, based on the total amount of the liquid crystal composition, from the standpoint of further favorable alignment of the liquid crystal molecules. The upper limit of the content of the compound (i) is preferably 50% by mass or less, 30% by mass or less, 10% by mass or less, 7% by mass or less, 5% by mass or less, 4% by mass or less, or 3% by mass or less, based on the total amount of the liquid crystal composition, from the standpoint of excellent response characteristics.

The liquid crystal composition may further contain a compound selected from a group of compounds represented by any of the general formulae (N-1), (N-2), and (N-3).

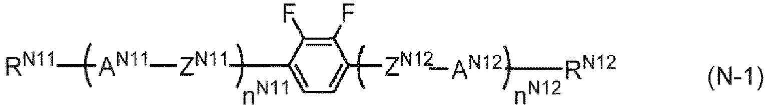

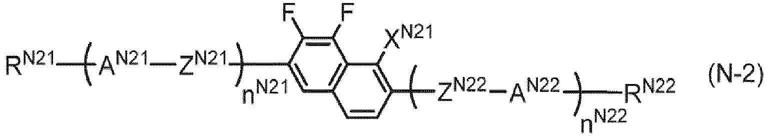

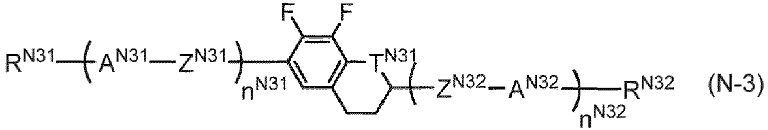

In the formulae (N-1), (N-2), and (N-3), R^{N11}, R^{N12}, R^{N21}, R^{N22}, R^{N31}, and R^{N32} each independently represent an alkyl group having 1 to 8 carbon atoms, in which one or two or more of -CH₂-that are not adjacent to each other in the alkyl group each independently may be replaced by -CH=CH-, -C≡C-, -O-, -CO-, -COO-, or -OCO-; A^{N11}, A^{N12}, A^{N21}, A^{N22}, A^{N31}, and A^{N32} each independently represent a group selected from the group consisting of (a) a 1,4-cyclohexylene group (in which one of -CH₂- or two or more of -CH₂- that are not adjacent to each other present in the group may be replaced by -O-), (b) a 1,4-phenylene group (in which one of -CH= or two or more of -CH= that are not adjacent to each other present in the group may be replaced by -N=), (c) a naphthalen-2,6-diyl group, a 1,2,3,4-tetrahydronaphthalen-2,6-diyl group, or a decahydronaphthalen-2,6-diyl group (in which one of -CH= or two or more of -CH= that are not adjacent to each other present in the naphthalen-2,6-diyl group or the 1,2,3,4-tetrahydronaphthalen-2,6-diyl group may be replaced by -N=), and (d) a 1, 4-cyclohexenylene group, in which the group (a), the group (b), the group (c), and the group (d) each independently may be substituted by a cyano group, a fluorine atom, or a chlorine atom; Z^{N11}, Z^{N12}, Z^{N21}, Z^{N22}, Z^{N31}, and Z^{N32} each independently represent a single bond, -CH₂CH₂-, -(CH₂)₄-, -OCH₂-, -CH₂O-, -COO-, -OCO-, -OCF₂-, -CF₂O-, -CH=N-N=CH-, -CH=CH-, -CF=CF-, or -C≡C-; X^{N21} represents a hydrogen atom or a fluorine atom; T^{N31} represents -CH₂- or an oxygen atom; and n^{N11}, n^{N12}, n^{N21}, n^{N22}, n^{N31}, and n^{N32} each independently represent an integer of 0 to 3, provided that n^{N11}+n^{N12}, n^{N21}+n^{N22}, and n^{N31}+n^{N32} each independently are 1, 2, or 3, in which in the case where a plurality of each of A^{N11} to A^{N32} and Z^{N11} to Z^{N32} are present, these may be the same as or different from each other.

The compound represented by any of the general formulae (N-1), (N-2), and (N-3) is preferably a compound having a negative value for Δε, in which the absolute value thereof is more than 3.

In the general formulae (N-1), (N-2), and (N-3), R^{N11}, R^{N12}, R^{N21}, R^{N22}, R^{N31}, and R^{N32} each independently preferably represent an alkyl group having 1 to 8 carbon atoms, an alkoxy group having 1 to 8 carbon atoms, an alkenyl group having 2 to 8 carbon atoms, or an alkenyloxy group having 2 to 8 carbon atoms, preferably represent an alkyl group having 1 to 5 carbon atoms, an alkoxy group having 1 to 5 carbon atoms, an alkenyl group having 2 to 5 carbon atoms, or an alkenyloxy group having 2 to 5 carbon atoms, further preferably represent an alkyl group having 1 to 5 carbon atoms or an alkenyl group having 2 to 5 carbon atoms, still further preferably represent an alkyl group having 2 to 5 carbon atoms or an alkenyl group having 2 to 3 carbon atoms, and particularly preferably represent an alkenyl group having three carbon atoms (i.e., a propenyl group) .

Moreover, in the case where the cyclic structure, to which the group is bonded, is a phenyl group (i.e., an aromatic group), the group preferably represents a linear alkyl group having 1 to 5 carbon atoms, a linear alkoxy group having 1 to 4 carbon atoms, or an alkenyl group having 4 to 5 carbon atoms, and in the case where the cyclic structure, to which the group is bonded, is a saturated cyclic structure, such as cyclohexane, pyrane, or dioxane, the group preferably represents a linear alkyl group having 1 to 5 carbon atoms, a linear alkoxy group having 1 to 4 carbon atoms, or a linear alkenyl group having 2 to 5 carbon atoms. For stabilizing the nematic phase, the total number of the carbon atom and the oxygen atom if any is preferably 5 or less, and the group is preferably linear.

The alkenyl group is preferably selected from groups represented by any of the formulae (R1) to (R5) (wherein the black dot represents a bonding site).

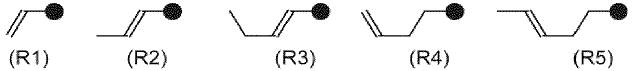

In the case where Δn is demanded to be increased, A^{N11}, A^{N12}, A^{N21}, A^{N22}, A^{N31}, and A^{N32} each independently preferably represent an aromatic group, and in the case where the response speed is demanded to be increased, each independently preferably represent an aliphatic group, preferably represent a trans-1,4-cyclohexylene group, a 1,4-phenylene group, a 2-fluoro-1,4-phenylene group, a 3-fluoro-1,4-phenylene group, a 3,5-difluoro-1,4-phenylene group, a 2,3-difluoro-1,4-phenylene group, a 1,4-cyclohexenylene group, a 1,4-bicyclo[2.2.2]octylene group, a pyperidin-1,4-diyl group, a naphthalen-2,6-diyl group, a decahydronaphthalen-2,6-diyl group, or a 1,2,3,4-tetrahydronaphthalen-2,6-diyl group, more preferably represent the following structures: 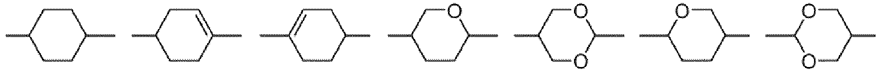 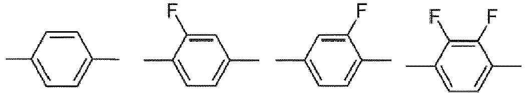 and more preferably represent a trans-1,4-cyclohexylene group, a 1,4-cyclohexenylene group, or a 1,4-phenylene group.

Z^{N11}, Z^{N12}, Z^{N21}, Z^{N22}, Z^{N31}, and Z^{N32} each independently preferably represent -CH₂O-, -CF₂O-, -CH₂CH₂-, -CF₂CF₂-, or a single bond, more preferably represent -CH₂O-, -CH₂CH₂-, or a single bond, and particularly preferably -CH₂O- or a single bond.

X^{N21} preferably represents a fluorine atom.

T^{N31} preferably represents an oxygen atom.

n^{N11}+n^{N12}, n^{N21}+n^{N22}, and n^{N31}+n^{N32} each independently are preferably 1 or 2, and a combination where n^{N11} is 1, and n^{N12} is 0, a combination where n^{N11} is 2, and n^{N12} is 0, a combination where n^{N11} is 1, and n^{N12} is 1, a combination where n^{N11} is 2, and n^{N12} is 1, a combination where n^{N21} is 1, and n^{N22} is 0, a combination where n^{N21} is 2, and n^{N22} is 0, a combination where n^{N31} is 1, and n^{N32} is 0, and a combination where n^{N31} is 2, and n^{N32} is 0 are preferred.

Based on the total amount of the composition of the embodiment, the lower limit of the content of the compound represented by the formula (N-1) may be 1% by mass or more, 10% by mass or more, 20% by mass or more, 30% by mass or more, 40% by mass or more, 50% by mass or more, 55% by mass or more, 60% by mass or more, 65% by mass or more, 70% by mass or more, 75% by mass or more, or 80% by mass or more. The upper limit of the content thereof may be 95% by mass or less, 85% by mass or less, 75% by mass or less, 65% by mass or less, 55% by mass or less, 45% by mass or less, 35% by mass or less, 25% by mass or less, or 20% by mass or less.

Based on the total amount of the composition of the embodiment, the lower limit of the content of the compound represented by the formula (N-2) may be 1% by mass or more, 10% by mass or more, 20% by mass or more, 30% by mass or more, 40% by mass or more, 50% by mass or more, 55% by mass or more, 60% by mass or more, 65% by mass or more, 70% by mass or more, 75% by mass or more, or 80% by mass or more. The upper limit of the content thereof may be 95% by mass or less, 85% by mass or less, 75% by mass or less, 65% by mass or less, 55% by mass or less, 45% by mass or less, 35% by mass or less, 25% by mass or less, or 20% by mass or less.

Based on the total amount of the composition of the embodiment, the lower limit of the content of the compound represented by the formula (N-3) may be 1% by mass or more, 10% by mass or more, 20% by mass or more, 30% by mass or more, 40% by mass or more, 50% by mass or more, 55% by mass or more, 60% by mass or more, 65% by mass or more, 70% by mass or more, 75% by mass or more, or 80% by mass or more. The upper limit of the content thereof may be 95% by mass or less, 85% by mass or less, 75% by mass or less, 65% by mass or less, 55% by mass or less, 45% by mass or less, 35% by mass or less, 25% by mass or less, or 20% by mass or less.

In the case where it is demanded that the viscosity of the composition of the embodiment is kept low, and a composition having a high response speed is provided, it is preferred that the lower limit is low, and the upper limit is low. Moreover, in the case where it is demanded that Tni of the composition of the embodiment is kept high, and a composition having good temperature stability is provided, it is preferred that the lower limit is low, and the upper limit is low. Moreover, in the case where it is demanded that the dielectric anisotropy is increased for keeping the driving voltage low, it is preferred that the lower limit is high, and the upper limit is high.

Examples of the compound represented by the general formula (N-1) include the group of compounds represented by the following general formulae (N-1a) to (N-1g): 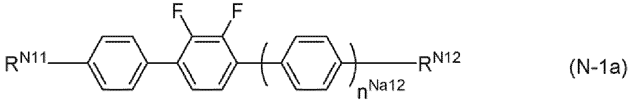 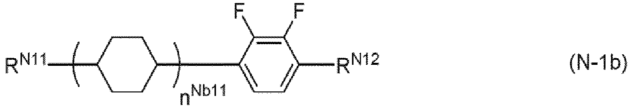 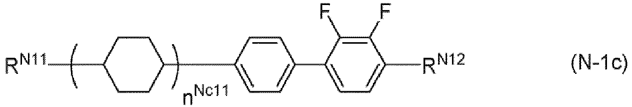 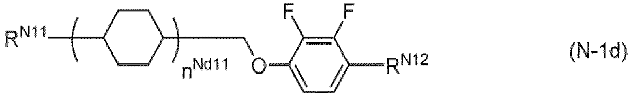 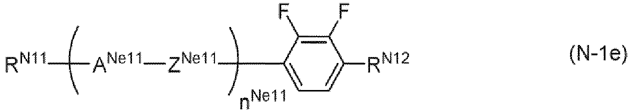 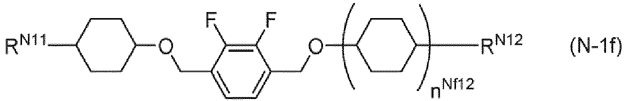 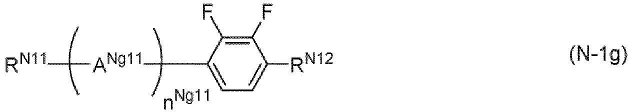 wherein R^{N11} and R^{N12} have the same meanings as R^{N11} and R^{N12} in the general formula (N-1) ; n^{Na11} represents 0 or 1; n^{Nb11} represents 0 or 1; n^{Nc11} represents 0 or 1; n^{Nd11} represents 0 or 1; n^{Ne11} represents 1 or 2; n^{Nf11} represents 1 or 2; n^{Ng11} represents 1 or 2; A^{Ne11} represents a trans-1, 4-cyclohexylene group or a 1,4-phenylene group; A^{Ng11} represents a trans-1,4-cyclohexylene group, a 1,4-cyclohexenylene group, or a 1,4-phenylene group, provided that at least one thereof represents a 1,4-cyclohexenylene group; and Z^{Ne11} represents a single bond or ethylene, provided that at least one thereof represents ethylene.

More specifically, the compound represented by the general formula (N-1) is preferably a compound selected from the group of compounds represented by the general formulae (N-1-1) to (N-1-21).

The compound represented by the general formula (N-1-1) is the following compound: 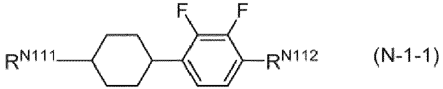 wherein R^{N111} and R^{N112} each independently have the same meanings as R^{N11} and R^{N12} in the general formula (N).

R^{N111} preferably represents an alkyl group having 1 to 5 carbon atoms or an alkenyl group having 2 to 5 carbon atoms, and preferably represents a propyl group, a pentyl group or a vinyl group. R^{N112} preferably represents an alkyl group having 1 to 5 carbon atoms, an alkenyl group having 4 to 5 carbon atoms, or an alkoxy group having 1 to 4 carbon atoms, and preferably represents an ethoxy group or a butoxy group.

The compound represented by the general formula (N-1-1) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a large value; and in the case where T_{NI} is important, a high effect may be obtained by setting the content thereof to a small value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-1) may be 5% by mass or more, 10% by mass or more, 13% by mass or more, 15% by mass or more, 17% by mass or more, 20% by mass or more, 23% by mass or more, 25% by mass or more, 27% by mass or more, 30% by mass or more, 33% by mass or more, or 35% by mass or more. Based on the total amount of the composition of the embodiment, the upper limit of the preferred content thereof may be 50% by mass or less, 40% by mass or less, 38% by mass or less, 35% by mass or less, 33% by mass or less, 30% by mass or less, 28% by mass or less, 25% by mass or less, 23% by mass or less, 20% by mass or less, 18% by mass or less, 15% by mass or less, 13% by mass or less, 10% by mass or less, 8% by mass or less, 7% by mass or less, 6% by mass or less, 5% by mass or less, or 3% by mass or less.

Moreover, the compound represented by the general formula (N-1-1) is preferably a compound selected from the group of compounds represented by the formulae (N-1-1.1) to (N-1-1.23), preferably compounds represented by the formulae (N-1-1.1) to (N-1-1.4), and preferably compounds represented by the formulae (N-1-1.1) and (N-1-1.3).

| | | | |
|---|---|---|---|
| 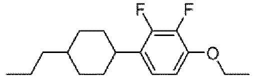 | (N-1-1.1) | 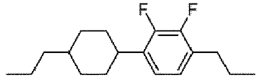 | (N-1-1.11) |
| 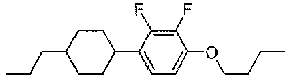 | (N-1-1.2) | 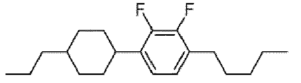 | (N-1-1.12) |
| 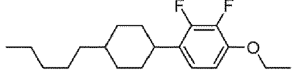 | (N-1-1.3) | 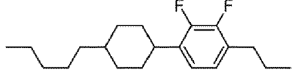 | (N-1-1.13) |
| 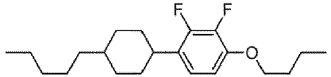 | (N-1-1.4) | 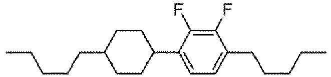 | (N-1-1.14) |
| | | 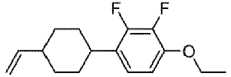 | (N-1-1.20) |
| | | 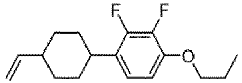 | (N-1-1.21) |
| | | 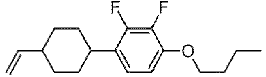 | (N-1-1.22) |

The compounds represented by the formulae (N-1-1.1) to (N-1-1.22) may be used solely or may be used as a combination, and based on the total amount of the composition of the embodiment, the lower limit of the preferred content of the sole compound or the combination of the compounds may be 5% by mass or more, 10% by mass or more, 13% by mass or more, 15% by mass or more, 17% by mass or more, 20% by mass or more, 23% by mass or more, 25% by mass or more, 27% by mass or more, 30% by mass or more, 33% by mass or more, or 35% by mass or more. Based on the total amount of the composition of the embodiment, the upper limit of the preferred content thereof may be 50% by mass or less, 40% by mass or less, 38% by mass or less, 35% by mass or less, 33% by mass or less, 30% by mass or less, 28% by mass or less, 25% by mass or less, 23% by mass or less, 20% by mass or less, 18% by mass or less, 15% by mass or less, 13% by mass or less, 10% by mass or less, 8% by mass or less, 7% by mass or less, 6% by mass or less, 5% by mass or less, or 3% by mass or less.

The compound represented by the general formula (N-1-2) is the following compound: 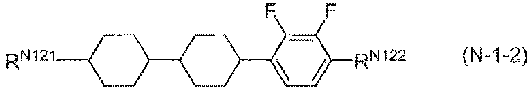 wherein R^{N121} and R^{N122} each independently have the same meanings as R^{N11} and R^{N12} in the general formula (N).

R^{N121} preferably represents an alkyl group having 1 to 5 carbon atoms or an alkenyl group having 2 to 5 carbon atoms, and preferably represents an ethyl group, a propyl group, a butyl group, or a pentyl group. R^{N122} preferably represents an alkyl group having 1 to 5 carbon atoms, an alkenyl group having 4 to 5 carbon atoms, or an alkoxy group having 1 to 4 carbon atoms, and preferably represents a methyl group, a propyl group, a methoxy group, an ethoxy group or a propoxy group.

The compound represented by the general formula (N-1-2) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a small value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-2) may be 5% by mass or more, 7% by mass or more, 10% by mass or more, 13% by mass or more, 15% by mass or more, 17% by mass or more, 20% by mass or more, 23% by mass or more, 25% by mass or more, 27% by mass or more, 30% by mass or more, 33% by mass or more, 35% by mass or more, 37% by mass or more, 40% by mass or more, or 42% by mass or more. Based on the total amount of the composition of the embodiment, the upper limit of the preferred content thereof may be 50% by mass or less, 48% by mass or less, 45% by mass or less, 43% by mass or less, 40% by mass or less, 38% by mass or less, 35% by mass or less, 33% by mass or less, 30% by mass or less, 28% by mass or less, 25% by mass or less, 23% by mass or less, 20% by mass or less, 18% by mass or less, 15% by mass or less, 13% by mass or less, 10% by mass or less, 8% by mass or less, 7% by mass or less, 6% by mass or less, or 5% by mass or less.

Moreover, the compound represented by the general formula (N-1-2) is preferably a compound selected from the group of compounds represented by the formulae (N-1-2.1) to (N-1-2.22), and preferably compounds represented by the formulae (N-1-2.3) to (N-1-2.7), (N-1-2.10), (N-1-2.11), (N-1-2.13), and (N-1-2.20) ; in the case where the improvement of Δε is important, the compounds represented by the formulae (N-1-2.3) to (N-1-2.7) are preferred; in the case where the improvement of T_{NI} is important, the compounds represented by the formulae (N-1-2.10), (N-1-2.11), and (N-1-2.13) are preferred; and in the case where the improvement of the response speed is important, the compound represented by the formula (N-1-2.20) is preferred.

| | | | |
|---|---|---|---|
| 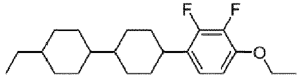 | (N-1-2.1) | 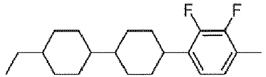 | (N-1-2.10) |
| 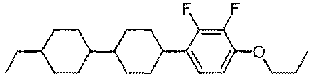 | (N-1-2.2) | 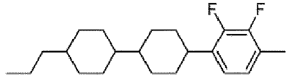 | (N-1-2.11) |
| 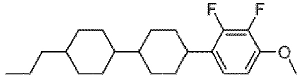 | (N-1-2.3) | 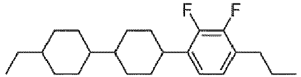 | (N-1-2.12) |
| 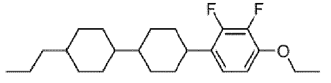 | (N-1-2.4) | 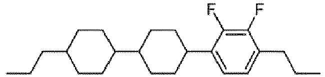 | (N-1-2.13) |
| 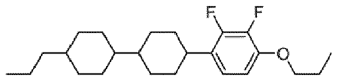 | (N-1-2.5) | 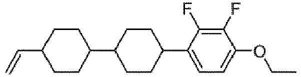 | (N-1-2.20) |
| 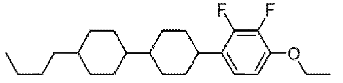 | (N-1-2.6) | 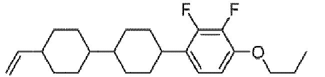 | (N-1-2.21) |
| 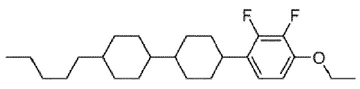 | (N-1-2.7) | 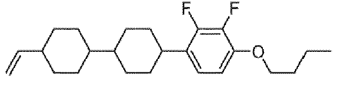 | (N-1-2.22) |

The compounds represented by the formulae (N-1-2.1) to (N-1-2.22) may be used solely or may be used as a combination, and based on the total amount of the composition of the embodiment, the lower limit of the preferred content of the sole compound or the combination of the compounds may be 5% by mass or more, 10% by mass or more, 13% by mass or more, 15% by mass or more, 17% by mass or more, 20% by mass or more, 23% by mass or more, 25% by mass or more, 27% by mass or more, 30% by mass or more, 33% by mass or more, or 35% by mass or more. Based on the total amount of the composition of the embodiment, the upper limit of the preferred content thereof may be 50% by mass or less, 40% by mass or less, 38% by mass or less, 35% by mass or less, 33% by mass or less, 30% by mass or less, 28% by mass or less, 25% by mass or less, 23% by mass or less, 20% by mass or less, 18% by mass or less, 15% by mass or less, 13% by mass or less, 10% by mass or less, 8% by mass or less, 7% by mass or less, 6% by mass or less, 5% by mass or less, or 3% by mass or less.

The compound represented by the general formula (N-1-3) is the following compound: 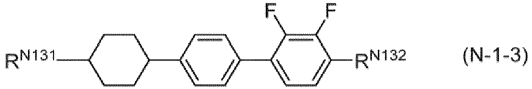 wherein R^{N131} and R^{N132} each independently have the same meanings as R^{N11} and R^{N12} in the general formula (N).

R^{N131} preferably represents an alkyl group having 1 to 5 carbon atoms or an alkenyl group having 2 to 5 carbon atoms, and preferably represents an ethyl group, a propyl group, or a butyl group. R^{N132} preferably represents an alkyl group having 1 to 5 carbon atoms, an alkenyl group having 3 to 5 carbon atoms, or an alkoxy group having 1 to 4 carbon atoms, and preferably represents a 1-propenyl group, an ethoxy group, a propoxy group, or a butoxy group.

The compound represented by the general formula (N-1-3) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-3) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

Moreover, the compound represented by the general formula (N-1-3) is preferably a compound selected from the group of compounds represented by the formulae (N-1-3.1) to (N-1-3.21), preferably compounds represented by the formulae (N-1-3.1) to (N-1-3.7) and (N-1-3.21), and preferably the compounds represented by the formulae (N-1-3.1), (N-1-3.2), (N-1-3.3), (N-1-3.4), and (N-1-3.6).

| | | | |
|---|---|---|---|
| 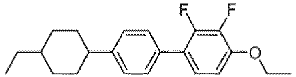 | (N-1-3.1) | 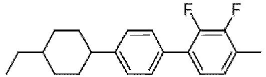 | (N-1-3.10) |
| 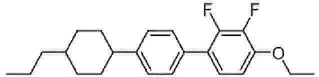 | (N-1-3.2) | 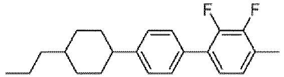 | (N-1-3.11) |
| 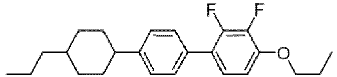 | (N-1-3.3) | 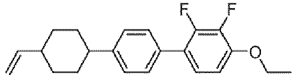 | (N-1-3.20) |
| 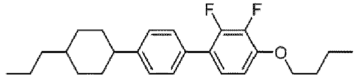 | (N-1-3.4) | 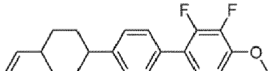 | (N-1-3.21) |
| 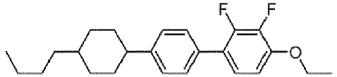 | (N-1-3.5) | | |
| 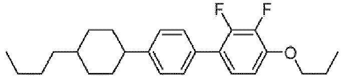 | (N-1-3.6) | | |
| 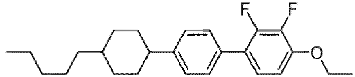 | (N-1-3.7) | | |

The compounds represented by the formulae (N-1-3.1) to (N-1-3.4), (N-1-3.6), and (N-1-3.21) may be used solely or may be used as a combination, and a combination of the formulae (N-1-3.1) and (N-1-3.2), and a combination of two kinds or three kinds selected from the formulae (N-1-3.3), (N-1-3.4), and (N-1-3.6) are preferred. Based on the total amount of the composition of the embodiment, the lower limit of the preferred content of the sole compound or these compounds may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-4) is the following compound: 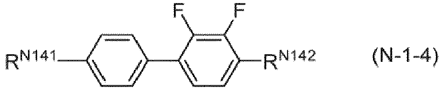 wherein R^{N141} and R^{N142} each independently have the same meanings as R^{N11} and R^{N12} in the general formula (N).

R^{N141} and R^{N142} each independently preferably represent an alkyl group having 1 to 5 carbon atoms, an alkenyl group having 4 to 5 carbon atoms, or an alkoxy group having 1 to 4 carbon atoms, and preferably represent a methyl group, a propyl group, an ethoxy group, or a butoxy group.

The compound represented by the general formula (N-1-4) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a large value; and in the case where T_{NI} is important, a high effect may be obtained by setting the content thereof to a small value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-4) may be 3% by mass or more, 5% by mass or more, 7% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, 13% by mass or less, 11% by mass or less, 10% by mass or less, or 8% by mass or less.

Moreover, the compound represented by the general formula (N-1-4) is preferably a compound selected from the group of compounds represented by the formulae (N-1-4.1) to (N-1-4.14), preferably compounds represented by the formulae (N-1-4.1) to (N-1-4.4), and preferably the compounds represented by the formulae (N-1-4.1), (N-1-4.2), and (N-1-4.4).

| | | | |
|---|---|---|---|
| 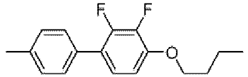 | (N-1-4.1) | 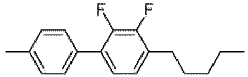 | (N-1-4-11) |
| 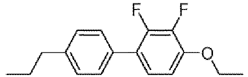 | (N-1-4.2) | 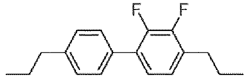 | (N-1-4.12) |
| 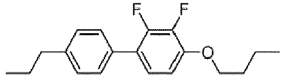 | (N-1-4.3) | 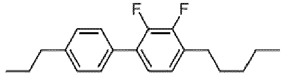 | (N-1-4.13) |
| 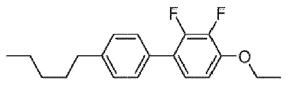 | (N-1-4.4) | 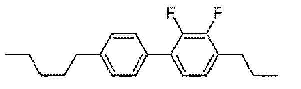 | (N-1-4.14) |

The compounds represented by the formulae (N-1-4.1) to (N-1-4.14) may be used solely or may be used as a combination, and based on the total amount of the composition of the embodiment, the lower limit of the preferred content of the sole compound or these compounds may be 3% by mass or more, 5% by mass or more, 7% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, 13% by mass or less, 11% by mass or less, 10% by mass or less, or 8% by mass or less.

The compound represented by the general formula (N-1-5) is the following compound: 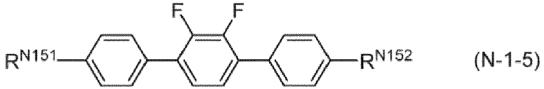 wherein R^{N151} and R^{N152} each independently have the same meanings as R^{N11} and R^{N12} in the general formula (N).

R^{N151} and R^{N152} each independently preferably represent an alkyl group having 1 to 5 carbon atoms, an alkenyl group having 4 to 5 carbon atoms, or an alkoxy group having 1 to 4 carbon atoms, and preferably represent an ethyl group, a propyl group, or a butyl group.

The compound represented by the general formula (N-1-5) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a small value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-5) may be 5% by mass or more, 8% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 33% by mass or less, 30% by mass or less, 28% by mass or less, 25% by mass or less, 23% by mass or less, 20% by mass or less, 18% by mass or less, 15% by mass or less, or 13% by mass or less.

Moreover, the compound represented by the general formula (N-1-5) is preferably a compound selected from the group of compounds represented by the formulae (N-1-5.1) to (N-1-5.6), and preferably compounds represented by the formulae (N-1-5.1), (N-1-5.2), and (N-1-5.4).

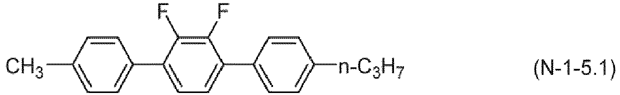

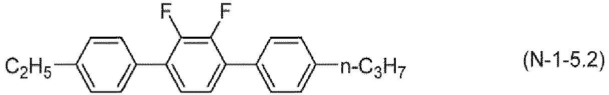

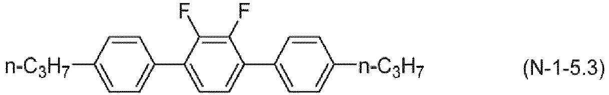

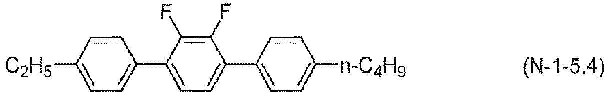

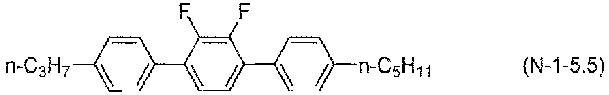

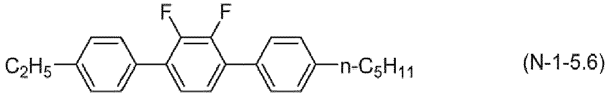

The compounds represented by the formulae (N-1-5.1), (N-1-5.2), and (N-5-1.4) may be used solely or may be used as a combination, and based on the total amount of the composition of the embodiment, the lower limit of the preferred content of the sole compound or these compounds may be 5% by mass or more, 8% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 33% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-10) is the following compound: 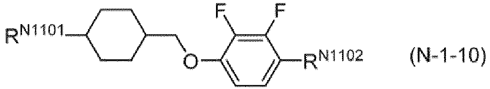 wherein R^{N1101} and R^{N1102} each independently have the same meanings as R^{N11} and R^{N12} in the general formula (N).

R^{N1101} preferably represents an alkyl group having 1 to 5 carbon atoms or an alkenyl group 2 to 5 carbon atoms, and preferably represents an ethyl group, a propyl group, a butyl group, a vinyl group, or a 1-propenyl group. R^{N1102} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-10) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment of the embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-10) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

Moreover, the compound represented by the general formula (N-1-10) is preferably a compound selected from the group of compounds represented by the formulae (N-1-10.1) to (N-1-10.21), preferably compounds represented by the formulae (N-1-10.1) to (N-1-10-5), (N-1-10.20), and (N-1-10.21), and preferably compounds represented by the formulae (N-1-10.1), (N-1-10.2), (N-1-10.20), and (N-1-10.21).

| | | | |
|---|---|---|---|
| 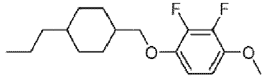 | (N-1-10.1) | 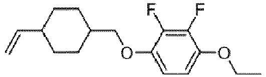 | (N-1-10.11) |
| 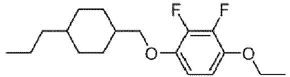 | (N-1-10.2) | 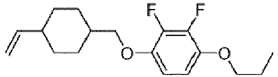 | (N-1-10.12) |
| 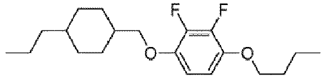 | (N-1-10.3) | 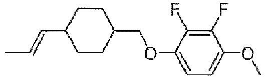 | (N-1-10.13) |
| 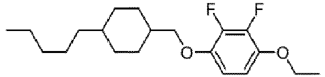 | (N-1-10.4) | 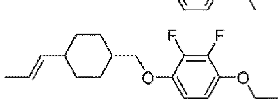 | (N-1-10.14) |
| 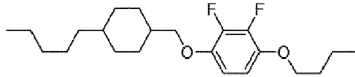 | (N-1-10.5) | | |

The compounds represented by the formulae (N-1-10.1), (N-1-10.2), (N-1-10.20), and (N-1-10.21) may be used solely or may be used as a combination, and based on the total amount of the composition of the embodiment, the lower limit of the preferred content of the sole compound or these compounds may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-11) is the following compound: 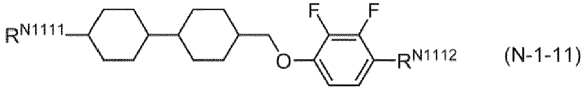 wherein R^{N1111} and R^{N1112} each independently have the same meanings as R^{N11} and R^{N12} in the general formula (N).

R^{N1111} preferably represents an alkyl group having 1 to 5 carbon atoms or an alkenyl group having 2 to 5 carbon atoms, and preferably represents an ethyl group, a propyl group, a butyl group, a vinyl group, or a 1-propenyl group. R^{N1112} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-11) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of Δε is important, the content of the compound is preferably large; in the case where the solubility at a low temperature is important, a high effect may be obtained by setting the content thereof to a small value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-11) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

Moreover, the compound represented by the general formula (N-1-11) is preferably a compound selected from the group of compounds represented by the formulae (N-1-11.1) to (N-1-11.15), preferably compounds represented by the formulae (N-1-11.1) to (N-1-11.15), and preferably compounds represented by the formulae (N-1-11.2) and (N-1-11.4).

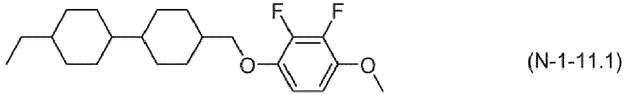

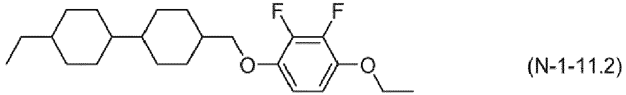

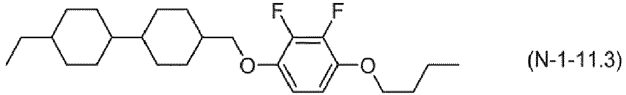

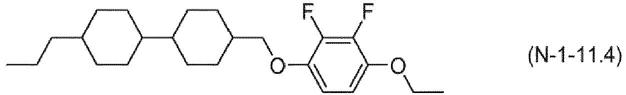

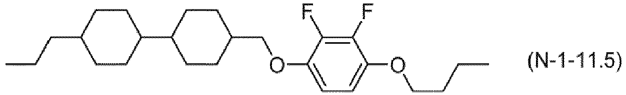

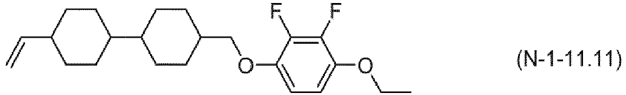

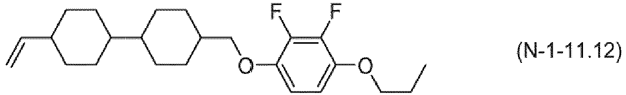

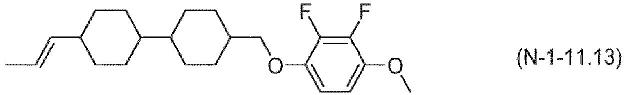

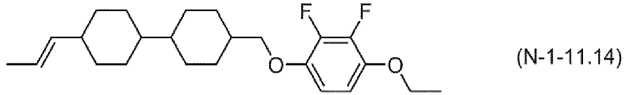

The compounds represented by the formulae (N-1-11.2) and (N-1-11.4) may be used solely or may be used as a combination, and based on the total amount of the composition of the embodiment, the lower limit of the preferred content of the sole compound or these compounds may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-12) is the following compound: 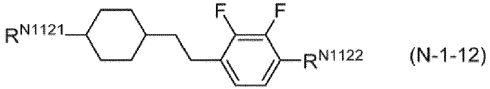 wherein R^{N1121} and R^{N1122} each independently have the same meanings as R^{N11} and R^{N12} in the general formula (N).

R^{N1121} preferably represents an alkyl group having 1 to 5 carbon atoms or an alkenyl group having 2 to 5 carbon atoms, and preferably represents an ethyl group, a propyl group, or a butyl group. R^{NH22} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-12) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-12) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-13) is the following compound: 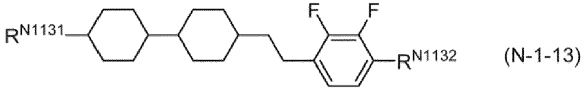 wherein R^{N1131} and R^{N1132} each independently have the same meanings as R^{N11} and R^{N12} in the general formula (N).

R^{N1131} preferably represents an alkyl group having 1 to 5 carbon atoms or an alkenyl group having 2 to 5 carbon atoms, and preferably represents an ethyl group, a propyl group, or a butyl group. R^{N1132} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-13) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-13) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-14) is the following compound: 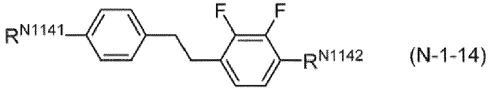 wherein R^{N1141} and R^{N1142} each independently have the same meanings as R^{N11} and R^{N12} in the general formula (N).

R^{N1141} preferably represents an alkyl group having 1 to 5 carbon atoms or an alkenyl group having 2 to 5 carbon atoms, and preferably represents an ethyl group, a propyl group, or a butyl group. R^{N1142} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-14) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment of the embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-14) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-15) is the following compound: 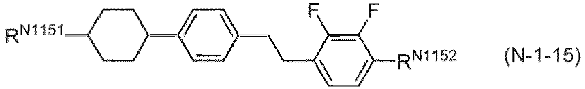 wherein R^{N1151} and R^{N1152} each independently have the same meanings as R^{N11} and R^{N12} in the general formula (N).

R^{N1151} preferably represents an alkyl group having 1 to 5 carbon atoms or an alkenyl group having 2 to 5 carbon atoms, and preferably represents an ethyl group, a propyl group, or a butyl group. R^{N1152} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-15) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-15) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-16) is the following compound: 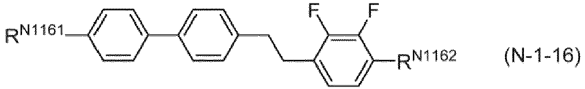 wherein R^{N1161} and R^{N1162} each independently have the same meanings as R^{N11} and R^{N12} in the general formula (N).

R^{N1161} preferably represents an alkyl group having a 1 to 5 carbon atoms or an alkenyl group having 2 to 5 carbon atoms, and preferably represents an ethyl group, a propyl group, or a butyl group. R^{N1162} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-16) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-16) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-17) is the following compound: 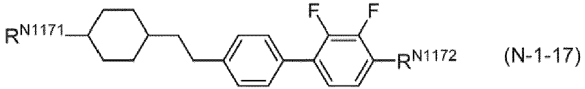 wherein R^{N1171} and R^{N1172} each independently have the same meanings as R^{N11} and R^{N12} in the general formula (N).

R^{N1171} preferably represents an alkyl group having 1 to 5 carbon atoms or an alkenyl group having 2 to 5 carbon atoms, and preferably represents an ethyl group, a propyl group, or a butyl group. R^{N1172} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-17) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-17) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-18) is the following compound: 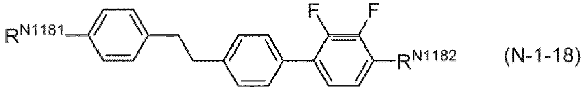 wherein R^{N1181} and R^{N1182} each independently have the same meanings as R^{N11} and R^{N12} in the general formula (N).

R^{N1181} preferably represents an alkyl group having 1 to 5 carbon atoms or an alkenyl group having 2 to 5 carbon atoms, and preferably represents a methyl group, an ethyl group, a propyl group, or a butyl group. R^{N1182} preferably represents an alkyl group having 1 to 5 carbon atoms, an alkenyl group having 4 to 5 carbon atoms, or an alkoxy group having 1 to 4 carbon atoms, and preferably represents an ethoxy group, a propoxy group, or a butoxy group.

The compound represented by the general formula (N-1-18) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-18) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

Moreover, the compound represented by the general formula (N-1-18) is preferably a compound selected from the group of compounds represented by the formulae (N-1-18.1) to (N-1-18.5), preferably compounds represented by the formulae (N-1-18.1) to (N-1-11.3), and preferably compounds represented by the formulae (N-1-18.2) and (N-1-18.3).

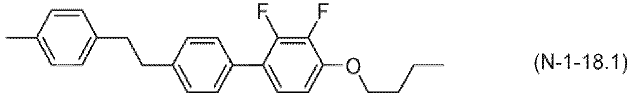

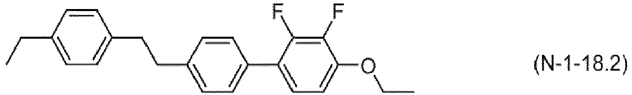

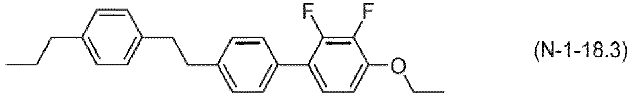

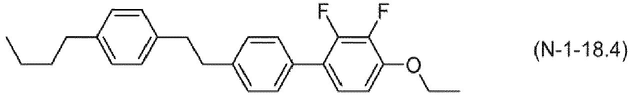

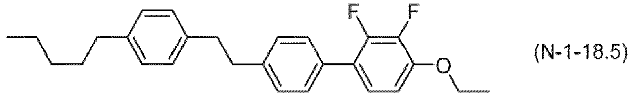

The compound represented by the general formula (N-1-20) is the following compound: 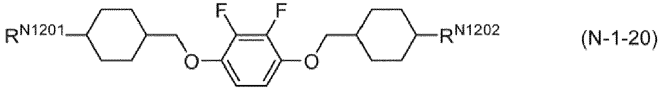 wherein R^{N1201} and R^{N1202} each independently have the same meanings as R^{N11} and R^{N12} in the general formula (N).

R^{N1201} and R^{N1202} each independently preferably represent an alkyl group having 1 to 5 carbon atoms or an alkenyl group having 2 to 5 carbon atoms, and preferably represent an ethyl group, a propyl group, or a butyl group.

The compound represented by the general formula (N-1-20) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-20) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-21) is the following compound: 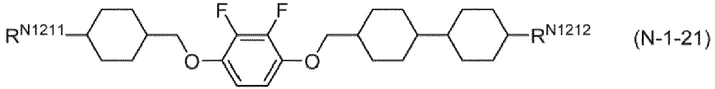 wherein R^{N1211} and R^{N1212} each independently have the same meanings as R^{N11} and R^{N12} in the general formula (N).

R^{N1211} and R^{N1212} each independently preferably represent an alkyl group having 1 to 5 carbon atoms or an alkenyl group having 2 to 5 carbon atoms, and preferably represent an ethyl group, a propyl group, or a butyl group.

The compound represented by the general formula (N-1-21) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-21) may be 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, or 13% by mass or less.

The compound represented by the general formula (N-1-22) is the following compound: 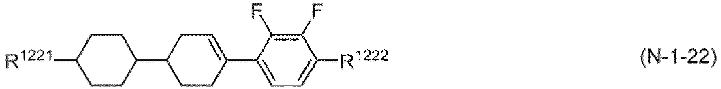 wherein R^{N1221} and R^{N1222} each independently have the same meanings as R^{N11} and R^{N12} in the general formula (N).

R^{N1221} and R^{N1222} each independently preferably represent an alkyl group having 1 to 5 carbon atoms or an alkenyl group having 2 to 5 carbon atoms, and preferably represent an ethyl group, a propyl group, or a butyl group.

The compound represented by the general formula (N-1-22) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a large value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-1-21) may be 1% by mass or more, 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 35% by mass or less, 30% by mass or less, 28% by mass or less, 25% by mass or less, 23% by mass or less, 20% by mass or less, 18% by mass or less, 15% by mass or less, 13% by mass or less, 10% by mass or less, or 5% by mass or less.

Moreover, the compound represented by the general formula (N-1-22) is preferably a compound selected from the group of compounds represented by the formulae (N-1-22.1) to (N-1-22.12), preferably compounds represented by the formulae (N-1-22.1) to (N-1-22.5), and preferably compounds represented by the formulae (N-1-22.1) to (N-1-22.4).

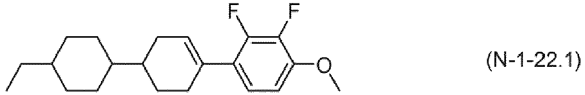

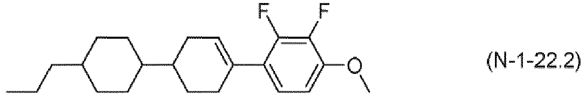

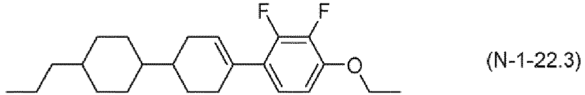

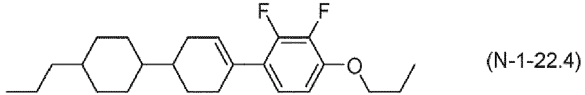

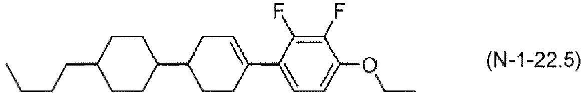

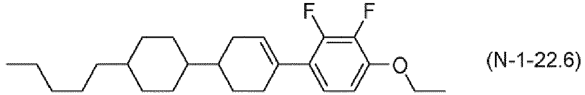

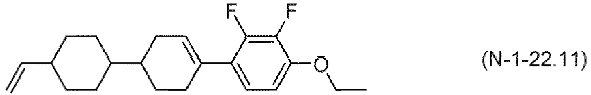

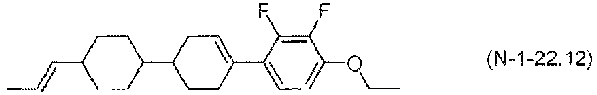

The compound represented by the general formula (N-3) is preferably a compound selected from the group of compounds represented by the general formula (N-3-2): 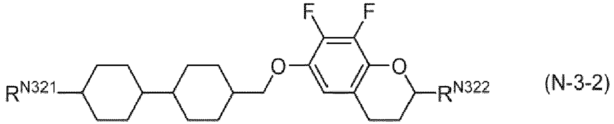 wherein R^{N321} and R^{N322} each independently have the same meanings as R^{N11} and R^{N12} in the general formula (N).

R^{N321} and R^{N322} each preferably represent an alkyl group having 1 to 5 carbon atoms or an alkenyl group having 2 to 5 carbon atoms, and preferably represent a propyl group or a pentyl group.

The compound represented by the general formula (N-3-2) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the improvement of **Δε** is important, the content of the compound is preferably large; in the case where the solubility at a low temperature is important, a high effect may be obtained by setting the content thereof to a large value; and in the case where T_{NI} is important, a high effect may be obtained by setting the content thereof to a small value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (N-3-2) may be 3% by mass or more, 5% by mass or more, 10% by mass or more, 13% by mass or more, 15% by mass or more, 17% by mass or more, 20% by mass or more, 23% by mass or more, 25% by mass or more, 27% by mass or more, 30% by mass or more, 33% by mass or more, or 35% by mass or more. Based on the total amount of the composition of the embodiment, the upper limit of the preferred content thereof may be 50% by mass or less, 40% by mass or less, 38% by mass or less, 35% by mass or less, 33% by mass or less, 30% by mass or less, 28% by mass or less, 25% by mass or less, 23% by mass or less, 20% by mass or less, 18% by mass or less, 15% by mass or less, 13% by mass or less, 10% by mass or less, 8% by mass or less, 7% by mass or less, 6% by mass or less, or 5% by mass or less.

Moreover, the compound represented by the general formula (N-3-2) is preferably a compound selected from the group of compounds represented by the formulae (N-3-2.1) to (N-3-2.3).

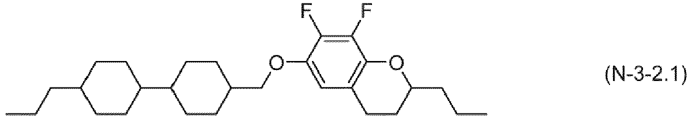

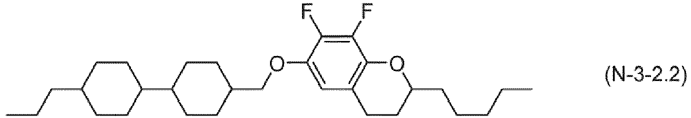

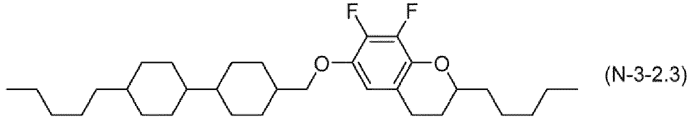

The liquid crystal composition may further contain a compound represented by the general formula (L).

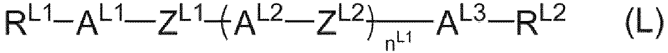

In the formula (L), R^{L1} and R^{L2} each independently represent an alkyl group having 1 to 8 carbon atoms, in which one or two or more of -CH₂- that are not adjacent to each other in the alkyl group each independently may be replaced by -CH=CH-, -C=C-, -O-, -CO-, -COO-, or -OCO-; n^{L1} represents 0, 1, 2, or 3; A^{L1}, A^{L2}, and A^{L3} each independently represent a group selected from the group consisting of (a) a 1,4-cyclohexylene group (in which one of -CH₂- or two or more of -CH₂- that are not adjacent to each other present in the group may be replaced by -0-), (b) a 1,4-phenylene group (in which one of -CH= or two or more of -CH= that are not adjacent to each other present in the group may be replaced by -N=), and (c) a naphthalen-2,6-diyl group, a 1,2,3,4-tetrahydronaphthalen-2,6-diyl group, or a decahydronaphthalen-2,6-diyl group (in which one of -CH= or two or more of -CH= that are not adjacent to each other present in the naphthalen-2,6-diyl group or the 1,2,3,4-tetrahydronaphthalen-2,6-diyl group may be replaced by -N=), and Z^{L1} and Z^{L2} each independently represent a single bond, -CH₂CH₂-, -(CH₂)₄-, -OCH₂-, -CH₂O-, -COO-, -OCO-, -OCF₂-, -CF₂O-, -CH=N-N=CH-, -CH=CH-, -CF=CF-, or -C=C-; in which the group (a), the group (b), and the group (c) each independently may be substituted by a cyano group, a fluorine atom, or a chlorine atom; in the case where n^{L1} is 2 or 3, and a plurality of A^{L2} are present, these may be the same as or different from each other; in the case where n^{L1} is 2 or 3, and a plurality of Z^{L2} are present, these may be the same as or different from each other; and compounds represented by the general formulae (N-1), (N-2), and (N-3) are excluded.

The compound represented by the general formula (L) corresponds to a compound that is dielectrically nearly neutral (i.e., a value of **Δε** of from -2 to 2). The compound represented by the general formula (L) may be used solely or as a combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kind of the compound used may be, for example, one kind in one embodiment. The kinds thereof may be two kinds, three, kinds, four kinds, five kinds, six kinds, seven kinds, eight kinds, nine kinds, or ten kinds in other embodiments.

In the composition of the embodiment, the content of the compound represented by the general formula (L) is necessarily controlled appropriately in accordance with the demanded capabilities, such as the solubility at a low temperature, the transition temperature, the electric reliability, the birefringence, the process adaptability, the drop marks, the burn-in, and the dielectric anisotropy.

Based on the total amount of the composition of the embodiment, the lower limit of the preferred content of the compound represented by the formula (L) may be 1% by mass or more, 10% by mass or more, 20% by mass or more, 30% by mass or more, 40% by mass or more, 50% by mass or more, 55% by mass or more, 60% by mass or more, 65% by mass or more, 70% by mass or more, 75% by mass or more, or 80% by mass or more. Based on the total amount of the composition of the embodiment, the upper limit of the preferred content thereof may be 95% by mass or less, 85% by mass or less, 75% by mass or less, 65% by mass or less, 55% by mass or less, 45% by mass or less, 35% by mass or less, or 25% by mass or less.

In the case where it is demanded that the viscosity of the composition of the embodiment is kept low, and a composition having a high response speed is provided, it is preferred that the lower limit is high, and the upper limit is high. Moreover, in the case where it is demanded that Tni of the composition of the embodiment is kept high, and a composition having good temperature stability is provided, it is preferred that the lower limit is high, and the upper limit is high. Furthermore, in the case where it is demanded that the dielectric anisotropy is increased for keeping the driving voltage low, it is preferred that the lower limit is low, and the upper limit is low.

In the case where the reliability is important, both R^{L1} and R^{L2} are preferably alkyl groups; in the case where the decrease of the volatility of the compound is important, both of them are preferably alkoxy group; and in the case where the decrease of the viscosity is important, at least one of them is preferably an alkenyl group.

The number of a halogen atom present in the molecule is preferably 0, 1, 2, or 3, preferably 0 or 1, and in the case where the compatibility with the other liquid crystal molecules is important, preferably 1.

In the case where the cyclic structure, to which R^{L1} or R^{L2} is bonded, is a phenyl group (i.e., an aromatic group), the group preferably represents a linear alkyl group having 1 to 5 carbon atoms, a linear alkoxy group having 1 to 4 carbon atoms, or an alkenyl group having 4 to 5 carbon atoms, and in the case where the cyclic structure, to which the group is bonded, is a saturated cyclic structure, such as cyclohexane, pyrane, or dioxane, the group preferably represents a linear alkyl group having 1 to 5 carbon atoms, a linear alkoxy group having 1 to 4 carbon atoms, or a linear alkenyl group having 2 to 5 carbon atoms. For stabilizing the nematic phase, the total number of the carbon atom and the oxygen atom if any is preferably 5 or less, and the group is preferably linear.

The alkenyl group is preferably selected from groups represented by any of the formulae (R1) to (R5) (wherein the black dot represents a bonding site).

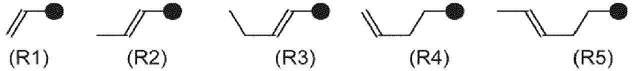

n^{L1} is preferably 0 in the case where the response speed is important, preferably 2 or 3 for improving the upper limit temperature of the nematic phase, and preferably 1 for balancing these factors. Moreover, the compounds having different values are preferably combined for satisfying the characteristics demanded in the composition.

In the case where **Δ**n is demanded to be increased, A^{L1}, A^{L2}, and A^{L3} each preferably represent an aromatic group, and in the case where the response speed is demanded to be increased, each preferably represent an aliphatic group, each independently preferably represent a trans-1,4-cyclohexylene group, a 1,4-phenylene group, a 2-fluoro-1,4-phenylene group, a 3-fluoro-1,4-phenylene group, a 3,5-difluoro-1,4-phenylene group, a 1,4-cyclohexenylene group, a 1,4-bicyclo[2.2.2]octylene group, a pyperidin-1,4-diyl group, a naphthalen-2,6-diyl group, a decahydronaphthalen-2,6-diyl group, or a 1,2,3,4-tetrahydronaphthalen-2,6-diyl group, more preferably represent the following structures: 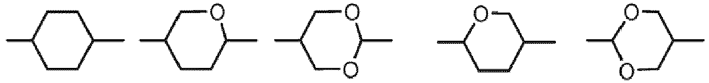 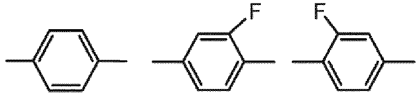 and more preferably represent a trans-1, 4-cyclohexylene group or a 1,4-phenylene group.

Z^{L1} and Z^{L2} are preferably single bond in the case where the response speed is important.

The number of a halogen atom in the molecule of the compound represented by the general formula (L) is preferably 0 or 1.

The compound represented by the general formula (L) is preferably a compound selected from the group of compounds represented by the following general formulae (L-1) to (L-7) .

The compound represented by the general formula (L-1) is the following compound: 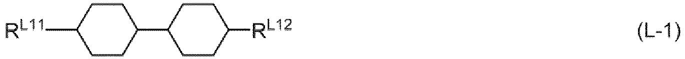 wherein R^{L11} and R^{L12} each independently have the same meanings as R^{L1} and R^{L2} in the general formula (L) .

R^{L11} and R^{L12} each preferably represent a linear alkyl group having 1 to 5 carbon atoms, a linear alkoxy group having 1 to 4 carbon atoms, or an alkenyl group having 2 to 5 carbon atoms.

The compound represented by the general formula (L-1) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

Based on the total amount of the composition of the embodiment, the lower limit of the preferred content of the compound may be 1% by mass or more, 2% by mass or more, 3% by mass or more, 5% by mass or more, 7% by mass or more, 10% by mass or more, 15% by mass or more, 20% by mass or more, 25% by mass or more, 30% by mass or more, 35% by mass or more, 40% by mass or more, 45% by mass or more, 50% by mass or more, or 55% by mass or more. Based on the total amount of the composition of the embodiment, the upper limit of the preferred content thereof may be 95% by mass or less, 90% by mass or less, 85% by mass or less, 80% by mass or less, 75% by mass or less, 70% by mass or less, 65% by mass or less, 60% by mass or less, 55% by mass or less, 50% by mass or less, 45% by mass or less, 40% by mass or less, 35% by mass or less, 30% by mass or less, or 25% by mass or less.

In the case where it is demanded that the viscosity of the composition of the embodiment is kept low, and a composition having a high response speed is provided, it is preferred that the lower limit is high, and the upper limit is high. Moreover, in the case where it is demanded that Tni of the composition of the embodiment is kept high, and a composition having good temperature stability is provided, it is preferred that the lower limit is intermediate, and the upper limit is intermediate. Furthermore, in the case where it is demanded that the dielectric anisotropy is increased for keeping the driving voltage low, it is preferred that the lower limit is low, and the upper limit is low.

The compound represented by the general formula (L-1) is preferably a compound selected from the group of compounds represented by the general formula (L-1-1) : 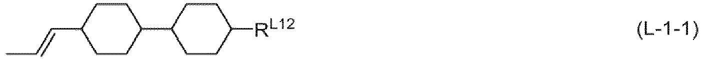 wherein R^{L12} has the same meaning as in the general formula (L).

The compound represented by the general formula (L-1-1) is preferably a compound selected from the group of compounds represented by the formulae (L-1-1.1) to (L-1-1.3), preferably the compounds represented by the formulae (L-1-1.2) or (L-1-1.3), and preferably a compound represented by the formulae (L-1-1.3).

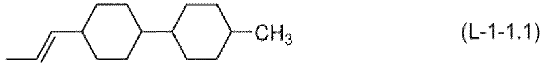

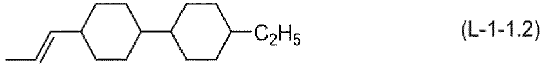

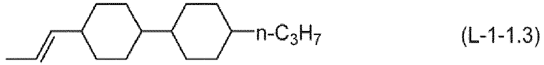

Based on the total amount of the composition of the embodiment, the lower limit of the preferred content of the compound represented by the formula (L-1-1.3) may be 1% by mass or more, 2% by mass or more, 3% by mass or more, 5% by mass or more, 7% by mass or more, or 10% by mass or more. Based on the total amount of the composition of the embodiment, the upper limit of the preferred content thereof may be 20% by mass or less, 15% by mass or less, 13% by mass or less, 10% by mass or less, 8% by mass or less, 7% by mass or less, 6% by mass or less, 5% by mass or less, or 3% by mass or less.

The compound represented by the general formula (L-1) is preferably a compound selected from the group of compounds represented by the general formula (L-1-2) : 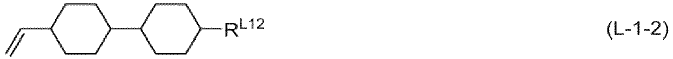 wherein R^{L12} has the same meaning as in the general formula (L-1).

Based on the total amount of the composition of the embodiment, the lower limit of the preferred content of the compound represented by the formula (L-1-2) may be 1% by mass or more, 5% by mass or more, 10% by mass or more, 15% by mass or more, 17% by mass or more, 20% by mass or more, 23% by mass or more, 25% by mass or more, 27% by mass or more, 30% by mass or more, or 35% by mass or more. Based on the total amount of the composition of the embodiment, the upper limit of the preferred content thereof may be 60% by mass or less, 55% by mass or less, 50% by mass or less, 45% by mass or less, 42% by mass or less, 40% by mass or less, 38% by mass or less, 35% by mass or less, 33% by mass or less, or 30% by mass or less.

Moreover, the compound represented by the general formula (L-1-2) is preferably a compound selected from the group of compounds represented by the formulae (L-1-2.1) to (L-1-2.4), and preferably the compounds represented by the formulae (L-1-2.2) to (L-1-2.4). In particular, the compound represented by the formula (L-1-2.2) is preferred for improving the response speed of the composition of the embodiment. Furthermore, in the case where a high Tni is demanded rather than the response speed, the compound represented by the formula (L-1-2.3) or (L-1-2.4) is preferably used. The content of the compounds represented by the formulae (L-1-2.3) and (L-1-2.4) is preferably not 30% by mass or more for improving the solubility at a low temperature.

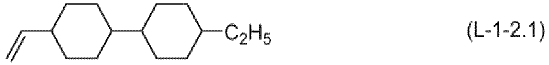

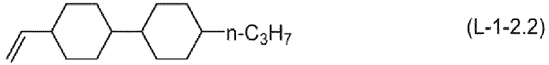

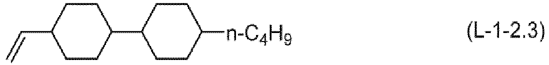

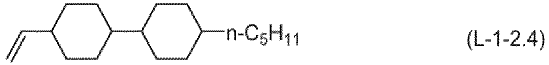

Based on the total amount of the composition of the embodiment, the lower limit of the preferred content of the compound represented by the formula (L-1-2.2) may be 10% by mass or more, 15% by mass or more, 18% by mass or more, 20% by mass or more, 23% by mass or more, 25% by mass or more, 27% by mass or more, 30% by mass or more, 33% by mass or more, 35% by mass or more, 38% by mass or more, or 40% by mass or more. Based on the total amount of the composition of the embodiment, the upper limit of the preferred content thereof may be 60% by mass or less, 55% by mass or less, 50% by mass or less, 45% by mass or less, 43% by mass or less, 40% by mass or less, 38% by mass or less, 35% by mass or less, 32% by mass or less, 30% by mass or less, 27% by mass or less, 25% by mass or less, or 22% by mass or less.

Based on the total amount of the composition of the embodiment, the lower limit of the preferred total content of the compound represented by the formula (L-1-1.3) and the compound represented by the formula (L-1-2.2) may be 10% by mass or more, 15% by mass or more, 20% by mass or more, 25% by mass or more, 27% by mass or more, 30% by mass or more, 35% by mass or more, or 40% by mass or more. Based on the total amount of the composition of the embodiment, the upper limit of the preferred content thereof may be 60% by mass or less, 55% by mass or less, 50% by mass or less, 45% by mass or less, 43% by mass or less, 40% by mass or less, 38% by mass or less, 35% by mass or less, 32% by mass or less, 30% by mass or less, 27% by mass or less, 25% by mass or less, or 22% by mass or less.

The compound represented by the general formula (L-1) is preferably a compound selected from the group of compounds represented by the general formula (L-1-3) : 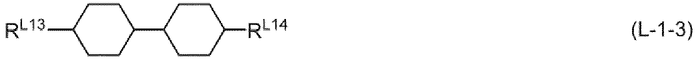 wherein R^{L13} and R^{L14} each independently represent an alkyl group having 1 to 8 carbon atoms or an alkoxy group having 1 to 8 carbon atoms.

R^{L13} and R^{L14} each preferably represent a linear alkyl group having 1 to 5 carbon atoms, a linear alkoxy group having 1 to 4 carbon atoms, or a linear alkenyl group having 2 to 5 carbon atoms.

Based on the total amount of the composition of the embodiment, the lower limit of the preferred content of the compound represented by the formula (L-1-3) may be 1% by mass or more, 5% by mass or more, 10% by mass or more, 13% by mass or more, 15% by mass or more, 17% by mass or more, 20% by mass or more, 23% by mass or more, 25% by mass or more, or 30% by mass or more. Based on the total amount of the composition of the embodiment, the upper limit of the preferred content thereof may be 60% by mass or less, 55% by mass or less, 50% by mass or less, 45% by mass or less, 40% by mass or less, 37% by mass or less, 35% by mass or less, 33% by mass or less, 30% by mass or less, 27% by mass or less, 25% by mass or less, 23% by mass or less, 20% by mass or less, 17% by mass or less, 15% by mass or less, 13% by mass or less, or 10% by mass or less.

Moreover, the compound represented by the general formula (L-1-3) is preferably a compound selected from the group of compounds represented by the formulae (L-1-3.1) to (L-1-3.13), and preferably the compounds represented by the formula (L-1-3.1), (L-1-3.3), or (L-1-3.4). In particular, the compound represented by the formula (L-1-3.1) is preferred for improving the response speed of the composition of the embodiment. Furthermore, in the case where a high Tni is demanded rather than the response speed, the compounds represented by the formulae (L-1-3.3), (L-1-3.4), (L-1-3.11), and (L-1-3.12) are preferably used. The total content of the compounds represented by the formulae (L-1-3.3), (L-1-3.4), (L-1-3.11), and (L-1-3.12) is preferably not 20% or more for improving the solubility at a low temperature.

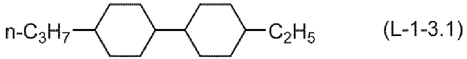

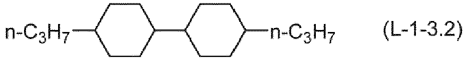

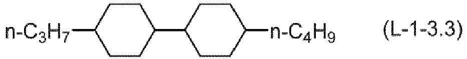

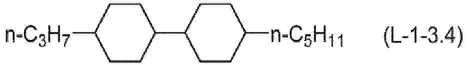

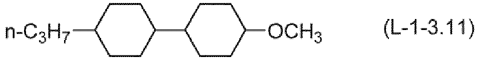

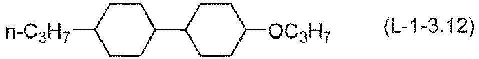

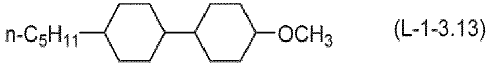

Based on the total amount of the composition of the embodiment, the lower limit of the preferred content of the compound represented by the formula (L-1-3.1) may be 1% by mass or more, 2% by mass or more, 3% by mass or more, 5% by mass or more, 7% by mass or more, 10% by mass or more, 13% by mass or more, 15% by mass or more, 18% by mass or more, or 20% by mass or more. Based on the total amount of the composition of the embodiment, the upper limit of the preferred content thereof may be 20% by mass or less, 17% by mass or less, 15% by mass or less, 13% by mass or less, 10% by mass or less, 8% by mass or less, 7% by mass or less, or 6% by mass or less.

The compound represented by the general formula (L-1) is preferably a compound selected from the group of compounds represented by the general formula (L-1-4) and/or (L-1-5): 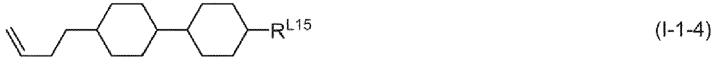 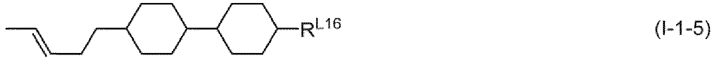 wherein R^{L15} and R^{L16} each independently represent an alkyl group having 1 to 8 carbon atoms or an alkoxy group having 1 to 8 carbon atoms.

R^{L15} and R^{L16} each preferably represent a linear alkyl group having 1 to 5 carbon atoms, a linear alkoxy group having 1 to 4 carbon atoms, or a linear alkenyl group having 2 to 5 carbon atoms.

Based on the total amount of the composition of the embodiment, the lower limit of the preferred content of the compound represented by the formula (L-1-4) may be 1% by mass or more, 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 25% by mass or less, 23% by mass or less, 20% by mass or less, 17% by mass or less, 15% by mass or less, 13% by mass or less, or 10% by mass or less.

Based on the total amount of the composition of the embodiment, the lower limit of the preferred content of the compound represented by the formula (L-1-5) may be 1% by mass or more, 5% by mass or more, 10% by mass or more, 13% by mass or more, 15% by mass or more, 17% by mass or more, or 20% by mass or more. Based on the total amount of the composition of the embodiment, the upper limit of the preferred content thereof may be 25% by mass or less, 23% by mass or less, 20% by mass or less, 17% by mass or less, 15% by mass or less, 13% by mass or less, or 10% by mass or less.

Moreover, the compound represented by the general formulae (L-1-4) and (L-1-5) is preferably a compound selected from the group of compounds represented by the formulae (L-1-4.1) to (L-1-4.3) and (L-1-5.1) to (L-1-5.3), and preferably the compounds represented by the formula (L-1-4.2) or (L-1-5.2).

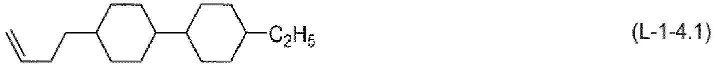

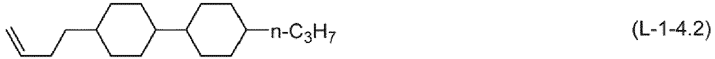

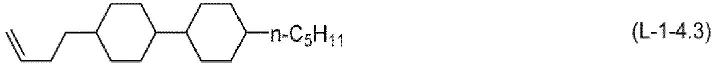

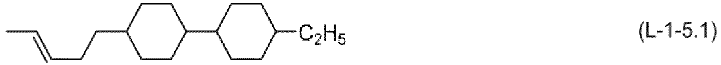

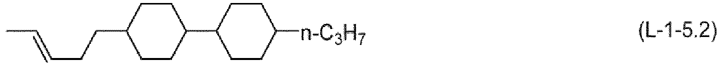

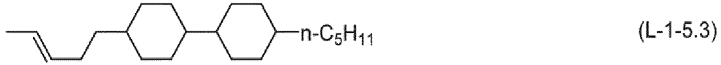

Based on the total amount of the composition of the embodiment, the lower limit of the preferred content of the compound represented by the formula (L-1-4.2) may be 1% by mass or more, 2% by mass or more, 3% by mass or more, 5% by mass or more, 7% by mass or more, 10% by mass or more, 13% by mass or more, 15% by mass or more, 18% by mass or more, or 20% by mass or more. Based on the total amount of the composition of the embodiment, the upper limit of the preferred content thereof may be 20% by mass or less, 17% by mass or less, 15% by mass or less, 13% by mass or less, 10% by mass or less, 8% by mass or less, 7% by mass or less, or 6% by mass or less.

Two or more kinds of compounds selected from the group of compounds represented by the formulae (L-1-1.3), (L-1-2.2), (L-1-3.1), (L-1-3.3), (L-1-3.4), (L-1-3.11), and (L-1-3.12) are preferably combined, and two or more kinds of compounds selected from the group of compounds represented by the formulae (L-1-1.3), (L-1-2.2), (L-1-3.1), (L-1-3.3), (L-1-3.4), and (L-1-4.2) are preferably combined. Based on the total amount of the composition of the embodiment, the lower limit of the preferred total content of these compounds may be 1% by mass or more, 2% by mass or more, 3% by mass or more, 5% by mass or more, 7% by mass or more, 10% by mass or more, 13% by mass or more, 15% by mass or more, 18% by mass or more, 20% by mass or more, 23% by mass or more, 25% by mass or more, 27% by mass or more, 30% by mass or more, 33% by mass or more, or 35% by mass or more. Based on the total amount of the composition of the embodiment, the upper limit of the preferred content thereof may be 80% by mass or less, 70% by mass or less, 60% by mass or less, 50% by mass or less, 45% by mass or less, 40% by mass or less, 37% by mass or less, 35% by mass or less, 33% by mass or less, 30% by mass or less, 28% by mass or less, 25% by mass or less, 23% by mass or less, or 20% by mass or less.

In the case where the reliability of the composition is important, two or more kinds of compounds selected from the group of compounds represented by the formulae (L-1-3.1), (L-1-3.3), and (L-1-3.4) are preferably combined, and in the case where the response speed of the composition is important, two or more kinds of compounds selected from the group of compounds represented by the formulae (L-1-1.3) and (L-1-2.2) are preferably combined.

The compound represented by the general formula (L-1) is preferably a compound selected from the group of compounds represented by the general formula (L-1-6) : 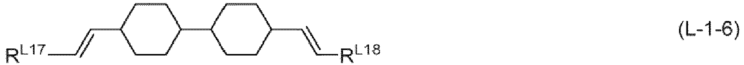 wherein R^{L17} and R^{L18} each independently represent a methyl group or a hydrogen atom.

Based on the total amount of the composition of the embodiment, the lower limit of the preferred content of the compound represented by the formula (L-1-6) may be 1% by mass or more, 5% by mass or more, 10% by mass or more, 15% by mass or more, 17% by mass or more, 20% by mass or more, 23% by mass or more, 25% by mass or more, 27% by mass or more, 30% by mass or more, or 35% by mass or more. Based on the total amount of the composition of the embodiment, the upper limit of the preferred content thereof may be 60% by mass or less, 55% by mass or less, 50% by mass or less, 45% by mass or less, 42% by mass or less, 40% by mass or less, 38% by mass or less, 35% by mass or less, 33% by mass or less, or 30% by mass or less.

Moreover, the compound represented by the general formula (L-1-6) is preferably a compound selected from the group of compounds represented by the formulae (L-1-6.1) to (L-1-6.3).

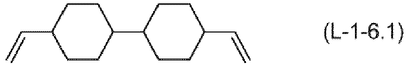

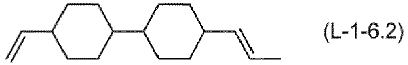

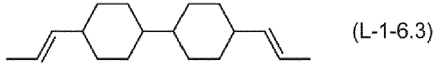

The compound represented by the general formula (L-2) is the following compound: 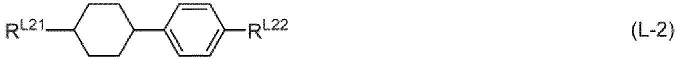 wherein R^{L21} and R^{L22} each independently have the same meanings as R^{L1} and R^{L2} in the general formula (L).

R^{L21} preferably represents an alkyl group having 1 to 5 carbon atoms or an alkenyl group having 2 to 5 carbon atoms, and R^{L22} preferably represents an alkyl group having 1 to 5 carbon atoms, an alkenyl group having 4 to 5 carbon atoms, or an alkoxy group having 1 to 4 carbon atoms.

The compound represented by the general formula (L-1) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the case where the solubility at a low temperature is important, a high effect may be obtained by setting the content thereof to a large value, and on the other hand, in the case where the response speed is important, a high effect may be obtained by setting the content thereof to a small value. Moreover, in the case where the drop marks and the burn-in characteristics are improved, the range of the content thereof is preferably set to the intermediate.

Based on the total amount of the composition of the embodiment, the lower limit of the preferred content of the compound represented by the formula (L-2) may be 1% by mass or more, 2% by mass or more, 3% by mass or more, 5% by mass or more, 7% by mass or more, or 10% by mass or more. Based on the total amount of the composition of the embodiment, the upper limit of the preferred content thereof may be 20% by mass or less, 15% by mass or less, 13% by mass or less, 10% by mass or less, 8% by mass or less, 7% by mass or less, 6% by mass or less, 5% by mass or less, or 3% by mass or less.

Moreover, the compound represented by the general formula (L-2) is preferably a compound selected from the group of compounds represented by the formulae (L-2.1) to (L-2.6), and preferably the compounds represented by the formulae (L-2.1), (L-2.3), (L-2.4), and (L-2.6).

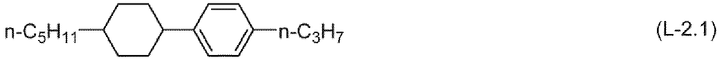

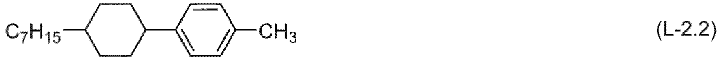

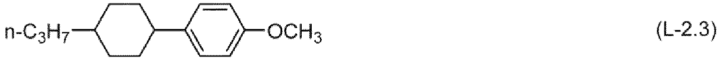

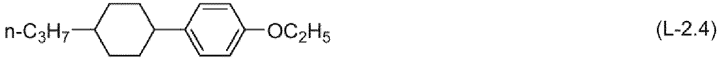

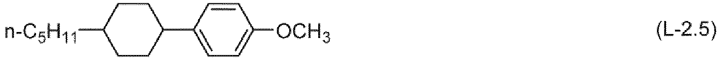

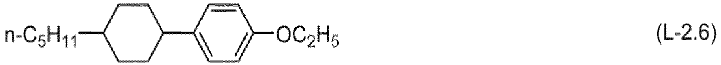

The compound represented by the general formula (L-3) is the following compound: 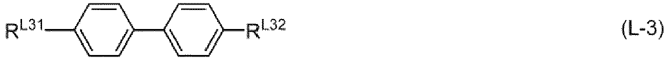 wherein R^{L31} and R^{L32} each independently have the same meanings as R^{L1} and R^{L2} in the general formula (L).

R^{L31} and R^{L32} each preferably represent an alkyl group having 1 to 5 carbon atoms, an alkenyl group having 4 to 5 carbon atoms, or an alkoxy group having 1 to 4 carbon atoms.

The compound represented by the general formula (L-3) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

Based on the total amount of the composition of the embodiment, the lower limit of the preferred content of the compound represented by the formula (L-3) may be 1% by mass or more, 2% by mass or more, 3% by mass or more, 5% by mass or more, 7% by mass or more, or 10% by mass or more. Based on the total amount of the composition of the embodiment, the upper limit of the preferred content thereof may be 20% by mass or less, 15% by mass or less, 13% by mass or less, 10% by mass or less, 8% by mass or less, 7% by mass or less, 6% by mass or less, 5% by mass or less, or 3% by mass or less.

In the case where a high birefringence is obtained, a high effect may be obtained by setting the content thereof to a large value, and on the other hand, in the case where a high Tni is obtained, a high effect may be obtained by setting the content thereof to a small value. Moreover, in the case where the drop marks and the burn-in characteristics are improved, the range of the content thereof is preferably set to the intermediate.

Moreover, the compound represented by the general formula (L-3) is preferably a compound selected from the group of compounds represented by the formulae (L-3.1) to (L-3.7), and preferably the compounds represented by the formulae (L-3.2) to (L-3.7).

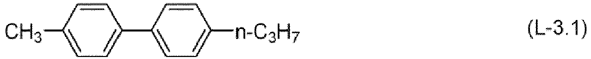

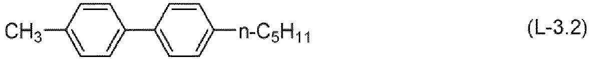

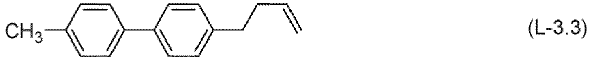

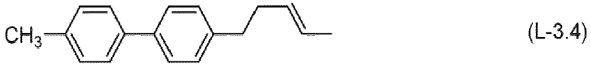

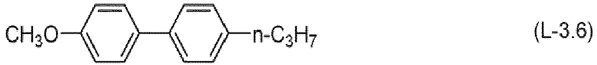

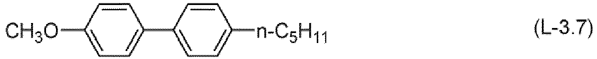

The compound represented by the general formula (L-4) is the following compound: 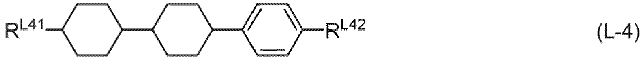 wherein R^{L41} and R^{L42} each independently have the same meanings as R^{L1} and R^{L2} in the general formula (L).

R^{L41} preferably represents an alkyl group having 1 to 5 carbon atoms or an alkenyl group having 2 to 5 carbon atoms, and R^{L42} preferably represents an alkyl group having 1 to 5 carbon atoms, an alkenyl group having 4 to 5 carbon atoms, or an alkoxy group having 1 to 4 carbon atoms.

The compound represented by the general formula (L-4) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the composition of the embodiment, the content of the compound represented by the general formula (L-4) is necessarily controlled appropriately in accordance with the demanded capabilities, such as the solubility at a low temperature, the transition temperature, the electric reliability, the birefringence, the process adaptability, the drop marks, the burn-in, and the dielectric anisotropy.

Based on the total amount of the composition of the embodiment, the lower limit of the preferred content of the compound represented by the formula (L-4) may be 1% by mass or more, 2% by mass or more, 3% by mass or more, 5% by mass or more, 7% by mass or more, 10% by mass or more, 14% by mass or more, 16% by mass or more, 20% by mass or more, 23% by mass or more, 26% by mass or more, 30% by mass or more, 35% by mass or more, or 40% by mass or more. Based on the total amount of the composition of the embodiment, the upper limit of the preferred content of the compound represented by the formula (L-4) may be 50% by mass or less, 40% by mass or less, 35% by mass or less, 30% by mass or less, 20% by mass or less, 15% by mass or less, 10% by mass or less, or 5% by mass or less.

The compound represented by the general formula (L-4) is preferably, for example, the compounds represented by the formulae (L-4.1) to (L-4.3).

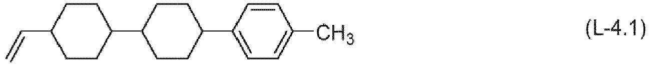

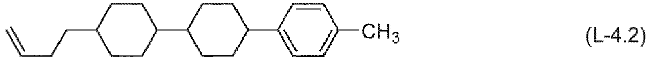

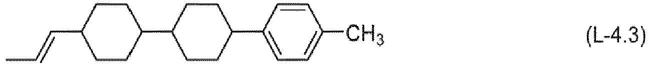

In accordance with the demanded capabilities, such as the solubility at a low temperature, the transition temperature, the electric reliability, and the birefringence, the compound represented by the formula (L-4.1) may be contained, the compound represented by the formula (L-4.2) may be contained, both the compounds represented by the formulae (L-4.1) and (L-4.2) may be contained, and all the compounds represented by the formulae (L-4.1) to (L-4.3) may be contained.

Based on the total amount of the composition of the embodiment, the lower limit of the preferred content of the compound represented by the formula (L-4.1) or (L-4.2) may be 3% by mass or more, 5% by mass or more, 7% by mass or more, 9% by mass or more, 11% by mass or more, 12% by mass or more, 13% by mass or more, 18% by mass or more, or 21% by mass or more. The upper limit of the preferred content thereof may be 45% by mass or less, 40% by mass or less, 35% by mass or less, 30% by mass or less, 25% by mass or less, 23% by mass or less, 20% by mass or less, 18% by mass or less, 15% by mass or less, 13% by mass or less, 10% by mass or less, or 8% by mass or less.

In the case where both the compounds represented by the formulae (L-4.1) and (L-4.2) are contained, based on the total amount of the composition of the embodiment, the lower limit of the preferred content of the compounds may be 15% by mass or more, 19% by mass or more, 24% by mass or more, or 30% by mass or more. The upper limit of the preferred content thereof may be 45% by mass or less, 40% by mass or less, 35% by mass or less, 30% by mass or less, 25% by mass or less, 23% by mass or less, 20% by mass or less, 18% by mass or less, 15% by mass or less, or 13% by mass or less.

The compound represented by the general formula (L-4) is preferably, for example, the compounds represented by the formulae (L-4.4) to (L-4.6), and preferably the compound represented by the formula (L-4.4).

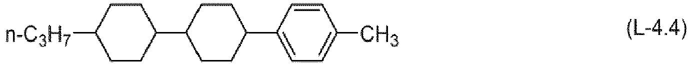

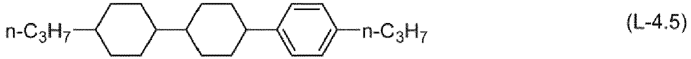

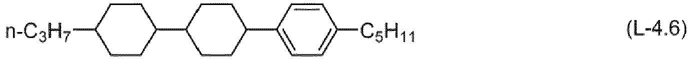

In accordance with the demanded capabilities, such as the solubility at a low temperature, the transition temperature, the electric reliability, and the birefringence, the compound represented by the formula (L-4.4) may be contained, the compound represented by the formula (L-4.5) may be contained, and both the compounds represented by the formulae (L-4.4) and (L-4.5) may be contained.

Based on the total amount of the composition of the embodiment, the lower limit of the preferred content of the compound represented by the formula (L-4.4) or (L-4.5) may be 3% by mass or more, 5% by mass or more, 7% by mass or more, 9% by mass or more, 11% by mass or more, 12% by mass or more, 13% by mass or more, 18% by mass or more, or 21% by mass or more. The preferred upper limit thereof may be 45% by mass or less, 40% by mass or less, 35% by mass or less, 30% by mass or less, 25% by mass or less, 23% by mass or less, 20% by mass or less, 18% by mass or less, 15% by mass or less, 13% by mass or less, 10% by mass or less, or 8% by mass or less.

In the case where both the compounds represented by the formulae (L-4.4) and (L-4.5) are contained, based on the total amount of the composition of the embodiment, the lower limit of the preferred content of the compounds may be 15% by mass or more, 19% by mass or more, 24% by mass or more, or 30% by mass or more. The preferred upper limit thereof may be 45% by mass or less, 40% by mass or less, 35% by mass or less, 30% by mass or less, 25% by mass or less, 23% by mass or less, 20% by mass or less, 18% by mass or less, 15% by mass or less, or 13% by mass or less.

The compound represented by the general formula (L-4) is preferably the compounds represented by the formulae (L-4.7) to (L-4.10), and particularly preferably the compound represented by the formula (L-4.9).

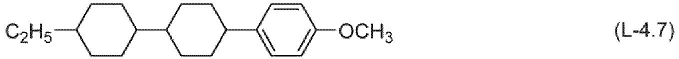

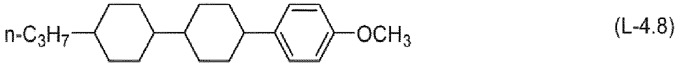

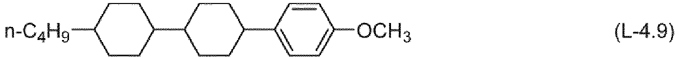

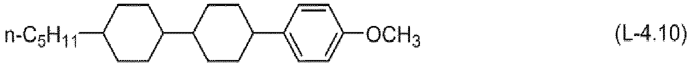

The compound represented by the general formula (L-5) is the following compound: 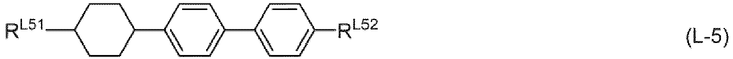 wherein R^{L51} and R^{L52} each independently have the same meanings as R^{L1} and R^{L2} in the general formula (L).

R^{L51} preferably represents an alkyl group having 1 to 5 carbon atoms or an alkenyl group having 2 to 5 carbon atoms, and R^{L52} preferably represents an alkyl group having 1 to 5 carbon atoms, an alkenyl group having 4 to 5 carbon atoms, or an alkoxy group having 1 to 4 carbon atoms.

The compound represented by the general formula (L-5) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

In the composition of the embodiment, the content of the compound represented by the general formula (L-5) is necessarily controlled appropriately in accordance with the demanded capabilities, such as the solubility at a low temperature, the transition temperature, the electric reliability, the birefringence, the process adaptability, the drop marks, the burn-in, and the dielectric anisotropy.

Based on the total amount of the composition of the embodiment, the lower limit of the preferred content of the compound represented by the formula (L-5) may be 1% by mass or more, 2% by mass or more, 3% by mass or more, 5% by mass or more, 7% by mass or more, 10% by mass or more, 14% by mass or more, 16% by mass or more, 20% by mass or more, 23% by mass or more, 26% by mass or more, 30% by mass or more, 35% by mass or more, or 40% by mass or more. Based on the total amount of the composition of the embodiment, the upper limit of the preferred content of the compound represented by the formula (L-5) may be 50% by mass or less, 40% by mass or less, 35% by mass or less, 30% by mass or less, 20% by mass or less, 15% by mass or less, 10% by mass or less, or 5% by mass or less.

The compound represented by the general formula (L-5) is preferably the compounds represented by the formulae (L-5.1) and (L-5.2), and particularly preferably the compound represented by the formula (L-5.1).

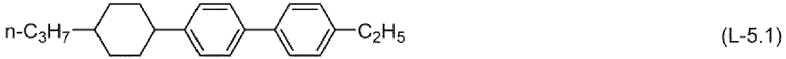

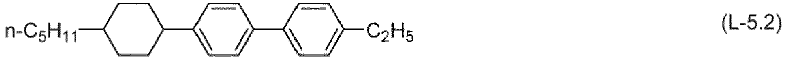

Based on the total amount of the composition of the embodiment, the lower limit of the preferred content of the compounds may be 1% by mass or more, 2% by mass or more, 3% by mass or more, 5% by mass or more, or 7% by mass or more. The upper limit of the preferred content of the compounds may be 20% by mass or less, 15% by mass or less, 13% by mass or less, 10% by mass or less, or 9% by mass or less.

The compound represented by the general formula (L-5) is preferably the compounds represented by the formulae (L-5.3) and (L-5.4).

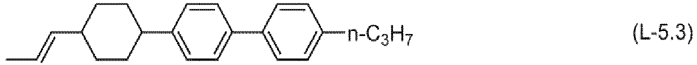

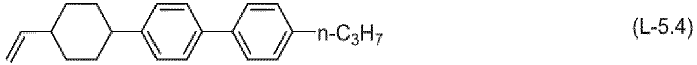

Based on the total amount of the composition of the embodiment, the lower limit of the preferred content of the compounds may be 1% by mass or more, 2% by mass or more, 3% by mass or more, 5% by mass or more, or 7% by mass or more. The upper limit of the preferred content of the compounds may be 20% by mass or less, 15% by mass or less, 13% by mass or less, 10% by mass or less, or 9% by mass or less.

The compound represented by the general formula (L-5) is preferably the compounds represented by the formulae (L-5.5) to (L-5.7), and particularly preferably the compound represented by the formula (L-5.7).

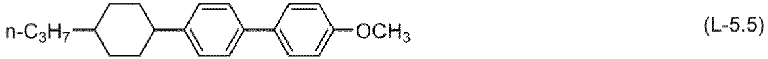

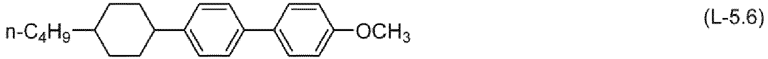

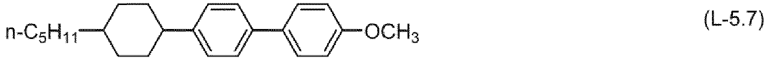

Based on the total amount of the composition of the embodiment, the lower limit of the preferred content of the compounds may be 1% by mass or more, 2% by mass or more, 3% by mass or more, 5% by mass or more, or 7% by mass or more. The upper limit of the preferred content of the compounds may be 20% by mass or less, 15% by mass or less, 13% by mass or less, 10% by mass or less, or 9% by mass or less.

The compound represented by the general formula (L-6) is the following compound: 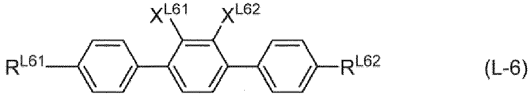 wherein R^{L61} and R^{L62} each independently have the same meanings as R^{L1} and R^{L2} in the general formula (L) ; and X^{L61} and X^{L62} each independently represent a hydrogen atom or a fluorine atom.

R^{L61} and R^{L62} each independently preferably represent an alkyl group having 1 to 5 carbon atoms or an alkenyl group having 2 to 5 carbon atoms, and it is preferred that one of X^{L61} and X^{L62} represents a fluorine atom, and the other thereof represents a hydrogen atom.

The compound represented by the general formula (L-6) may be used solely, or two or more kinds of the compounds may be used in combination. The kinds of the compounds capable of being used in combination are not particularly limited, and the compounds may be used as an appropriate combination in accordance with the demanded capabilities, such as the solubility at a low temperature, the transition temperature, the electric reliability, and the birefringence. The kinds of the compound used may be, for example in one embodiment, one kind, two kinds, three, kinds, four kinds, or five or more kinds.

Based on the total amount of the composition of the embodiment, the lower limit of the preferred content of the compound represented by the formula (L-6) may be 1% by mass or more, 2% by mass or more, 3% by mass or more, 5% by mass or more, 7% by mass or more, 10% by mass or more, 14% by mass or more, 16% by mass or more, 20% by mass or more, 23% by mass or more, 26% by mass or more, 30% by mass or more, 35% by mass or more, or 40% by mass or more. Based on the total amount of the composition of the embodiment, the upper limit of the preferred content of the compound represented by the formula (L-6) may be 50% by mass or less, 40% by mass or less, 35% by mass or less, 30% by mass or less, 20% by mass or less, 15% by mass or less, 10% by mass or less, or 5% by mass or less. The content thereof is preferably large in the case where the increase of Δn is important, and the content thereof is preferably small in the case where the deposition at a low temperature is important.

The compound represented by the general formula (L-6) is preferably the compounds represented by the formulae (L-6.1) to (L-6.9).

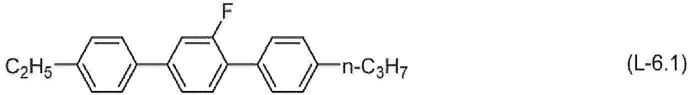

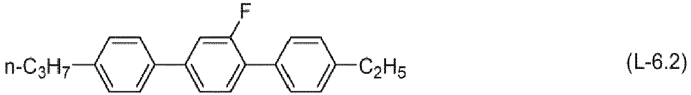

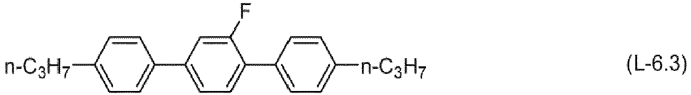

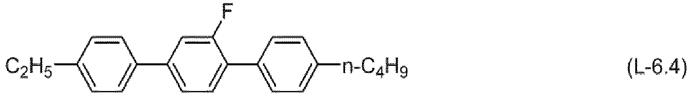

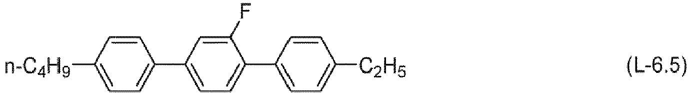

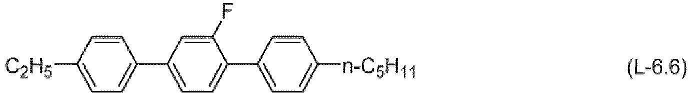

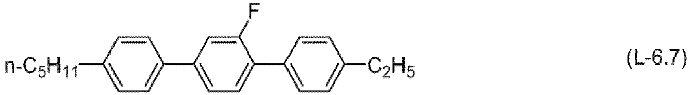

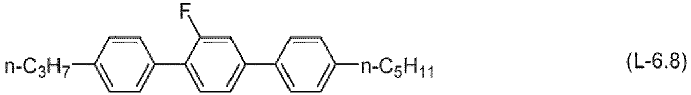

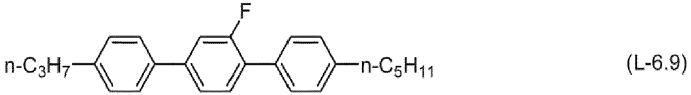

The kinds of the compounds capable of being used in combination are not particularly limited, and from one to three kinds of these compounds are preferably contained, and from one to four kinds thereof are more preferably contained. Moreover, a broad molecular weight distribution of the selected compounds is effective for the solubility, and therefore, for example, one kind selected from the compounds represented by the formula (L-6.1) and (L-6.2), one kind selected from the compounds represented by the formula (L-6.4) and (L-6.5), one kind selected from the compounds represented by the formula (L-6.6) and (L-6.7), and one kind selected from the compounds represented by the formula (L-6.8) and (L-6.9) are preferably appropriately combined. Among these, the compounds represented by the formulae (L-6.1), (L-6.3), (L-6.4), (L-6.6), and (L-6.9) are preferably contained.

Moreover, the compound represented by the general formula (L-6) is preferably the compounds represented by the formulae (L-6.10) to (L-6.17), and particularly preferably the compound represented by the formula (L-6.11).

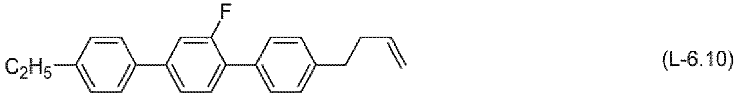

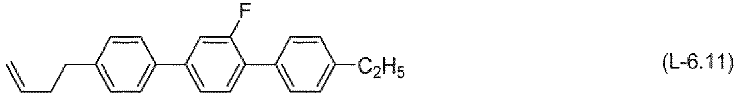

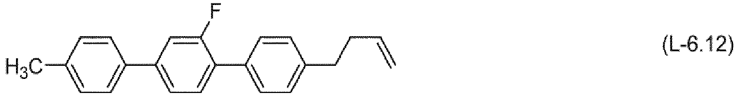

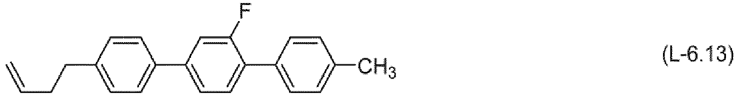

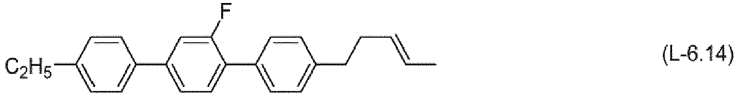

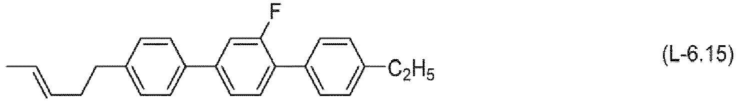

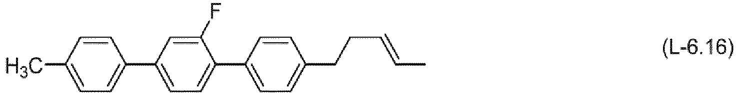

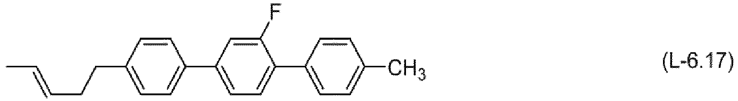

Based on the total amount of the composition of the embodiment, the lower limit of the preferred content of the compounds may be 1% by mass or more, 2% by mass or more, 3% by mass or more, 5% by mass or more, or 7% by mass or more. The upper limit of the preferred content of the compounds may be 20% by mass or less, 15% by mass or less, 13% by mass or less, 10% by mass or less, or 9% by mass or less.

The compound represented by the general formula (L-7) is the following compound: 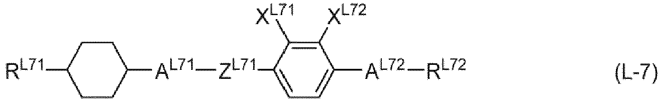 wherein R^{L71} and R^{L72} each independently have the same meanings as R^{L1} and R^{L2} in the general formula (L) ; A^{L71} and A^{L72} each independently have the same meanings as A^{L2} and A^{L3} in the general formula (L), in which hydrogen atoms on A^{L71} and A^{L72} each independently may be replaced by a fluorine atom; Z^{L71} has the same meaning as Z^{L2} in the general formula (L) ; and X^{L71} and X^{L72} each independently represent a hydrogen atom or a fluorine atom.

In the formula, R^{L71} and R^{L72} each independently preferably represent an alkyl group having 1 to 5 carbon atoms, an alkenyl group having 2 to 5 carbon atoms, or an alkoxy group having 1 to 4 carbon atoms; A^{L71} and A^{L72} each independently preferably represent a 1, 4-cyclohexylene group or a 1,4-phenylene group, in which hydrogen atoms on A^{L71} and A^{L72} each independently may be replaced by a fluorine atom; Z^{L71} preferably represents a single bond or COO-, and preferably a single bond; and X^{L71} and X^{L72} each preferably represent a hydrogen atom.

The kinds of the compounds capable of being used in combination are not particularly limited, and the compounds may be appropriately combined in accordance with the demanded capabilities, such as the solubility at a low temperature, the transition temperature, the electric reliability, and the birefringence. The kinds of the compound used may be, for example in one embodiment, one kind, two kinds, three, kinds, or four kinds.

In the composition of the embodiment, the content of the compound represented by the general formula (L-7) is necessarily controlled appropriately in accordance with the demanded capabilities, such as the solubility at a low temperature, the transition temperature, the electric reliability, the birefringence, the process adaptability, the drop marks, the burn-in, and the dielectric anisotropy.

Based on the total amount of the composition of the embodiment, the lower limit of the preferred content of the compound represented by the formula (L-7) may be 1% by mass or more, 2% by mass or more, 3% by mass or more, 5% by mass or more, 7% by mass or more, 10% by mass or more, 14% by mass or more, 16% by mass or more, or 20% by mass or more. Based on the total amount of the composition of the embodiment, the upper limit of the preferred content of the compound represented by the formula (L-7) may be 30% by mass or less, 25% by mass or less, 23% by mass or less, 20% by mass or less, 18% by mass or less, 15% by mass or less, 10% by mass or less, or 5% by mass or less.

In the case where an embodiment where the composition of the embodiment has a high Tni is demanded, the content of the compound represented by the formula (L-7) is preferably large, and in the case where an embodiment with a low viscosity is demanded, the content thereof is preferably small.

Moreover, the compound represented by the general formula (L-7) is preferably the compounds represented by the formulae (L-7.1) to (L-7.4), and particularly preferably the compound represented by the formula (L-7.2).

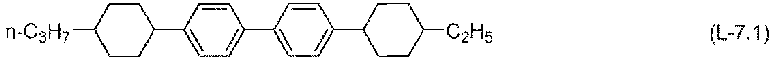

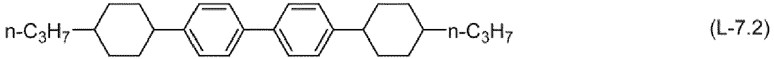

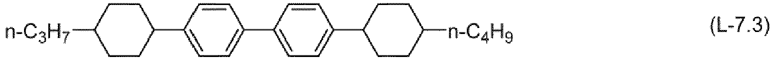

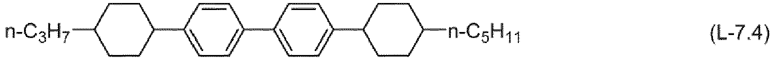

Moreover, the compound represented by the general formula (L-7) is preferably the compounds represented by the formulae (L-7.11) to (L-7.13), and particularly preferably the compound represented by the formula (L-7.11).

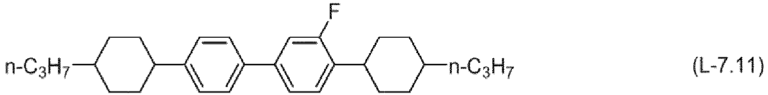

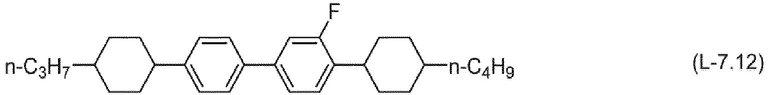

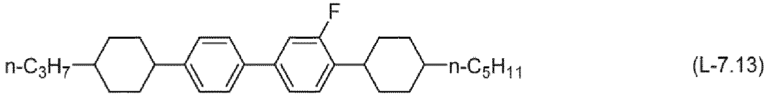

Moreover, the compound represented by the general formula (L-7) is preferably the compounds represented by the formulae (L-7.21) to (L-7.23), and particularly preferably the compound represented by the formula (L-7.21).

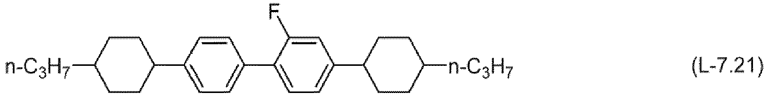

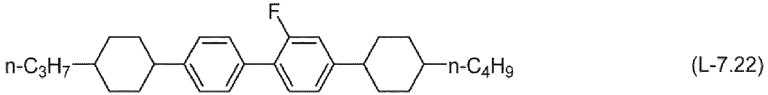

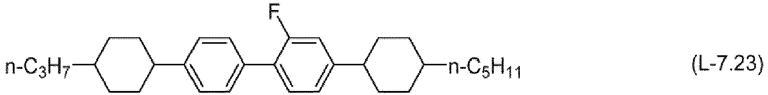

Moreover, the compound represented by the general formula (L-7) is preferably the compounds represented by the formulae (L-7.31) to (L-7.34), and particularly preferably the compound represented by the formula (L-7.31) and/or (L-7.32) .

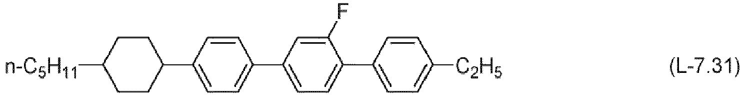

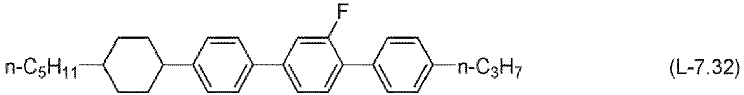

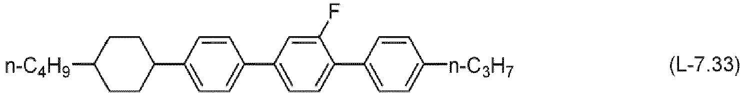

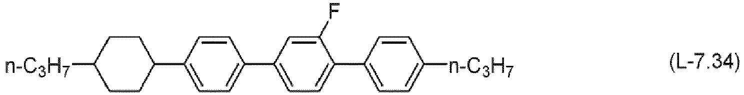

Moreover, the compound represented by the general formula (L-7) is preferably the compounds represented by the formulae (L-7.41) to (L-7.44), and particularly preferably the compound represented by the formula (L-7.41) and/or (L-7.42) .

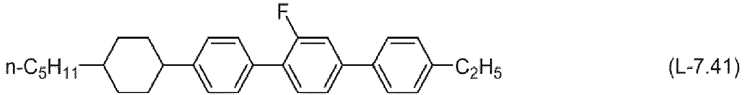

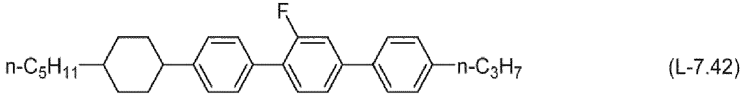

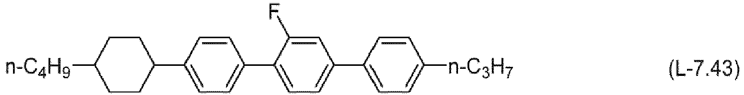

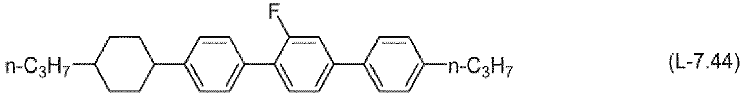

Moreover, the compound represented by the general formula (L-7) is preferably the compounds represented by the formulae (L-7.51) to (L-7.53).

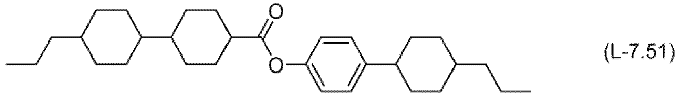

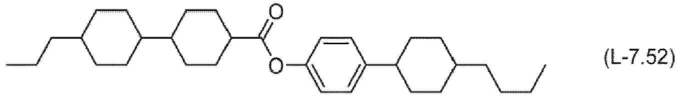

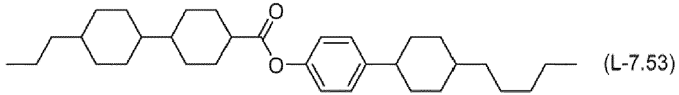

The liquid crystal composition may further contain a polymerizable compound. The polymerizable compound may be a known polymerizable compound having been used in a liquid crystal composition. Examples of the polymerizable compound include a compound represented by the general formula (P).

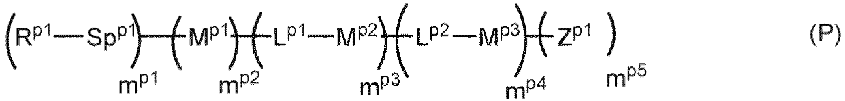

In the formula (P), Z^{p1} represents a fluorine atom, a cyano group, a hydrogen atom, an alkyl group having 1 to 15 carbon atoms, in which a hydrogen atom may be replaced by a halogen atom, an alkoxy group having 1 to 15 carbon atoms, in which a hydrogen atom may be replaced by a halogen atom, an alkenyl group having 1 to 15 carbon atoms, in which a hydrogen atom may be replaced by a halogen atom, an alkenyloxy group having 1 to 15 carbon atoms, in which a hydrogen atom may be replaced by a halogen atom, or -Sp^{p2}-R^{p2}; R^{p1} and R^{p2} each represent any of the following formulae (R-I) to (R-IX): 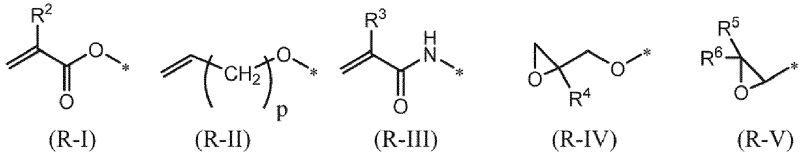 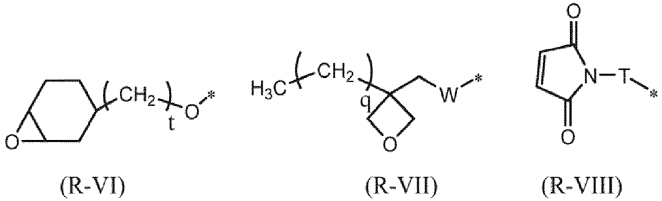 HS-* (R-IX) wherein * represents a bonding site to Sp^{p1}; R² to R⁶ each independently represent a hydrogen atom, an alkyl group having 1 to 5 carbon atoms, or a halogenated alkyl group having 1 to 5 carbon atoms; W represents a single bond, -O-, or a methylene group; T represents a single bond or -COO-; and p, t, and q each independently represent 0, 1, or 2; Sp^{p1} and Sp^{p2} each represent a spacer group; L^{p1} and L^{p2} each independently represent a single bond, -O-, -S-, -CH₂-, -OCH₂-, -CH₂O-, -CO-, -C₂H₄-, -COO-, -OCO-, -OCOOCH₂-, -CH₂OCOO-, -OCH₂CH₂O-, -CO-NR^{a}-, -NR^{a}-CO-, -SCH₂-, -CH₂S-, -CH=CR^{a}-COO-, -CH=CR^{a}-OCO-, -COO-CR^{a}=CH-, -OCO-CR^{a}=CH-, -COO-CR^{a}=CH-COO-, -COO-CR^{a}=CH-OCO-, -OCO-CR^{a}=CH-COO-, -OCO-CR^{a}=CH-OCO-, -(CH₂)_{z}-C(=O)-O-, -(CH₂)-O-(C=O)-, -O-(C=O)-(CH₂)_{z}-, -(C=O)-O-(CH₂)_{z}-, -CH=CH-, -CF=CF-, -CF=CH-, -CH=CF-, -CF₂-, -CF₂O-, -OCF₂-, -CF₂CH₂-, -CH₂CF₂-, -CF₂CF₂-, or -C≡C-, wherein R^{a} each independently represent a hydrogen atom or an alkyl group having 1 to 4 carbon atoms; and z represents an integer of 1 to 4; M^{p2} represents a 1,4-phenylene group, a 1,4-cyclohexylene group, an anthracen-2,6-diyl group, a phenanthren-2,7-diyl group, a pyridin-2,5-diyl group, a pyrimidin-2,5-diyl group, a naphthalen-2,6-diyl group, an indan-2,5-diyl group, a 1,2,3,4-tetrahydronaphthalen-2,6-diyl group, a 1,3-dioxan-2,5-diyl group, or a single bond, in which M^{p2} may be unsubstituted, or may be substituted by an alkyl group having 1 to 12 carbon atoms, a halogenated alkyl group having 1 to 12 carbon atoms, an alkoxy group having 1 to 12 carbon atoms, a halogenated alkoxy group having 1 to 12 carbon atoms, a halogen atom, a cyano group, a nitro group, or -R^{p1}; M^{p1} represents any of the following formulae (i-11) to (ix-11): 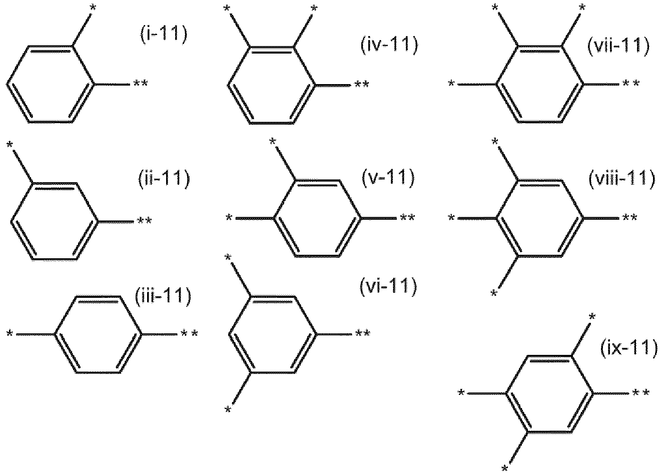 wherein * represents a bonding site to Sp^{p1}; and ** represents a bonding site to L^{p1}, L^{p2}, or Z^{p1}; M^{p3} represents any of the following formulae (i-13) to (ix-13): 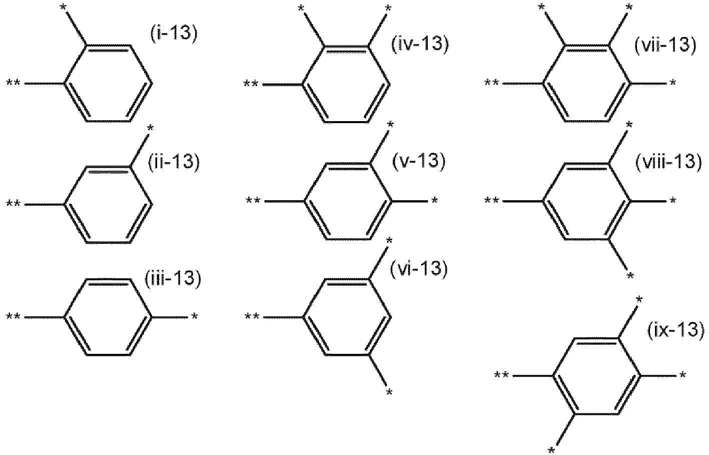 wherein * represents a bonding site to Z^{p1}_{;} and ** represents a bonding site to L^{p2}; m^{p2} to m^{p4} each independently represent 0, 1, 2, or 3; and m^{p1} and m^{p5} each independently represent 1, 2, or 3, in which in the case where a plurality of Z^{p1} are present, these may be the same as or different from each other; in the case where a plurality of R^{p1} are present, these may be the same as or different from each other; in the case where a plurality of R^{p2} are present, these may be the same as or different from each other; in the case where a plurality of Sp^{p1} are present, these may be the same as or different from each other; in the case where a plurality of Sp^{p2} are present, these may be the same as or different from each other; in the case where a plurality of L^{p1} are present, these may be the same as or different from each other; and in the case where a plurality of M^{p2} are present, these may be the same as or different from each other.

In the case where the liquid crystal composition of the embodiment contains the polymerizable compound in addition to the compound (i), a pretilt angle of liquid crystal molecules can be favorably formed.

The composition of the embodiment preferably does not contain a compound having a structure containing oxygen atoms bonded to each other, such as a peracid (-CO-OO-) structure, in the molecule thereof.

In the case where the reliability and the long-term stability of the composition are important, the content of a compound having a carbonyl group is preferably 5% by mass or less, more preferably 3% by mass or less, and further preferably 1% by mass or less, based on the total mass of the composition, and it is most preferred that the compound is substantially not contained.

In the case where the stability against UV irradiation is important, the content of a compound having a chlorine atom substituted thereon is preferably 15% by mass or less, preferably 10% by mass or less, preferably 8% by mass or less, preferably 5% by mass or less, and preferably 3% by mass or less, based on the total mass of the composition, and it is most preferred that the compound is substantially not contained.

The content of a compound having cyclic structures in the molecule thereof that are all 6-membered rings is preferably large, the content of a compound having cyclic structures in the molecule thereof that are all 6-membered rings is preferably 80% by mass or more, more preferably 90% by mass or more, and further preferably 95% by mass or more, based on the total mass of the composition, and it is most preferred that the composition is constituted substantially only by a compound having cyclic structures in the molecule thereof that are all 6-membered rings.

For suppressing deterioration of the composition due to oxidation, the content of a compound having a cyclohexenylene group as a cyclic structure is preferably small, the content of a compound having a cyclohexenylene group is preferably 10% by mass or less, preferably 8% by mass or less, 5% by mass or less, and preferably 3% by mass or less, based on the total mass of the composition, and it is most preferred that the compound is substantially not contained.

In the case where the improvement of the viscosity and the improvement of Tni are important, the content of a compound having a 2-methylbenzen-1,4-diyl group, in which a hydrogen atom may be replaced by a halogen, in the molecule thereof is preferably small, the content of a compound having a 2-methylbenzen-1,4-diyl group in the molecule thereof is preferably 10% by mass or less, 8% by mass or less, 5% by mass or less, and preferably 3% by mass or less, based on the total mass of the composition, and it is most preferred that the compound is substantially not contained.

In the description herein, the language substantially not contained means that the compound is not contained except for a matter that is unintentionally contained (i.e., unavoidable impurity).

The lower limit of the average elastic constant (K_{AVG}) of the liquid crystal composition is preferably 10 or more, preferably 10.5 or more, preferably 11 or more, preferably 11.5 or more, preferably 12 or more, preferably 12.3 or more, preferably 12.5 or more, preferably 12.8 or more, preferably 13 or more, preferably 13.3 or more, preferably 13.5 or more, preferably 13.8 or more, preferably 14 or more, preferably 14.3 or more, preferably 14.5 or more, preferably 14.8 or more, preferably 15 or more, preferably 15.3 or more, preferably 15.5 or more, preferably 15.8 or more, preferably 16 or more, preferably 16.3 or more, preferably 16.5 or more, preferably 16.8 or more, preferably 17 or more, preferably 17.3 or more, preferably 17.5 or more, preferably 17.8 or more, and preferably 18 or more. The upper limit of the average elastic constant (K_{AVG}) of the liquid crystal composition is preferably 25 or less, preferably 24.5 or less, preferably 24 or less, preferably 23.5 or less, preferably 23 or less, preferably 22.8 or less, preferably 22.5 or less, preferably 22.3 or less, preferably 22 or less, preferably 21.8 or less, preferably 21.5 or less, preferably 21.3 or less, preferably 21 or less, preferably 20.8 or less, preferably 20.5 or less, preferably 20.3 or less, preferably 20 or less, preferably 19.8 or less, preferably 19.5 or less, preferably 19.3 or less, preferably 19 or less, preferably 18.8 or less, preferably 18.5 or less, preferably 18.3 or less, preferably 18 or less, preferably 17.8 or less, preferably 17.5 or less, preferably 17.3 or less, and preferably 17 or less. In the case where the reduction of the electric power consumption is important, the suppression of the light intensity of the backlight is effective, the liquid crystal display element preferably has an enhanced light transmittance, and therefor the value of K_{AVG} is preferably set to a small value. In the case where the improvement of the response speed is important, the value of K_{AVG} is preferably set to a large value.

### Liquid Crystal Display Element

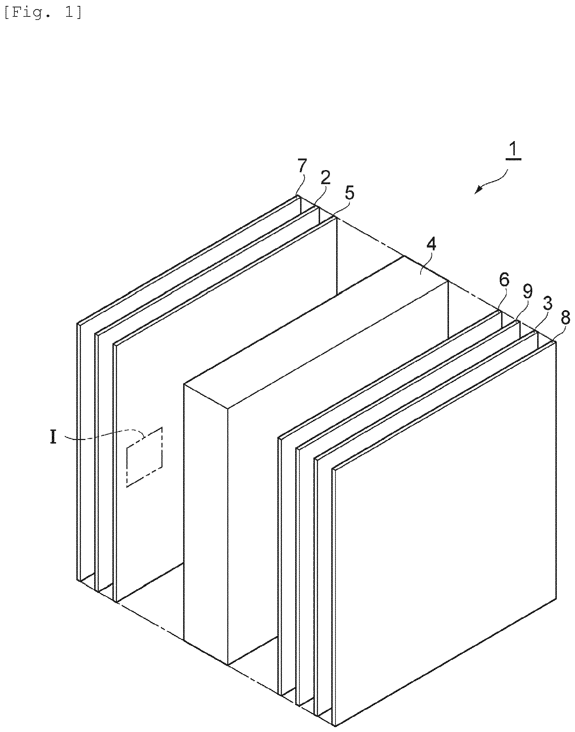

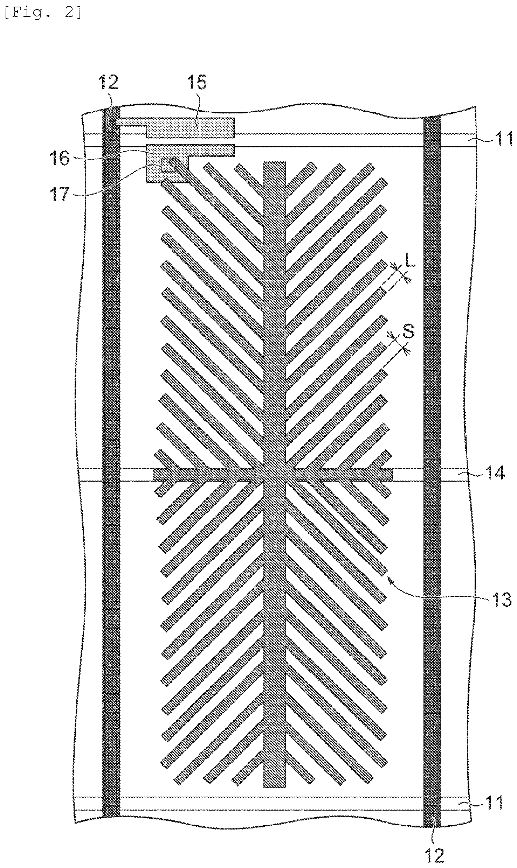

The liquid crystal composition of the embodiment may be applied to a liquid crystal display element. An example of the liquid crystal display element of the embodiment will be described below with reference to Figs. 1 and 2.

Fig. 1 is a diagram schematically showing a structure of a liquid crystal display element. In Fig. 1, the constitutional elements are shown with spaces intervening among them for expedient purposes. The liquid crystal display element 1 according to the embodiment is equipped with a first substrate 2 and a second substrate 3 that are disposed to face each other, and a liquid crystal layer 4 provided between the first substrate 2 and the second substrate 3, as shown in Fig. 1, and the liquid crystal layer 4 is constituted by the liquid crystal composition of the embodiment described above.

The first substrate 2 has a pixel electrode layer 5 provided on the surface thereof on the side of the liquid crystal layer 4. The second substrate 3 has a common electrode layer 6 provided on the surface thereof on the side of the liquid crystal layer 4. The first substrate 2 and the second substrate 3 may be held with a pair of polarizing plates 7 and 8. A color filter 9 may be further provided on the second substrate 3 on the side of the liquid crystal layer 4.

Accordingly, a liquid crystal display element 1 according to one embodiment has a structure containing a first polarizing plate 7, a first substrate 2, a pixel electrode layer 5, a liquid crystal layer 4 containing the liquid crystal composition, a common electrode layer 6, a color filter 9, a second substrate 3, and a second polarizing plate 8, which are laminated in this order.

The first substrate 2 and the second substrate 3 are formed, for example, of glass or a material having flexibility, such as plastics. At least one of the first substrate 2 and the second substrate 3 is formed of a transparent material, and the other thereof may be formed of a transparent material or may be formed of an opaque material, such as a metal and silicon. The first substrate 2 and the second substrate 3 are adhered to each other with a sealing material or a sealant, such as an epoxy thermosetting composition, disposed on the peripheral portions thereof, and a particle spacer, such as glass particles, plastic particles, or alumina particles, or a spacer column formed of a resin formed by the photolithography method may be disposed between the substrates for retaining the distance between them.

The first polarizing plate 7 and the second polarizing plate 8 may be regulated to improve the viewing angle and the contrast through adjustment of the polarizing axes of the polarizing plates, and are preferably regulated in such a manner that the transmission axes thereof are perpendicular to each other to make the transmission axes functioning in a normally black mode. In particular, any of the first polarizing plate 7 and the second polarizing plate 8 is preferably disposed to have a transmission axis that is in parallel to the alignment direction of the liquid crystal molecules without application of voltage.

The color filter 9 preferably forms a black matrix from the standpoint of the prevention of light leakage, and preferably forms a black matrix (not shown in the figure) at the portion corresponding to thin film transistors.

The black matrix may be provided on the substrate opposite to the array substrate along with the color filter, and may be provided on the side of the array substrate along with the color filter, and the black matrix and the color filter may be provided separately on the array substrate and the other substrate, respectively. Moreover, the black matrix may be provided separately from the color filter, and may be one that decreases the transmittance through the superposition of the colors of the color filter.

Fig. 2 is an enlarged plane view of the region surrounded by the line I, which is a part of the pixel electrode layer 5 formed on the first substrate 2 in Fig. 1. As shown in Fig. 2, the pixel electrode layer 5 containing thin film transistors formed on the first substrate 2 has a plurality of gate bus lines 11 for supplying a scanning signal and a plurality of data bus lines 12 for supplying a display signal, which are disposed crosswise to form a matrix. In Fig. 2, only one pair of the gate bus lines 11 and 11 and one pair of the data bus lines 12 and 12 are shown.

The region surrounded by the plurality of gate bus lines 11 and the plurality of data bus lines 12 forms a unit pixel of the liquid crystal display element, and a pixel electrode 13 is formed in the unit pixel. The pixel electrode 13 has a so-called fishbone structure having two backbone portions that are disposed perpendicularly to each other to form a cruciform, and a plurality of branch portions extending from the backbone portions. Between a pair of the gate bus lines 11 and 11, a Cs electrode 14 is also provided in substantially parallel to the gate bus line 11. In the vicinity of the crossover point where the gate bus line 11 and the data bus line 12 are crossed with each other, a thin film transistor containing a source electrode 15 and a drain electrode 16 is also provided. The drain electrode 16 has a contact hole 17 provided therein.

The gate bus lines 11 and the data bus lines 12 each are preferably formed of a metal film, more preferably formed of Al, Cu, Au, Ag, Cr, Ta, Ti, Mo, W, Ni, or alloys thereof, and further preferably formed of Mo, Al, or alloys thereof.

The pixel electrode 13 is preferably a transparent electrode for enhancing the transmittance. The transparent electrode may be formed by sputtering or the like of an oxide semiconductor (such as ZnO, InGaZnO, SiGe, GaAs, IZO (indium zinc oxide), ITO (indium tin oxide), SnO, TiO, and AZTO (AlZnSnO)). At this time, the film thickness of the transparent electrode may be from 10 to 200 nm. Moreover, for decreasing the electric resistance, the transparent electrode may be formed as a polycrystalline ITO film obtained by baking an amorphous ITO film.

In the liquid crystal display element of the embodiment, for example, a wiring pattern may be formed by sputtering a metal material, such as Al or an alloy thereof, on the first substrate 2 and the second substrate 3, thereby forming the pixel electrode layer 5 and the common electrode layer 6 respectively. Moreover, the color filter 9 may be formed, for example, by a pigment dispersion method, a printing method, an electrodeposition method, a dying method, or the like. In a method for forming the color filter by a pigment dispersion method, for example, a curable colored composition for a color filter is coated on the transparent substrate, patterned, and then cured through heating or light irradiation. The process may be performed for each of three colors, red, green, and blue, and thereby pixel portions for the color filter can be formed. The color filter 9 may also be provided on the side of the substrate having TFT and the like.

The first substrate 2 and the second substrate 3 are disposed to face each other in such a manner that the pixel electrode layer 5 and the common electrode layer 6 are directed inward, and at this time, the distance between the first substrate 2 and the second substrate 3 may be controlled through a spacer. In this case, the distance is preferably controlled to provide a thickness of the liquid crystal layer 4, for example, of from 1 to 100 µm.

In the case where the polarizing plates 7 and 8 are used, the product of the refractive index anisotropy Δn of the liquid crystal layer 4 and the thickness of the liquid crystal layer 4 is preferably controlled to maximize the contrast. Moreover, in the case where the two polarizing plates 7 and 8 are used, the polarizing axes of the polarizing plates may be controlled to improve the viewing angle and the contrast. A retardation film for enhancing the viewing angle may also be used. Thereafter, a sealing material, such as an epoxy thermosetting composition, is screen-printed on the substrates while providing a liquid crystal filling port, and then the substrates are adhered to each other, followed by curing the sealing material through heating.

The method for holding the composition between the two substrates 2 and 3 may be an ordinary method, such as a vacuum filling method, a one drop fill (ODF) method, or the like. The vacuum filling method causes no drop mark, but has a problem of occurrence of an injection mark, and in the embodiment, the ODF method can be more favorably applied to the display element produced. In the production process of a liquid crystal display element by the ODF method, a sealing material, such as an epoxy photothermal curing material, is coated in a mound form with a closed loop pattern with a dispenser on any one of the substrates as the backplane or the frontplane, and after dropping a prescribed amount of the composition into the pattern under a vacuum atmosphere, the frontplane and the backplane are adhered to produce the liquid crystal display element. In the embodiment, the drop marks occurring in dropping the liquid crystal composition onto the substrate can be prevented in the ODF method. The drop marks referred herein are defined as such a phenomenon that a mark formed in dropping a liquid crystal composition emerges whitely in black display.

Moreover, in the production process of a liquid crystal display element by the ODF method, while it is necessary to drop the optimum amount of the liquid crystal corresponding to the size of the liquid crystal display element, the liquid crystal composition of the embodiment is less influenced, for example, by a rapid change of pressure and an impact occurring in the dropping apparatus in dropping the liquid crystal, and therefore the liquid crystal can be continuously dropped stably for a prolonged period of time, thereby retaining the yield of the liquid crystal display element to a high level. In particular, in a small-size liquid crystal display element, which is frequently used in recently popular smartphones, due to the small optimum amount of the liquid crystal, it may be difficult to control the deviation of the amount of the liquid crystal from the optimum value, but by using the liquid crystal composition of the embodiment, a stable ejection amount of the liquid crystal material can be achieved even for the small-size liquid crystal display element.

In the case where the liquid crystal composition of the embodiment contains the polymerizable compound, the method for polymerizing the polymerizable compound is preferably a method of polymerizing the compound by irradiating active energy rays, such as an ultraviolet ray and an electron beam, solely, in combination, or sequentially. In the case where an ultraviolet ray is used, a polarized light source may be used, and an unpolarized light source may also be used. Moreover, in the case where the composition containing the polymerizable compound is polymerized in the state where the composition is held between two substrates, at least the substrate on the irradiation side necessarily has suitable transparency to the active energy ray. Furthermore, such a method may be employed that after polymerizing only a particular part by using a mask in light irradiation, the alignment state of the unpolymerized part is changed by changing the conditions, such as the electric field, the magnetic field, and the temperature, and then the polymerization is performed through further irradiation of an active energy ray. In the case where the exposure to an ultraviolet ray is performed, in particular, the exposure to an ultraviolet ray is preferably performed under application of an alternating electric field to the composition containing the polymerizable compound. The alternating electric field applied is preferably an alternating current having a frequency of from 10 Hz to 10 kHz, and more preferably a frequency of from 60 Hz to 10 kHz, and the voltage may be selected depending on the desired pretilt angle of the liquid crystal display element. In other words, the pretilt angle of the liquid crystal display element can be controlled by the voltage applied. In a liquid crystal display element of a lateral electric field MVA mode, the pretilt angle is preferably controlled to from 80 to 89.9 degrees from the standpoint of the alignment stability and the contrast.

The temperature in the irradiation is preferably in such a temperature range that the liquid crystal state of the composition of the embodiment is retained. The polymerization is preferably performed at a temperature near room temperature, which is typically a temperature of from 15 to 35°C. The lamp generating an ultraviolet ray used may be a metal halide lamp, a high pressure mercury lamp, an extra high pressure mercury lamp, or the like. Moreover, the wavelength of the ultraviolet ray radiated is preferably in a wavelength range that is not the absorption wavelength range of the composition, and the ultraviolet ray is preferably used after cutting depending on necessity. The intensity of the ultraviolet ray radiated is preferably from 0.1 mW/cm² to 100 W/cm², and more preferably from 2 mW/cm² to 50 W/cm². The energy amount of the ultraviolet ray radiated may be appropriately controlled, and is preferably from 10 mJ/cm² to 500 J/cm², and more preferably from 100 mJ/cm² to 200 J/cm². The intensity of the ultraviolet ray may be changed in the irradiation thereof. The period of time of the irradiation of the ultraviolet ray may be appropriately selected depending on the intensity of the ultraviolet ray, and is preferably from 10 to 3,600 seconds, and more preferably from 10 to 600 seconds.

In the liquid crystal composition of the embodiment, the compound (i) does not inhibit the polymerization reaction of the polymerizable compound, and therefore the polymerizable compound can be favorably polymerized to prevent the unreacted polymerizable compound from remaining in the liquid crystal composition.

In the case where the polymerizable compound used is, for example, the compound (ii), the resulting liquid crystal display element 1 has the two substrates 2 and 3 and the liquid crystal layer 4, which is provided between the two substrates 2 and 3 and contains the liquid crystal composition and the polymerized product of the compound represented by the general formula (ii). In this case, it is considered that the polymerized product of the compound represented by the general formula (ii) is located on the sides of the substrates 2 and 3 in the liquid crystal layer 4.

The liquid crystal display element 1 may be an active matrix driving liquid crystal display element. The liquid crystal display element 1 may be a liquid crystal display element of a PSA type, a PSVA type, a VA type, an IPS type, an FFS type, or an ECB type, and is preferably a PSA type liquid crystal display element.

The liquid crystal display element of the embodiment uses the liquid crystal composition containing the compound (i), and therefore does not require an alignment film, such as a polyimide alignment film, provided on the first substrate 2 and the second substrate 3 on the side of the liquid crystal layer 4. Accordingly, the liquid crystal display element of the embodiment can have such a structure that at least one substrate of the two substrates does not have an alignment film, such as a polyimide alignment film.

### Examples

The invention will be described more specifically with reference to examples below, but the invention is not limited to the examples.

### Synthesis Example: Synthesis of 3-fluoro-4'-[11-[(2-oxo-1,3-dioxan-5-yl)methoxy]undecyloxy ]biphenyl-4-yl 2-methylacrylate

In a reaction vessel equipped with a stirring device, a condenser, and a thermometer, 38 g (155 mmol) of 3-fluoro-4-benzyloxyphenylboric acid, 30.5 g (150 mmol) of 4-bromo-2-methoxyphenol, 32 g (232 mmol) of potassium carbonate, 1.8 g of tetrakis(triphenylphosphine) palladium, 200 mL of tetrahydrofuran, and 100 mL of pure water were charged, and reacted at 70°C for 5 hours. After completing the reaction, the reaction mixture was cooled, and after adding 10% hydrochloric acid, the target product was extracted with ethyl acetate. The organic layer was washed with water and a saturated sodium chloride aqueous solution, and the solvent was distilled away. Thereafter, dispersion washing with toluene and purification with an alumina column were performed to provide 39 g of the compound represented by the formula (1) .

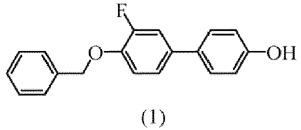

Subsequently, in a reaction vessel equipped with a stirring device, a condenser, and a thermometer, 26.5 g (90 mmol) of the compound represented by the formula (1), 5-((11-hydroxyundecyl)oxy)-1,3-dioxan-2-onebromoundecanol, 26 g (90 mmol) of triphenylphosphine, and 300 mL of dichloromethane were charged, and the reaction vessel was cooled to 5°C. Thereafter, 22 g (107 mmol) of diisopropyl diazocarboxylate (DIAD) was added dropwise thereto. After completing the dropwise addition, the reaction mixture was stirred at room temperature for 5 hours to complete the reaction. After completing the reaction, 200 mL of dichloromethane was added, and the organic layer was washed with pure water and a saturated sodium chloride aqueous solution. After distilling away the solvent, the product was purified with a silica gel column to provide 42 g of the compound represented by the formula (2).

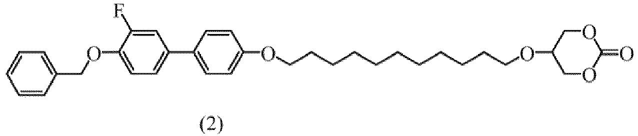

Subsequently, an autoclave equipped with a stirring device, 42 g of the compound represented by the formula (2) and 300 mL of THF were charged, and 25 mL of an ethanol solution and 2 g of 5% palladium-carbon (hydrous) were charged to perform catalytic hydrogen reduction with hydrogen gas, so as to provide 33 g of the compound represented by the formula (3).

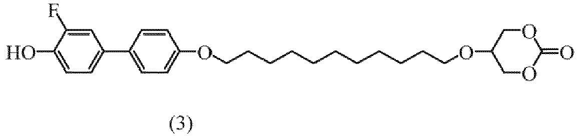

Furthermore, in a reaction vessel equipped with a stirring device, a condenser, and a thermometer, 33 g (70 mmol) of the compound represented by the formula (3), 9 g (104 mmol) of methacrylic acid, 1 g of dimethylaminopyridine, and 300 mL of dichloromethane were charged, and while retaining the reaction vessel to 5°C or less with an ice bath, 12 g (104 mmol) of diisopropylcarbodiimide was slowly added dropwise thereto under a nitrogen gas atmosphere. After completing the dropwise addition, the reaction vessel was returned to room temperature, and the reaction was performed for 5 hours . After filtering the reaction liquid, 150 mL of dichloromethane was added to the filtrate, which was then washed with water and a saturated sodium chloride aqueous solution, and the organic layer was dried over anhydrous sodium sulfate. After distilling away the solvent, the product was purified by column chromatography using twice the amount of silica gel (weight ratio), so as to provide 32 g of the target compound, 3-fluoro-4'-[11-[(2-oxo-1,3-dioxan-5-yl)methoxy]undecyloxy ]biphenyl-4-yl 2-methylacrylate represented by the formula (4) .

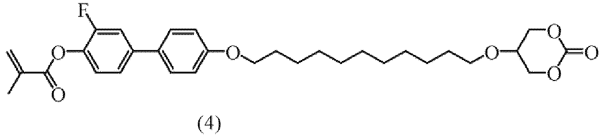

### Analytical Value of Resulting Compound

¹H-NMR (solvent: deuterated chloroform) : δ: 2.06 (s, 3H), 1.26-1.30 (m, 10H), 1.43-1.50 (m, 6H), 1.74 (m, 2H), 3.46 (t, 2H), 3.90 (t, 2H), 4.10-4.14 (m, 4H), 4.5 (m, 1H), 6.18-6.20 (m, 1H), 6.40-6.45 (m, 1H), 6.99 (s, 2H), 7.40-7.61 (m, 5H)

### Example 1

To 100% by weight of a composition containing the following compounds in the following mixing ratio, 0.4% by weight of the following polymerizable compound (R1-1-1) was added to provide a composition LC-1.

| | |
|---|---|
| 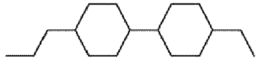 | 20 wt% |
| 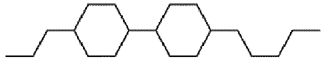 | 5 wt% |
| 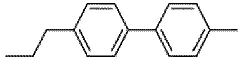 | 9 wt% |
| 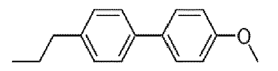 | 6 wt% |
| 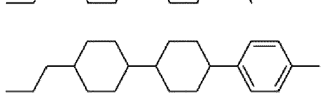 | 7 wt% |
| 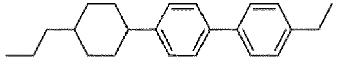 | 6 wt% |
| 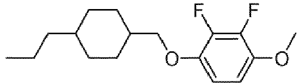 | 6 wt% |
| 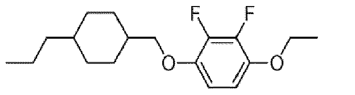 | 8 wt% |
| 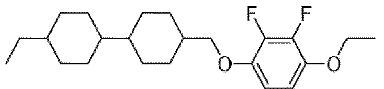 | 6 wt% |
| 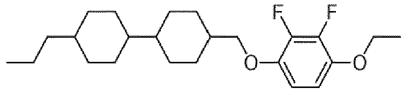 | 8 wt% |
| 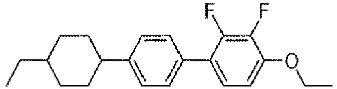 | 7 wt% |
| 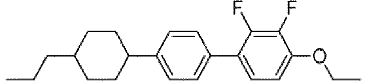 | 6 wt% |
| 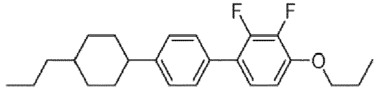 | 6 wt% |

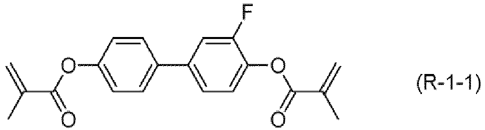

Furthermore, to 100% by weight of LC-1, 0.1% by weight of the compound (K1-1-1) corresponding to the compound (i) was added to prepare a liquid crystal composition.

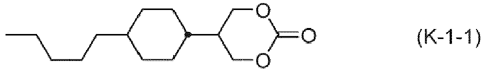

LC-1 had a nematic phase-isotropic liquid phase transition temperature (T_{NI}) of 75°C, a solid phase-nematic phase transition temperature (T_{CN}) of -33°C, a refractive index anisotropy (Δn) of 0.11, a dielectric anisotropy (Δε) of -2.8, and a rotational viscosity (γ₁) of 98 mPa·s. The refractive index anisotropy (Δn), the dielectric anisotropy (Δε), and the rotational viscosity (γ₁) were all measurement values at 25°C (hereinafter the same).

### Examples 2 to 15 and 18 to 23

Liquid crystal compositions were prepared in the same manner as in Example 1 except that the following compounds were added to LC-1 in the amounts shown in Table 1 instead of the compound (K1-1-1) in an amount of 0.1% by weight.

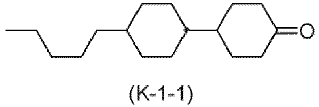

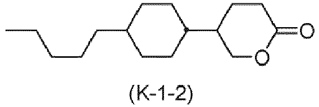

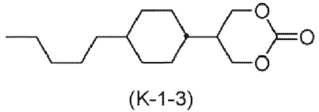

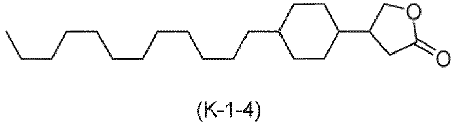

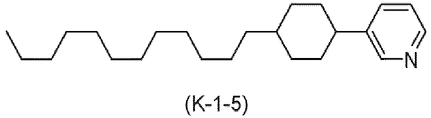

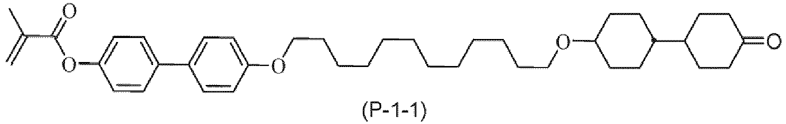

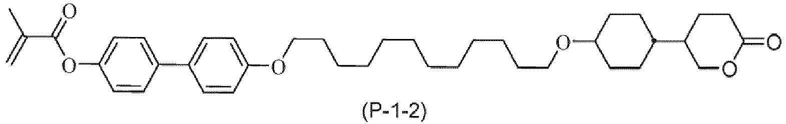

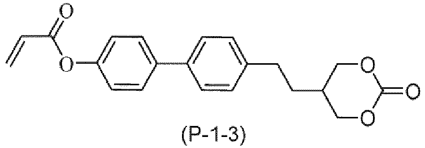

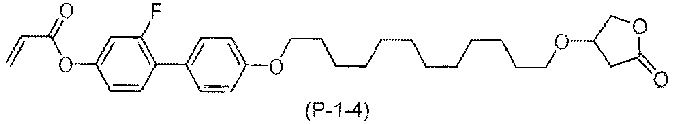

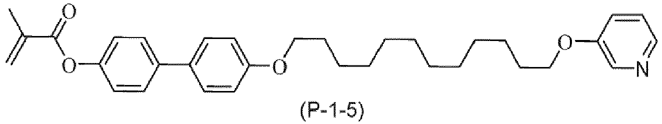

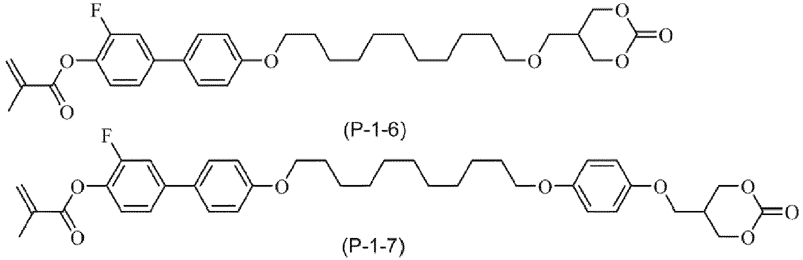

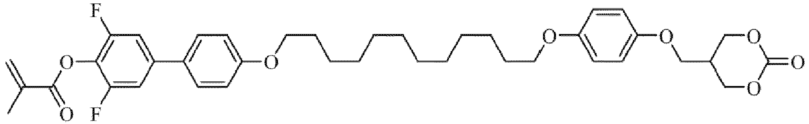

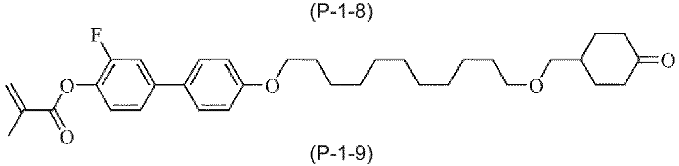

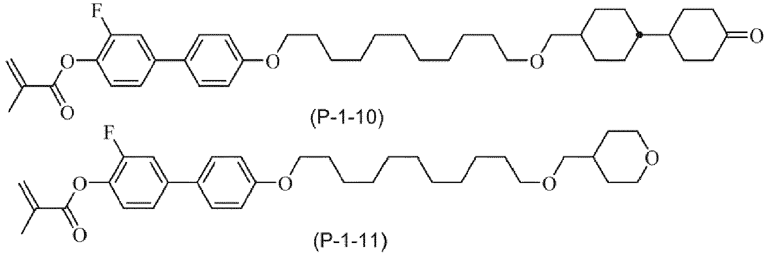

### Examples 16 and 17

Liquid crystal compositions were prepared in the same manner as in Examples 3 and 11 respectively except that a composition LC-2 obtained by adding 0.4% by weight of the polymerizable compound (R1-1-1) to 100% by weight of a composition containing the following compounds in the following mixing ratio was used instead of the composition LC-1.

| | |
|---|---|
| 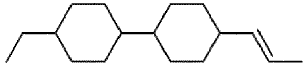 | 20 wt% |
| 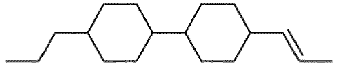 | 14.5 wt% |
| 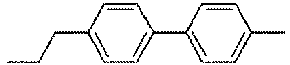 | 9.5 wt% |
| 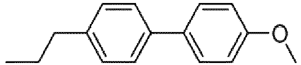 | 3 wt% |
| 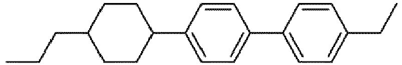 | 5.5 wt% |
| 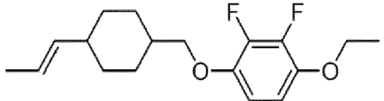 | 9wt% |
| 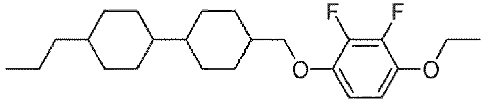 | 20.5 wt% |
| 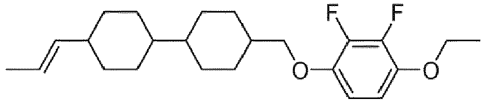 | 8 wt% |
| 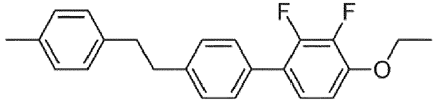 | 5 wt% |
| 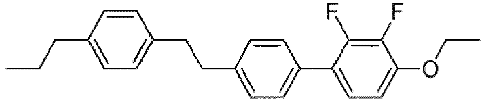 | 5 wt% |

LC-2 had T_{NI} of 81°C, T_{CN} of -54°C, Δn of 0.11, Δε of -3.0, and γi of 95 mPa·s.

### Comparative Example 1

A liquid crystal composition was prepared in the same manner as in Example 1 except that the compound (K1-1-1) was not used.

### Comparative Examples 2 to 7

Liquid crystal compositions were prepared in the same manner as in Example 1 except that the following compounds were added to LC-1 in the amounts shown in Table 1 instead of the compound (K1-1-1).

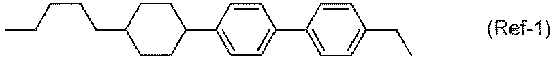

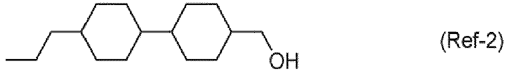

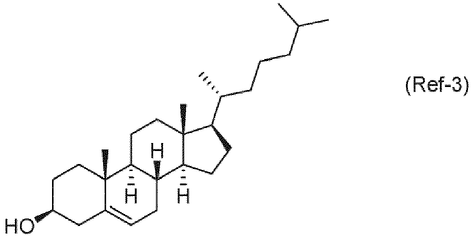

The liquid crystal compositions of Examples and Comparative Examples were subjected to the following evaluation tests. The results of the evaluation tests are shown in Table 1.

### Evaluation Test for Low Temperature Storage Stability

The liquid crystal composition was filtered with a membrane filter (produced by Agilent Technologies, Inc., PTFE, 13 m - 0.2 µm), and dissolved air was removed by allowing to stand under a vacuum condition for 15 minutes. 0.5 g of the liquid crystal composition was weighed into a vial container having been cleaned with acetone and sufficiently dried, and allowed to stand under an environment at -25°C for 10 days. Thereafter, the presence of deposition was visually observed, and judged by the following two grades.
A: No deposition was confirmed.
D: Deposition was confirmed.

### Evaluation Test for Vertical Alignment

A first substrate having a transparent electrode layer containing a transparent common electrode, and a color filter layer and having no alignment film (i.e., a common electrode substrate) and a second substrate having a pixel electrode layer containing a transparent pixel electrode driven by an active element and having no alignment film (i.e., a pixel electrode substrate) were produced. The liquid crystal composition was dropped on the first substrate, and held with the second substrate, and the sealing material was cured under a condition of 110°C for 2 hours, so as to provide a liquid crystal cell having a cell gap of 3.2 µm. The vertical alignment herein was observed with a polarization microscope, and evaluated by the following four grades.
A: Uniform vertical alignment was observed.
B: Alignment defects were observed slightly, which was an allowable level.
C: Alignment defects were observed, which was an unallowable level.
D: Alignment defects were significantly severe.

### Evaluation Test for Pretilt Angle Formation

The aforementioned liquid crystal cell (used for the evaluation test for the vertical alignment) was applied with a square alternating current wave of 10 V and 100 Hz, during which the liquid crystal cell was irradiated with UV light having an illuminance of 100 m/cm² at 365 nm for 200 seconds by using a high pressure mercury lamp. Thereafter, the cell was applied with a square alternating current wave of 10 V and 100 Hz, during which an external force was applied to the cell, and the stability of white display was evaluated by the following four grades.
A: Uniform alignment was observed.
B: Alignment defects were observed slightly, which was an allowable level.
C: Alignment defects were observed, which was an unallowable level.
D: Alignment defects were significantly severe.

### Evaluation Test for Residual Monomer Amount

The aforementioned liquid crystal cell (used for the evaluation test for the vertical alignment) was irradiated with a UV fluorescent lamp, produced by Toshiba Lighting and Technology Corporation, for 60 minutes (illuminance: 1.7 mW/cm² at 313 nm), and then the residual amount of the polymerizable compound (R1-1-1) was quantitatively determined by HPLC to determine the residual monomer amount. The evaluation was performed by the following four grades corresponding to the residual monomer amount.
A: less than 300 ppm
B: 300 ppm or more and less than 500 ppm
C: 500 ppm or more and less than 1,500 ppm
D: 1,500 ppm or more

### Evaluation Test for Response Characteristics

The liquid crystal compositions each were filled in a 10 µm liquid crystal cell having a transparent electrode and an alignment film, and measured for a rotational viscosity γ₁ (mPa · s) at 25°C with an equipment produced by Toyo Corporation (LCM-2), and thereby the response characteristics were evaluated by the following four grades.
A: less than 100 mPa·s
B: 100 mPa·s or more and less than 120 mPa·s
C: 120 mPa·s or more and less than 140 mPa·s
D: 140 mPa·s or more

**Table 1**

| | Base composition | Added compound | Amount added (% by weight) | Low temperature storage stability | Vertical alignment | Pretilt angle formation | Residual monomer amount | Response characteristics |
|---|---|---|---|---|---|---|---|---|
| Example 1 | LC-1 | (K-1-1) | 0.1 | A | B | A | A | B |
| Example 2 | LC-1 | (K-1-1) | 1 | A | A | A | A | B |
| Example 3 | LC-1 | (K-1-1) | 2 | A | A | A | A | B |
| Example 4 | LC-1 | (K-1-1) | 3 | A | A | A | A | B |
| Example 5 | LC-1 | (K-1-1) | 5 | A | A | A | A | B |
| Example 6 | LC-1 | (K-1-1) | 10 | A | A | A | A | C |
| Example 7 | LC-1 | (K-1-2) | 1.5 | A | A | A | A | B |
| Example 8 | LC-1 | (K-1-3) | 1.5 | A | A | A | A | B |
| Example 9 | LC-1 | (K-1-4) | 1.5 | A | A | A | A | B |
| Example 10 | LC-1 | (K-1-5) | 1.5 | A | A | A | A | B |
| Example 11 | LC-1 | (P-1-1) | 2 | A | A | A | A | A |
| Example 12 | LC-1 | (P-1-2) | 2 | A | A | A | A | A |
| Example 13 | LC-1 | (P-1-3) | 2 | A | A | A | A | A |
| Example 14 | LC-1 | (P-1-4) | 2 | A | A | A | A | A |
| Example 15 | LC-1 | (P-1-5) | 2 | A | A | A | A | A |
| Example 16 | LC-2 | (K-1-1) | 2 | A | A | A | A | A |
| Example 17 | LC-2 | (P-1-1) | 2 | A | A | A | A | A |
| Example 18 | LC-1 | (P-1-6) | 2 | A | A | A | A | A |
| Example 19 | LC-1 | (P-1-7) | 2 | A | A | A | A | A |
| Example 20 | LC-1 | (P-1-8) | 2 | A | A | A | A | A |
| Example 21 | LC-1 | (P-1-9) | 2 | A | A | A | A | A |
| Example 22 | LC-1 | (P-1-10) | 2 | A | A | A | A | A |
| Example 23 | LC-1 | (P-1-11) | 2 | A | A | A | A | A |
| Comparative Example 1 | LC-1 | - | - | A | D | - | A | A |
| Comparative Example 2 | LC-1 | Ref-1 | 1 | A | D | - | A | A |
| Comparative Example 3 | LC-1 | Ref-1 | 3 | D | D | - | A | A |
| Comparative Example 4 | LC-1 | Ref-2 | 1 | D | A | C | C | D |
| Comparative Example 5 | LC-1 | Ref-2 | 3 | D | A | C | D | D |
| Comparative Example 6 | LC-1 | Ref-3 | 1 | D | A | C | C | D |
| Comparative Example 7 | LC-1 | Ref-3 | 3 | D | A | C | D | D |

### Reference Signs List

- 1:: Liquid crystal display element
- 2:: First substrate
- 3:: Second substrate
- 4:: Liquid crystal layer
- 5:: Pixel electrode layer
- 6:: Common electrode layer
- 7:: First polarizing plate
- 8:: Second polarizing plate
- 9:: Color filter
- 11:: Gate bus line
- 12:: Data bus line
- 13:: Pixel electrode
- 14:: Cs electrode
- 15:: Source electrode
- 16:: Drain electrode
- 17:: Contact hole

## Claims

1. A spontaneous orientation aid for a liquid crystal composition, comprising one kind or two or more kinds of a compound having a partial structure represented by the general formula (i): 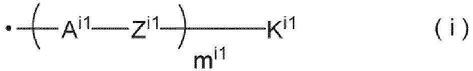 wherein
Zⁱ¹ represents a single bond, -CH=CH-, -CF=CF-, -C≡C-, -COO-, -OCO-, -CF₂O-, -OCF₂-, or an alkylene group having 2 to 20 carbon atoms, in which one or two or more of -CH₂- that are not adjacent to each other in the alkylene group each may be replaced by -O-, -COO-, or -OCO-;
Aⁱ¹ represents a divalent 6-membered aromatic group, a divalent 6-membered heteroaromatic group, a divalent 6-membered alicyclic group, a divalent 6-membered heteroalicyclic group, or a single bond, in which a hydrogen atom in the cyclic structures may be replaced by a halogen atom, and in a case where a plurality of each of Zⁱ¹ and Aⁱ¹ are present, these may be the same as or different from each other;
mⁱ¹ represents an integer of 1 to 5; and
Kⁱ¹ represents a structure represented by any of the following formulae (K-1) to (K-8): 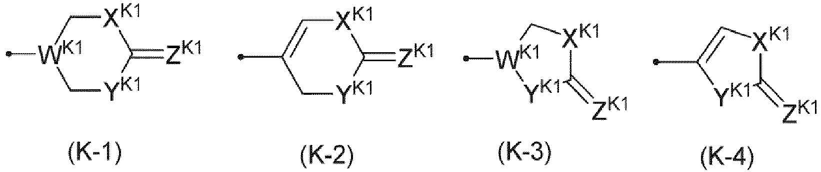 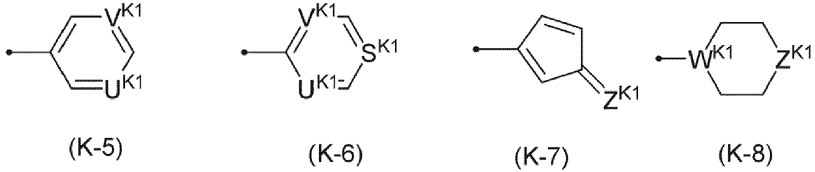 whe
rein
W^{K1} represents a methine group or a nitrogen atom;
X^{K1} and Y^{K1} each independently represent -CH₂-, an oxygen atom, or a sulfur atom;
Z^{K1} represents an oxygen atom or a sulfur atom; and U^{K1}, V^{K1}, and S^{K1} each independently represent a methine group or a nitrogen atom, provided that a combination where U^{K1} represents a methine group, V^{K1} represents a methine group, and S^{K1} represents a nitrogen atom is excluded, and
in the formula (i) and the formulae (K-1) to (K-8), the black dot on the left end represents a bonding site.

2. The spontaneous orientation aid for a liquid crystal composition according to claim 1, wherein the compound having a partial structure represented by the general formula (i) is a compound represented by the general formula (ii): 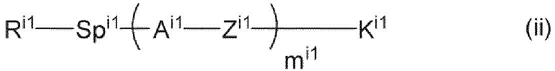 wherein
Zⁱ¹, Aⁱ¹, mⁱ¹, and Kⁱ¹ have the same meanings of Zⁱ¹, Aⁱ¹, mⁱ¹, and Kⁱ¹ in the formula (i) respectively;
Spⁱ¹ represents a linear alkylene group having 1 to 18 carbon atoms, a branched alkylene group having 1 to 18 carbon atoms, or a single bond, in which one or two or more of -CH₂-that are not adjacent to each other in the alkylene group each may be replaced by -O-, -COO-, or -OCO-; and
Rⁱ¹ represents a hydrogen atom or a substituent selected from the group consisting of the formulae (R-1) to (R-15): 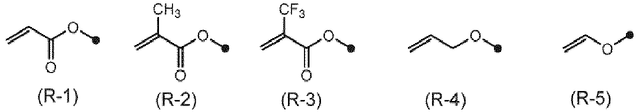 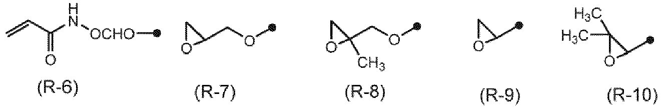 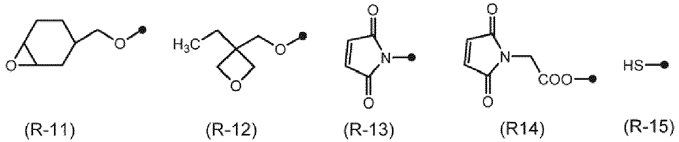 wherein the black dot on the right end represents a bonding site.

3. A liquid crystal composition having a negative dielectric anisotropy (Δε), comprising one kind or two or more kinds of a compound having a partial structure represented by the general formula (i): 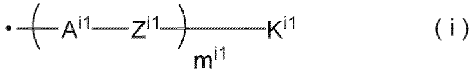 wherein
Zⁱ¹ represents a single bond, -CH=CH-, -CF=CF-, -C≡C-, -COO-, -OCO-, -CF₂O-, -OCF₂-, or an alkylene group having 2 to 20 carbon atoms, in which one or two or more of -CH₂- that are not adjacent to each other in the alkylene group each may be replaced by -O-, -COO-, or -OCO-;
Aⁱ¹ represents a divalent 6-membered aromatic group, a divalent 6-membered heteroaromatic group, a divalent 6-membered alicyclic group, a divalent 6-membered heteroalicyclic group, or a single bond, in which a hydrogen atom in the cyclic structures may be replaced by a halogen atom, and in a case where a plurality of each of Zⁱ¹ and Aⁱ¹ are present, these may be the same as or different from each other;
mⁱ¹ represents an integer of 1 to 5; and
Kⁱ¹ represents a structure represented by any of the following formulae (K-1) to (K-8): 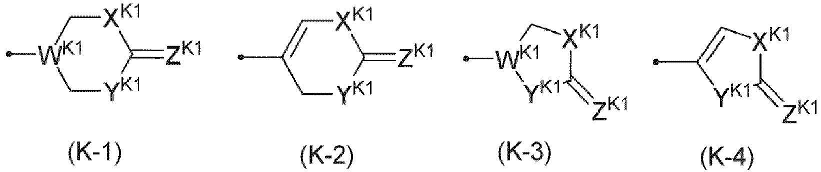 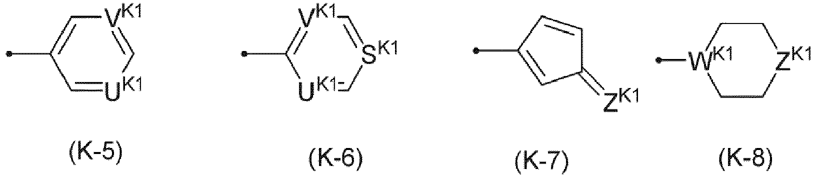
wherein
W^{K1} represents a methine group or a nitrogen atom;
X^{K1} and Y^{K1} each independently represent -CH₂-, an oxygen atom, or a sulfur atom;
Z^{K1} represents an oxygen atom or a sulfur atom; and
U^{K1}, V^{K1}, and S^{K1} each independently represent a methine group or a nitrogen atom, provided that a combination where U^{K1} represents a methine group, V^{K1} represents a methine group, and S^{K1} represents a nitrogen atom is excluded, and
in the formula (i) and the formulae (K-1) to (K-8), the black dot on the left end represents a bonding site.

4. The liquid crystal composition according to claim 3, wherein the liquid crystal composition comprises, as the compound having a partial structure represented by the general formula (i), a compound represented by the general formula (ii) : 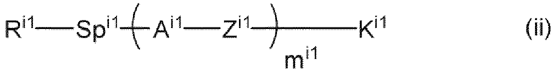 wherein
Zⁱ¹, Aⁱ¹, mⁱ¹, and Kⁱ¹ have the same meanings of Zⁱ¹, Aⁱ¹, mⁱ¹, and Kⁱ¹ in the formula (i) respectively;
Spⁱ¹ represents a linear alkylene group having 1 to 18 carbon atoms, a branched alkylene group having 1 to 18 carbon atoms, or a single bond, in which one or two or more of -CH₂-that are not adjacent to each other in the alkylene group each may be replaced by -O-, -COO-, or -OCO-; and
Rⁱ¹ represents a hydrogen atom or a substituent selected from the group consisting of the formulae (R-1) to (R-15): 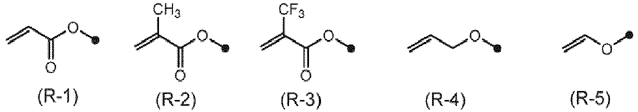 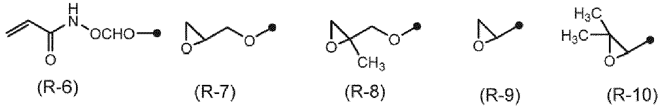 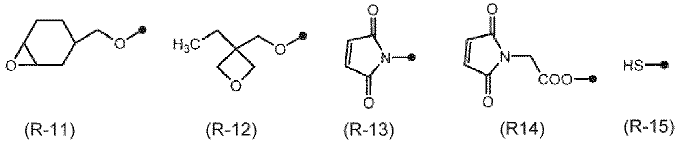
wherein the black dot on the right end represents a bonding site.

5. The liquid crystal composition according to claim 3 or 4, wherein the liquid crystal composition further comprises a compound selected from a group of compounds represented by any of the general formulae (N-1), (N-2), and (N-3): 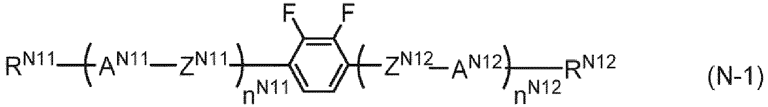 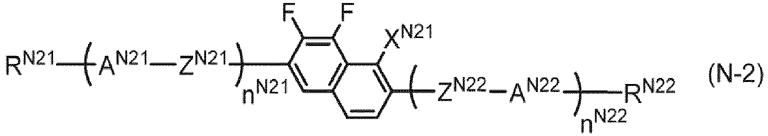 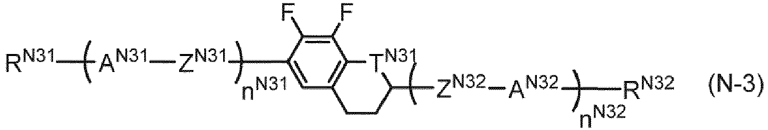 wherein
R^{N11}, R^{N12}, R^{N21}, R^{N22}, R^{N31}, and R^{N32} each independently represent an alkyl group having 1 to 8 carbon atoms, in which one or two or more of -CH₂- that are not adjacent to each other in the alkyl group each independently may be replaced by -CH=CH-, -C≡C-, -O-, -CO-, -COO-, or -OCO-;
A^{N11}, A^{N12}, A^{N21}, A^{N22}, A^{N31}, and A^{N32} each independently represent a group selected from the group consisting of
(a) a 1,4-cyclohexylene group (in which one of -CH₂- or two or more of -CH₂- that are not adjacent to each other present in the group may be replaced by -O-),
(b) a 1,4-phenylene group (in which one of -CH= or two or more of -CH= that are not adjacent to each other present in the group may be replaced by -N=),
(c) a naphthalen-2,6-diyl group, a 1,2,3,4-tetrahydronaphthalen-2,6-diyl group, or a decahydronaphthalen-2,6-diyl group (in which one of -CH= or two or more of -CH= that are not adjacent to each other present in the naphthalen-2,6-diyl group or the 1,2,3,4-tetrahydronaphthalen-2,6-diyl group may be replaced by -N=), and
(d) a 1,4-cyclohexenylene group,
in which the group (a), the group (b), the group (c), and the group (d) each independently may be substituted by a cyano group, a fluorine atom, or a chlorine atom;
Z^{N11}, Z^{N12}, Z^{N21}, Z^{N22}, Z^{N31}, and Z^{N32} each independently represent a single bond, -CH₂CH₂-, - (CH₂)₄-, -OCH₂-, -CH₂O-, -COO-, -OCO-, -OCF₂-, -CF₂O-, -CH=N-N=CH-, -CH=CH-, -CF=CF-, or -C≡C-;
X^{N21} represents a hydrogen atom or a fluorine atom; T^{N31} represents -CH₂- or an oxygen atom; and
n^{N11}, n^{N12}, n^{N21}, n^{N22}, n^{N31}, and n^{N32} each independently represent an integer of 0 to 3, provided that n^{N11}+n^{N12}, n^{N21}+n^{N22}, and n^{N31}+n^{N32} each independently are 1, 2, or 3,
in which in a case where a plurality of each of A^{N11} to A^{N32} and Z^{N11} to Z^{N32} are present, these may be the same as or different from each other.

6. The liquid crystal composition according to any one of claims 3 to 5, wherein the liquid crystal composition further comprises a compound represented by the general formula (L) : 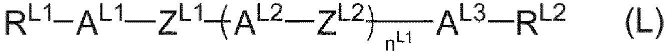 wherein
R^{L1} and R^{L2} each independently represent an alkyl group having 1 to 8 carbon atoms, in which one or two or more of -CH₂-that are not adjacent to each other in the alkyl group each independently may be replaced by -CH=CH-, -C≡C-, -O-, -CO-, -COO-, or -OCO-;
n^{L1} represents 0, 1, 2, or 3;
A^{L1}, A^{L2}, and A^{L3} each independently represent a group selected from the group consisting of
(a) a 1,4-cyclohexylene group (in which one of -CH₂- or two or more of -CH₂- that are not adjacent to each other present in the group may be replaced by -O-),
(b) a 1,4-phenylene group (in which one of -CH= or two or more of -CH= that are not adjacent to each other present in the group may be replaced by -N=), and
(c) a naphthalen-2,6-diyl group, a 1,2,3,4-tetrahydronaphthalen-2,6-diyl group, or a decahydronaphthalen-2,6-diyl group (in which one of -CH= or two or more of -CH= that are not adjacent to each other present in the naphthalen-2,6-diyl group or the 1,2,3,4-tetrahydronaphthalen-2,6-diyl group may be replaced by -N=),
in which the group (a), the group (b), and the group (c) each independently may be substituted by a cyano group, a fluorine atom, or a chlorine atom; and
Z^{L1} and Z^{L2} each independently represent a single bond, -CH₂CH₂-, -(CH₂)₄-, -OCH₂-, -CH₂O-, -COO-, -OCO-, -OCF₂-, -CF₂O-, -CH=N-N=CH-, -CH=CH-, -CF=CF-, or -C≡C-;
in the case where n^{L1} is 2 or 3, and a plurality of A^{L2} are present, these may be the same as or different from each other; in the case where n^{L1} is 2 or 3, and a plurality of Z^{L2} are present, these may be the same as or different from each other; and compounds represented by the general formulae (N-1), (N-2), and (N-3) are excluded.

7. The liquid crystal composition according to any one of claims 3 to 6, wherein the liquid crystal composition comprises the compound having a partial structure represented by the general formula (i) in an amount of from 0.01 to 50% by mass based on the total amount of the liquid crystal composition.

8. The liquid crystal composition according to any one of claims 3 to 7, wherein the liquid crystal composition further comprises a polymerizable compound.

9. The liquid crystal composition according to claim 8, wherein the liquid crystal composition comprises, as the polymerizable compound, one kind or two or more kinds of a compound represented by the general formula (P): 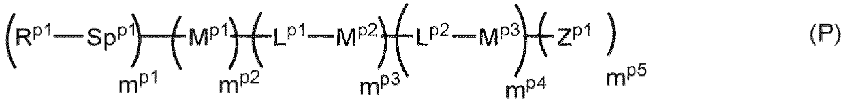 wherein
Z^{p1} represents a fluorine atom, a cyano group, a hydrogen atom, an alkyl group having 1 to 15 carbon atoms , in which a hydrogen atom may be replaced by a halogen atom, an alkoxy group having 1 to 15 carbon atoms, in which a hydrogen atom may be replaced by a halogen atom, an alkenyl group having 1 to 15 carbon atoms, in which a hydrogen atom may be replaced by a halogen atom, an alkenyloxy group having 1 to 15 carbon atoms, in which a hydrogen atom may be replaced by a halogen atom, or -Sp^{p2}-R^{p2};
R^{p1} and R^{p2} each represent any of the following formulae (R-I) to (R-IX): 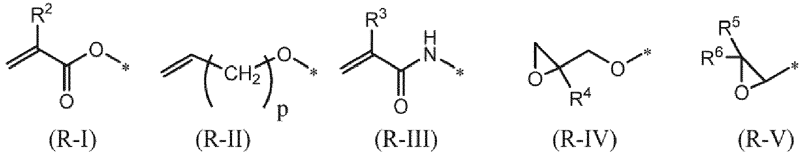 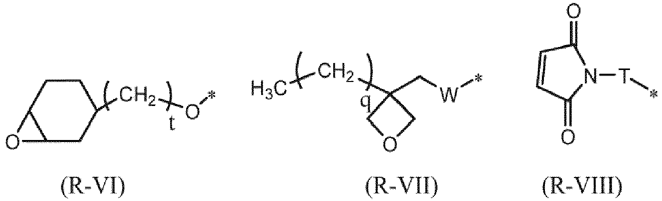 HS-* (R-IX)
wherein
* represents a bonding site to Sp^{p1};
R² to R⁶ each independently represent a hydrogen atom, an alkyl group having 1 to 5 carbon atoms, or a halogenated alkyl group having 1 to 5 carbon atoms;
W represents a single bond, -O-, or a methylene group;
T represents a single bond or -COO-; and
p, t, and q each independently represent 0, 1, or 2;
Sp^{p1} and Sp^{p2} each represent a spacer group;
L^{p1} and L^{p2} each independently represent a single bond, -O-, -S-, -CH₂-, -OCH₂-, -CH₂O-, -CO-, -C₂H₄-, -COO-, -OCO-, -OCOOCH₂-, -CH₂OCOO-, -OCH₂CH₂O-, -CO-NR^{a}-, -NR^{a}-CO-, -SCH₂-, -CH₂S-, -CH=CR^{a}-COO-, -CH=CR^{a}-OCO-, -COO-CR^{a}=CH-, -OCO-CR^{a}=CH-, -COO-CR^{a}=CH-COO-, -COO-CR^{a}=CH-OCO-, -OCO-CR^{a}=CH-COO-, -OCO-CR^{a}=CH-OCO-, -(CH₂)_{z}-C(=O)-O-, -(CH₂)_{z}-O-(C=O)-, -O-(C=O)-(CH₂)-, -(C=O)-O-(CH₂)_{z}-, -CH=CH-, -CF=CF-, -CF=CH-, -CH=CF-, -CF₂-, -CF₂O-, -OCF₂-, -CF₂CH₂-, -CH₂CF₂-, -CF₂CF₂-, or -C≡C-, wherein R^{a}'s each independently represent a hydrogen atom or an alkyl group having 1 to 4 carbon atoms; and z represents an integer of 1 to 4;
M^{p2} represents a 1,4-phenylene group, a 1,4-cyclohexylene group, an anthracen-2,6-diyl group, a phenanthren-2,7-diyl group, a pyridin-2,5-diyl group, a pyrimidin-2,5-diyl group, a naphthalen-2,6-diyl group, an indan-2,5-diyl group, a 1,2,3,4-tetrahydronaphthalen-2,6-diyl group, a 1,3-dioxan-2,5-diyl group, or a single bond, in which M^{p2} may be unsubstituted, or may be substituted by an alkyl group having 1 to 12 carbon atoms, a halogenated alkyl group having 1 to 12 carbon atoms, an alkoxy group having 1 to 12 carbon atoms , a halogenated alkoxy group having 1 to 12 carbon atoms, a halogen atom, a cyano group, a nitro group, or -R^{p1};
M^{p1} represents any of the following formulae (i-11) to (ix-11) : 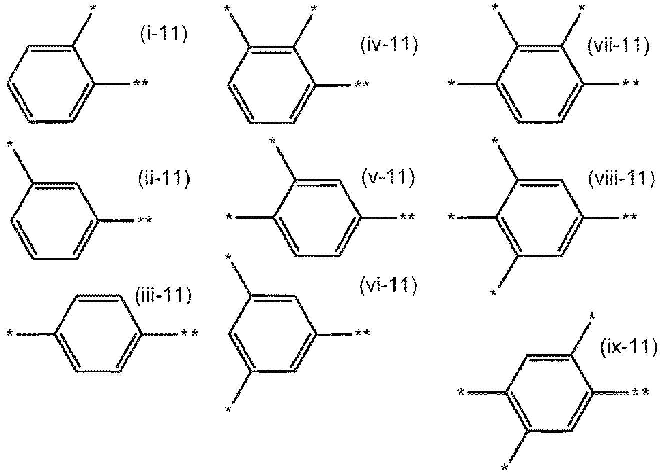
wherein * represents a bonding site to Sp^{p1}; and ** represents a bonding site to L^{p1}, L^{p2}, or Z^{p1};
M^{p3} represents any of the following formulae (i-13) to (ix-13) : 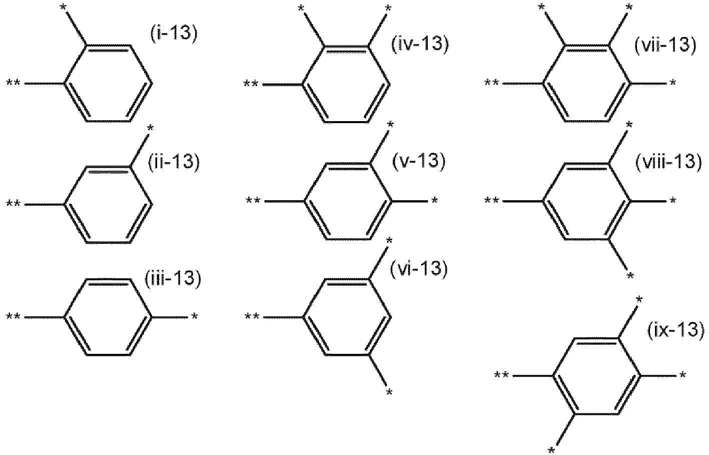
wherein * represents a bonding site to Z^{p1}; and ** represents a bonding site to L^{p2};
m^{p2} to m^{p4} each independently represent 0, 1, 2, or 3; and
m^{p1} and m^{p5} each independently represent 1, 2, or 3,
in which in a case where a plurality of Z^{p1} are present, these may be the same as or different from each other; in a case where a plurality of R^{p1} are present, these may be the same as or different from each other; in a case where a plurality of R^{p2} are present, these may be the same as or different from each other; in a case where a plurality of Sp^{p1} are present, these may be the same as or different from each other; in a case where a plurality of Sp^{p2} are present, these may be the same as or different from each other; in a case where a plurality of L^{p1} are present, these may be the same as or different from each other; and in a case where a plurality of M^{p2} are present, these may be the same as or different from each other.

10. A liquid crystal display element comprising two substrates and a liquid crystal layer provided between the two substrates, containing the liquid crystal composition according to any one of claims 3 to 9.

11. A liquid crystal display element comprising two substrates and a liquid crystal layer provided between the two substrates, containing the liquid crystal composition according to any one of claims 3 to 9 and a polymerized product of a compound represented by the general formula (ii): 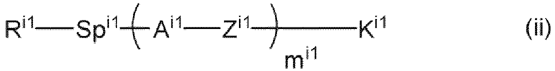 wherein
Zⁱ¹, Aⁱ¹, mⁱ¹, and Kⁱ¹ have the same meanings of Zⁱ¹, Aⁱ¹, mⁱ¹, and Kⁱ¹ in the formula (i) respectively;
Spⁱ¹ represents a linear alkylene group having 1 to 18 carbon atoms, a branched alkylene group having 1 to 18 carbon atoms, or a single bond, in which one or two or more of -CH₂-that are not adjacent to each other in the alkylene group each may be replaced by -O-, -COO-, or -OCO-; and
Rⁱ¹ represents a hydrogen atom or a substituent selected from the group consisting of the formulae (R-1) to (R-15): 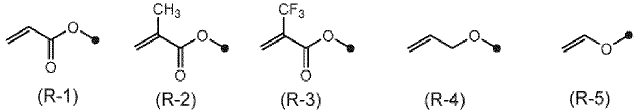 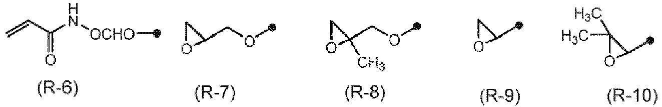 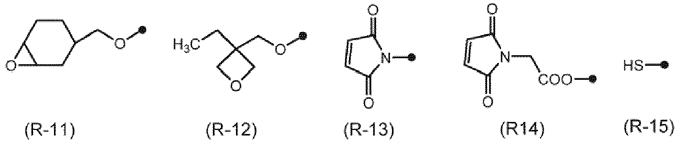 wherein the black dot on the right end represents a bonding site.

12. The liquid crystal display element according to claim 10 or 11, wherein the liquid crystal display element is an active matrix driving liquid crystal display element.

13. The liquid crystal display element according to any one of claims 10 to 12, wherein the liquid crystal display element is a liquid crystal display element of a PSA type, a PSVA type, a VA type, an IPS type, an FFS type, or an ECB type.

14. The liquid crystal display element according to any one of claims 10 to 13, wherein at least one substrate of the two substrates does not have an alignment film.

15. A compound represented by the general formula (ii): 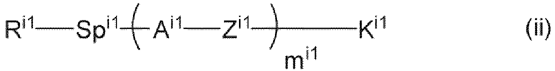 wherein
Zⁱ¹ represents a single bond, -CH=CH-, -CF=CF-, -C≡C-, -COO-, -OCO-, -CF₂O-, -OCF₂-, or an alkylene group having 2 to 20 carbon atoms, in which one or two or more of -CH₂- that are not adjacent to each other in the alkylene group each may be replaced by -O-, -COO-, or -OCO-;
Aⁱ¹ represents a divalent 6-membered aromatic group, a divalent 6-membered heteroaromatic group, a divalent 6-membered alicyclic group, a divalent 6-membered heteroalicyclic group, or a single bond, in which a hydrogen atom in the cyclic structures may be replaced by a halogen atom, and in a case where a plurality of each of Zⁱ¹ and Aⁱ¹ are present, these may be the same as or different from each other;
mⁱ¹ represents an integer of 1 to 5;
Kⁱ¹ represents a structure represented by any of the following formulae (K-1) to (K-8): 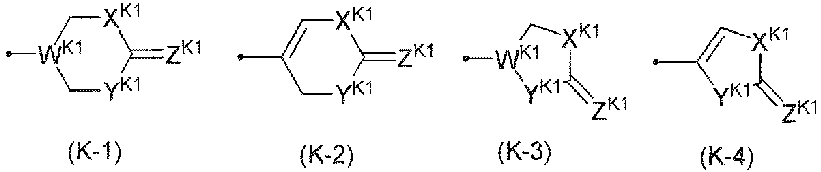 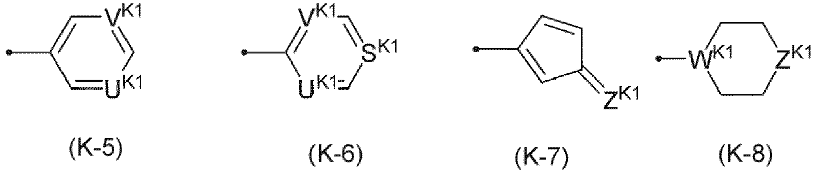
wherein
the black dot on the left end represents a bonding site;
W^{K1} represents a methine group or a nitrogen atom;
X^{K1} and Y^{K1} each independently represent -CH₂-, an oxygen atom, or a sulfur atom;
Z^{K1} represents an oxygen atom or a sulfur atom; and
U^{K1}, V^{K1}, and S^{K1} each independently represent a methine group or a nitrogen atom, provided that a combination where U^{K1} represents a methine group, V^{K1} represents a methine group, and S^{K1} represents a nitrogen atom is excluded;
Spⁱ¹ represents a linear alkylene group having 1 to 18 carbon atoms, a branched alkylene group having 1 to 18 carbon atoms, or a single bond, in which one or two or more of -CH₂-that are not adjacent to each other in the alkylene group each may be replaced by -O-, -COO-, or -OCO-; and
Rⁱ¹ represents a hydrogen atom or a substituent selected from the group consisting of the formulae (R-1) to (R-15): 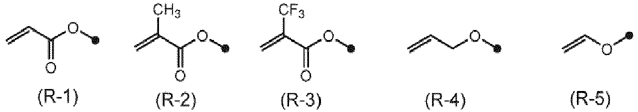 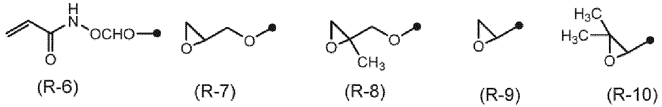 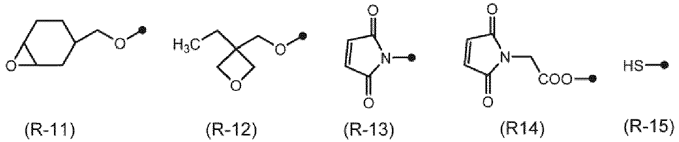
wherein the black dot on the right end represents a bonding site.
